# EUROPEAN PATENT SPECIFICATION

(11) **EP 1 922 334 B1**
(45) Date of publication and mention of the grant of the patent: **05.01.2011**
(21) Application number: 06777167.5
(22) Date of filing: 05.09.2006
(51) Int. Cl.: C07K 14/47, C12N 15/00, A61K 38/00, A61K 39/00, C12P 21/00

(54) **TUMOR-ASSOCIATED PEPTIDES BINDING TO HUMAN LEUKOCYTE ANTIGEN (HLA) CLASS I OR II MOLECULES AND RELATED ANTI-CANCER VACCINE**
AN HUMANE LEUKOZYTENANTIGEN-(HLA-)KLASSE-I- ODER II-MOLEKÜLE BINDENDE TUMORASSOZIIERTE PEPTIDE UND DAMIT IM ZUSAMMENHANG STEHENDER IMPFSTOFF GEGEN KREBS
PEPTIDES ASSOCIES AUX TUMEURS SE LIANT AUX MOLECULES D'ANTIGENE DE LEUCOCYTE HUMAIN (HLA) DE CLASSE I OU II ET VACCIN CONTRE LE CANCER ASSOCIE

(30) Priority: 05.09.2005 EP 05019255
(43) Date of publication of application: 21.05.2008
(73) Proprietor: Immatics Biotechnologies GmbH, 72076 Tübingen (DE)
(72) Inventor: SINGH, Harpreet, 72072 Tübingen (DE); EMMERICH, Niels, 72074 Tübingen (DE); WALTER, Steffen, 72411 Dusslingen (DE); WEINSCHENK, Toni, 73773 Aichwald (DE)
(74) Representative: Krauss, Jan
(86) International application number: PCT/EP2006/008641
(87) International publication number: WO 2007/028573

(56) References cited:
- WO-A-00/06723
- US-A- 6 096 313
- US-A1- 2004 171 543
- HEID H W ET AL: "Adipocyte differentiation-related protein is secreted into milk as a constituent of milk lipid globule membrane" BIOCHEMICAL JOURNAL, PORTLAND PRESS, LONDON, GB, vol. 320, no. PART 3, 15 December 1996 (1996-12-15), pages 1025-1030, XP002060680 ISSN: 0264-6021
- BARNETT J ET AL.: "Production, purification and characterization of human matrilysin (PUMP) from recombinant chinese hamster ovary cells" PROTEIN EXPRESSION AND PURIFICATION, vol. 5, 1994, pages 27-36, XP002352893
- WELCH AR ET AL.: "Purification of human matrilysin produced in Escherichia coli and characterization using a new optimized fluorogenic peptide substrate" ARCHIVES OF BIOCHEMISTRY AND BIOPHYSICS, vol. 324, no. 1, 1995, pages 59-64, XP002352894
- BROSSART P ET AL: "Identification of HLA-A2-restricted T-cell epitopes derived from the MUC1 tumor antigen for broadly applicable vaccine therapies" BLOOD, W.B.SAUNDERS COMPANY, ORLANDO, FL, US, vol. 93, no. 12, 15 June 1999 (1999-06-15), pages 4309-4317, XP002147432 ISSN: 0006-4971
- WEINSCHENK T ET AL: "Integrated functional genomics approach for the design of patient-individual antitumor vaccines" CANCER RESEARCH, AMERICAN ASSOCIATION FOR CANCER RESEARCH, BALTIMORE, MD, US, vol. 62, no. 20, 15 October 2002 (2002-10-15), pages 5818-5827, XP002266492 ISSN: 0008-5472 cited in the application
- SUMI TOSHIYUKI ET AL: "Expression of matrix metalloproteinases 7 and 2 in human renal cell carcinoma" ONCOLOGY REPORTS, NATIONAL HELLENIC RESEARCH FOUNDATION, ATHENS, GR, vol. 10, no. 3, May 2003 (2003-05), pages 567-570, XP002352032 ISSN: 1021-335X cited in the application
- SCHMIDT SM ET AL: "Induction of adipophilin-specific cytotoxic T lymphocytes using a novel HLA-A2 binding peptide that mediates tumor cell lysis" CANCER RESEARCH, vol. 64, 2004, pages 1164-1170, XP002352895 cited in the application
- BROSSART P ET AL: "Virus-mediated delivery of antigenic peptides into dendritic cells as a means to induce CTL" JOURNAL OF IMMUNOLOGY, THE WILLIAMS AND WILKINS CO. BALTIMORE, US, vol. 7, no. 158, 1 April 1997 (1997-04-01), pages 3270-3276, XP002075947 ISSN: 0022-1767

## Description

The present invention relates to immunotherapeutic methods, and molecules and cells for use in immunotherapeutic methods. In particular, the present invention relates to the immunotherapy of cancer, in particular renal cancer. The present invention furthermore relates to tumour-associated T-helper cell peptide epitopes, alone or in combination with other tumour-associated peptides that serve as active pharmaceutical ingredients of vaccine compositions which stimulate anti-tumour immune responses. In particular, the present invention relates to two novel peptide sequences derived from HLA class I molecules of human tumour cell lines which can be used in vaccine compositions for eliciting anti-tumour immune responses.

### Background of the invention

Stimulation of an immune response is dependent upon the presence of antigens recognised as foreign by the host immune system. The discovery of the existence of tumour associated antigens has now raised the possibility of using a host's immune system to intervene in tumour growth. Various mechanisms of harnessing both the humoral and cellular arms of the immune system are currently being explored for cancer immunotherapy.

Specific elements of the cellular immune response are capable of specifically recognising and destroying tumour cells. The isolation of cytotoxic T-cells (CTL) from tumour-infiltrating cell populations or from peripheral blood suggests that such cells play an important role in natural immune defences against cancer (Cheever et al., Annals N.Y. Acad. Sci. 1993 690:101-112). CD8-positive T-cells (TCD8-positive) in particular, which recognise Class I molecules of the major histocompatibility complex (MHC)-bearing peptides of usually 8 to 10 residues derived from proteins located in the cytosol, play an important role in this response. The MHC-molecules of humans are also designated as human leukocyte-antigens (HLA).

There are two classes of MHC-molecules: MHC-class I- molecules that can be found on most cells having a nucleus that present peptides that result from proteolytic cleavage of endogenous proteins and larger peptides. MHC-class II-molecules can be found only on professional antigen presenting cells (APC), and present peptides of exogenous proteins that are taken up by APCs during the course of endocytosis, and are subsequently processed. Complexes of peptide and MHC-I are recognised by CD8-positive cytotoxic T-lymphocytes, complexes of peptide and MHC-II are recognised by CD4-positive -helper-T-cells.

CD4-positive helper T-cells play an important role in orchestrating the effector functions of anti-tumour T-cell responses and for this reason the identification of CD4-positive T-cell epitopes derived from tumour associated antigens (TAA) may be of great importance for the development of pharmaceutical products for triggering anti-tumour immune responses (Kobayashi, H., R. Omiya, M. Ruiz, E. Huarte, P. Sarobe, J. J. Lasarte, M. Herraiz, B. Sangro, J. Prieto, F. Borras-Cuesta, and E. Celis. 2002. Identification of an antigenic epitope for helper T lymphocytes from carcinoembryonic antigen. Clin. Cancer Res. 8:3219-3225., Gnjatic, S., D. Atanackovic, E. Jäger, M. Matsuo, A. Selvakumar, N.K. Altorki, R.G. Maki, B. Dupont, G. Ritter, Y.T. Chen, A. Knuth, and L.J. Old. 2003. Survey of naturally occurring CD4+ T-cell responses against NY-ESO-1 in cancer patients: Correlation with antibody responses. Proc. Natl. Acad Sci. U.S.A. 100(15):8862-7).

It was shown in mammalian animal models, e.g., mice, that even in the absence of cytotoxic T lymphocyte (CTL) effector cells (i.e., CD8-positive T lymphocytes), CD4 positive T-cells are sufficient for inhibiting manifestation of tumours via inhibition of angiogenesis by secretion of interferon-gamma (IFNγ) (Qin, Z. and T. Blankenstein. 2000. CD4+ T-cell--mediated tumour rejection involves inhibition of angiogenesis that is dependent on IFN gamma receptor expression by nonhematopoietic cells. Immunity. 12:677-686). Additionally, it was shown that CD4 positive T-cells recognizing peptides from tumour-associated antigens presented by HLA class II molecules can counteract tumour progression via the induction of an Antibody (Ab) responses (Kennedy, R.C., M.H. Shearer, A.M. Watts, and R.K. Bright. 2003. CD4+ T lymphocytes play a critical role in antibody production and tumour immunity against simian virus 40 large tumour antigen. Cancer Res. 63:1040-1045). In contrast to tumour-associated peptides binding to HLA class I molecules, only a small number of class II ligands of TAA have been described so far (www.cancerimmunity.org, www.syfpeithi.de). Since the constitutive expression of HLA class II molecules is usually limited to cells of the immune system (Mach, B., V. Steimle, E. Martinez-Soria, and W. Reith. 1996. Regulation of MHC class II genes: lessons from a disease. Annu. Rev. Immunol. 14:301-331), the possibility of isolating class II peptides directly from primary tumours was not considered possible. Therefore, numerous strategies to target antigens into the class II processing pathway of antigen presenting cells (APCs) have been described, for example the incubation of APCs with the antigen of interest to enable it to be taken up, processed and presented (Chaux, P., V. Vantomme, V. Stroobant, K. Thielemans, J. Corthals, R. Luiten, A.M. Eggermont, T. Boon, and B.P. van der Bruggen. 1999. Identification of MAGE-3 epitopes presented by HLA-DR molecules to CD4(+) T lymphocytes. J. Exp. Med 189:767-778), or the transfection of cells with genes or minigenes encoding the antigen of interest and fused to the invariant chain, which mediates the translocation of antigens to the lysosomal MHC class II processing and assembling compartment (MIIC).

In order for a peptide to trigger (elicit) a cellular immune response, it must bind to an MHC-molecule. This process is dependent on the allele of the MHC-molecule and specific polymorphisms of the amino acid sequence of the peptide. MHC-class-I-binding peptides are usually 8-10 residues in length and contain two conserved residues ("anchors") in their primary amino acid sequence that interact with the corresponding binding groove of the MHC-molecule.

In the absence of inflammation, expression of MHC class II molecules is mainly restricted to cells of the immune system, especially professional antigen-presenting cells (APC), e.g., monocytes, monocyte-derived cells, macrophages, dendritic cells.

The antigens that are recognised by the tumour specific T- lymphocytes, that is, their epitopes, can be molecules derived from all protein classes, such as enzymes, receptors, transcription factors, etc. Furthermore, tumour associated antigens, for example, can also be present in tumour cells only, for example as products of mutated genes. Another important class of tumour associated antigens are tissue-specific structures, such as CT ("cancer testis")-antigens that are expressed in different kinds of tumours and in healthy tissue of the testis. Various tumour associated antigens have been identified. Further, much research effort is being expended to identify additional tumour associated antigens. Some groups of tumour associated antigens, also referred to in the art as tumour specific antigens, are tissue specific. Examples include, but are not limited to, tyrosinase for melanoma, PSA and PSMA for prostate cancer and chromosomal cross-overs (translocations) such as bcr/abl in lymphoma. However, many tumour associated antigens identified occur in multiple tumour types, and some, such as oncogenic proteins and/or tumour suppressor genes (tumour suppressor genes are, for example reviewed for renal cancer in Linehan WM, Walther MM, Zbar B. The genetic basis of cancer of the kidney. J Urol. 2003 Dec;170(6 Pt 1):2163-72) which actually cause the transformation event, occur in nearly all tumour types. For example, normal cellular proteins that control cell growth and differentiation, such as p53 (which is an example for a tumour suppressor gene), ras, c-met, myc, pRB, VHL, and HER-2/neu, can accumulate mutations resulting in upregulation of expression of these gene products thereby making them oncogenic (McCartey et al. Cancer Research 1998 15:58 2601-5; Disis et al. Ciba Found. Symp. 1994 187:198-211).

Mucin-1 (MUC1) is a highly glycosylated type I transmembrane glycoprotein that is abundantly overexpressed on the cell surface of many human adenocarcinomas like breast and ovarian cancers. Aberrant deglycosylation in malignancies is common and unmasks epitopes in tumour cells which might not be presented on normal cells. Moreover, MUC1 expression has been demonstrated in multiple myeloma and some B-cell Non-Hodgkin lymphomas (Gendler S, Taylor-Papadimitriou J, Duhig T, Rothbard J, and Burchell J. A highly immunogenic region of a human polymorphic epithelial mucin expressed by carcinomas is made up of tandem repeats. J. Biol. Chem. 263:12820-12823 (1988); Siddiqui 1988; Girling A, Bartkova J, Burchell J, Gendler S, Gillett C, and Taylor-Papadimitriou J. A core protein epitope of the polymorphic epithelial mucin detected by the monoclonal antibody SM-3 is selectively exposed in a range of primary carcinomas. Int. J. Cancer 43:1072-1076 (1989); Brossart 1999; Duperray 1989; Mark 1989; Delsol 1988; Apostolopoulos V and McKenzie IF. Cellular mucins: targets for immunotherapy. Crit Rev. Immunol. 14:293-309 (1994); Finn OJ, Jerome KR, Henderson RA, Pecher G, Domenech N, Magarian-Blander J, and Barratt-Boyes SM. MUC-1 epithelial tumor mucin-based immunity and cancer vaccines. Immunol. Rev. 145:61-89 (1995)). Several recent reports (Apostolopoulos V and McKenzie IF. Cellular mucins: targets for immunotherapy. Crit Rev. Immunol. 14:293-309 (1994); Finn OJ, Jerome KR, Henderson RA, Pecher G, Domenech N, Magarian-Blander J, and Barratt-Boyes SM. MUC-1 epithelial tumor mucin-based immunity and cancer vaccines. Immunol. Rev. 145:61-89 (1995); Bamd 1989; Takahashi 1994; Noto 1997) demonstrated that cytotoxic MHC-unrestricted T-cells from ovarian, breast, pancreatic, and multiple myeloma tumours can recognize epitopes of the MUC1 protein core localized in the tandem repeat. Two HLA-A2-restricted T-cell epitopes derived from the MUC1 protein have been identified (Brossart 1999, EP 1484397). One peptide is derived from the tandem repeat region of the MUC1 protein. The second peptide is localized within the signal sequence of MUC1. Induction of cytotoxic T-lymphocyte responses in vivo after vaccinations with peptide-pulsed dendritic cells in patients with advanced breast or ovarian cancer using those peptides has been successful (Brossart 2000) (Wierecky 2005). With respect to renal cell carcinoma, MUC1 expression is common in conventional tumours and has been reported to be associated with tumour grade and stage (Fujita 1999; Kraus 2002; Leroy 2002; Bamias 2003; Cao 2000). For MUC1, protein overexpression is not correlated to mRNA overexpression.

Adipophilin is a marker for specialized differentiated cells containing lipid droplets and for diseases associated with fat-accumulating cells (Heid 1998). Adipophilin occurs in a wide range of cultured cell lines, including fibroblasts and endothelial and epithelial cells. In tissues, however, expression of adipophilin is restricted to certain cell types, such as lactating mammary epithelial cells, adrenal cortex cells, Sertoli and Leydig cells of the male reproductive system, and steatosis or fatty change hepatocytes in alcoholic liver cirrhosis (Heid 1998). Adipophilin has been reported to be overexpressed in colorectal cancer (Saha 2001), hepatocellular carcinoma (Kurokawa 2004), and in renal cell carcinoma (Young 2001).

c-Met encodes a heterodimeric transmembranous receptor with tyrosine kinase activity that is composed of an α-chain that is disulfide-linked to a β-subunit (Bottaro 1991; Rubin 1993). Both subunits are expressed on the surface, the heavy β-subunit is responsible for the binding of the ligand, hepatocyte growth factor (HGF), the α-subunit contains an intracellular domain that mediates the activation of different signal transduction pathways. c-Met signalling is involved in organ regeneration, as demonstrated for liver and kidney, embryogenesis, haematopoiesis, muscle development, and in the regulation of migration and adhesion of normally activated B-cells and monocytes (Zamegar 1995; Naldini 1991; Montesano 1998; Schmidt 1995; Uehara 1995; Bladt 1995; Takayama 1996; Mizuno 1993; van, V 1997; Beilmann 2000). Furthermore, numerous studies indicated the involvement of c-Met overexpression in malignant transformation and invasiveness of malignant cells.

c-Met mediates the multifunctional and potentially oncogenic activities of the HGF/scatter factor including promotion of cell growth, motility, survival, extracellular matrix dissolution, and angiogenesis (Bottaro 1991; Rubin 1993; Zarnegar 1995). Binding of HGF to the receptor induces autophosphorylation of c-Met and activates downstream signalling events including the ras, phosphatidylinositol 3'-kinase, phospholipase Cγ, and mitogen-activated protein kinase-related pathways (Naldini 1991; Montesano 1998; Furge 2000; Ponzetto 1993; Dong 2001; Furge 2001). The c-Met gene is expressed predominantly in epithelial cells and is over-expressed in several malignant tissues and cell lines (Di Renzo 1995; Ferracini 1995; Tuck 1996; Koochekpour 1997; Li 2001; Fischer 1998; Maulik 2002; Qian 2002; Ramirez 2000). An increasing number of reports have shown that nonepithelial cells such as haematopoietic, neural, and skeletal cells respond to HGF and haematological malignancies like multiple myeloma, Hodgkin disease, leukaemia, and lymphoma express the c-Met protein (Gherardi 1991; Teofili 2001; Borset 1999; Jucker 1994; Pons 1998). Deregulated control of the invasive growth phenotype by oncogenically activated c-Met provoked by c-Met-activating mutations, c-Met amplification/over-expression, and the acquisition of HGF/c-Met autocrine loops confers invasive and metastatic properties to malignant cells. Notably, constitutive activation of c-Met in HGF-over-expressing transgenic mice promotes broad tumourigenesis (Wang 2001; Takayama 1997).

Regulator of G-Protein Signalling 5 (RGS5) is a negative regulator of heterotrimeric G-protein signalling pathways although its function in vivo remains elusive. RGS proteins comprise a family of molecules with a unifying catalytic function but varying tissue distribution. They stimulate the intrinsic guanosine triphosphatase (GTPase) activity of activated Gα subunits and thereby accelerate G-protein inactivation. Thus, RGS molecules inhibit signalling downstream of G-protein-coupled receptors (De 2000). Recently, it has been shown that Regulator of G-protein signaling-5 induction in pericytes coincides with active vessel remodelling during tumour neovascularization. In a mouse model of pancreatic islet cell carcinogenesis, as well as in highly angiogenic astrocytomas, overexpression of RGS5 has been shown in pericytes during the angiogenic switch accompanying active vessel remodelling. Overexpression was restricted to the tumour vasculature as compared to a normal islet of Langerhans. However, RGS5 is also upregulated during wound healing and ovulation (Berger 2005).

Expression of RGS5 is increased in RCC (Rae 2000). In another study, RT-PCR showed strong expression of RGS5 in all RCCs examined, and expression was very weak or undetectable in normal kidneys (6.6:1 by real-time PCR). Tumour endothelial cells were the main location of RGS5 in RCC (Furuya 2004). Furthermore, RGS5 was reported to be a sinusoidal endothelial cell marker in hepatocellular carcinoma (Chen 2004).

Apolipoprotein L1 (APOL1) is a secreted high density lipoprotein which binds to apolipoprotein A-I. Apolipoprotein A-I is a relatively abundant plasma protein and is the major apoprotein of HDL. It is involved in the formation of most cholesteryl esters in plasma and also promotes efflux of cholesterol from cells. Apolipoprotein L1 may play a role in lipid exchange and transport throughout the body, as well as in reverse cholesterol transport from peripheral cells to the liver. The plasma protein is a single chain polypeptide with an apparent molecular mass of about 40 kDa (Duchateau 1997; Duchateau 2001). APOL1 cDNA was isolated from an activated endothelial cell cDNA library and shown to be upregulated by TNF-α, which is a potent proinflammatory cytokine. (Monajemi 2002).

KIAA0367 was identified in the Kazusa cDNA Project that aims to identify unknown long human transcripts encoding for putative proteins (Ohara 1997). Although the function of the putative 820 amino acid long protein product of KIAA0367 is unknown, it contains a CRAL-TRIO lipid binding domain profile at the C-terminus which binds small hydrophobic molecules and that is present in several nucleotide exchange factors and in the BCL2/adenovirus E1B 19-kDa protein-interacting protein 2 (BNIP-2). BNIP-2 is involved in the control of diverse cellular functions including cell morphology, migration, endocytosis and cell cycle progression (Zhou 2005). KIAA0367 is located on the chromosomal region 9q21. This region is described as a common target of homozygous deletion in many tumours (Gursky 2001; Weber 2001) or loss of heterozygocity (Louhelainen 2000; Tripathi 2003).

Soluble guanylate cyclase (sGC), a heterodimeric protein consisting of an alpha and a beta subunit (1 heme group), catalyzes the conversion of GTP to the second messenger cGMP and functions as the main receptor for nitric oxide and nitrovasodilator drugs (Zabel 1998). GUCYa3 and b3 are overexpressed in human gliomas. Transfection of antisense GUCY1A3 or GUCY1B3 reduced vascularisation and tumour growth in nude mice. This might be due to the fact that VEGF is induced by cGMP (Saino 2004). GUCY1A3 promotes tumour cell migration of a mice mammary tumor cell line (Jadeski 2003).

Cyclin D1 belongs to the highly conserved cyclin family, more specific to the cyclin D subfamily (Xiong 1991; Lew 1991). Cyclins function as regulators of CDKs (cyclin-dependent kinases). Different cyclins exhibit distinct expression and degradation patterns which contribute to the temporal coordination of each mitotic event (Deshpande 2005). Cyclin D1 forms a complex with- and functions as a regulatory subunit of CDK4 or CDK6, whose activity is required for cell cycle G1/S transition. CCND1 forms with CDK4 and CDK6 a serine/threonine kinase holoenzyme complex imparting substrate specificity to the complex (Bates 1994). The protein has been shown to interact with tumour suppressor protein Rb (Loden 2002) and the expression of this gene is regulated positively by Rb (Halaban 1999). Mutations, amplification and overexpression of this gene, which alters cell cycle progression, are observed frequently in a variety of tumours and may contribute to tumorigenesis (Hedberg 1999; Vasef 1999; Troussard 2000).

Proteins of the matrix metalloproteinase (MMP) family are involved in the breakdown of extracellular matrix in normal physiological processes, such as embryonic development, reproduction, and tissue remodelling, as well as in disease processes, such as arthritis and metastasis (Mott 2004). Matrix metalloproteinase 7 (MMP7) is secreted as an inactive proprotein of 29.6 kDa which is activated when cleaved by extracellular proteinases. The active enzyme has a molecular weight of 19.1 kDa and binds two zinc ions and two calcium ions per subunit (Miyazaki 1990; Browner 1995). MMP7 degrades gelatins, fibronectin and casein (Miyazaki 1990; Quantin 1989) and differs from most MMP family members in that it lacks a conserved C-terminal protein domain (Gaire 1994). MMP7 is often found overexpressed in malignant tissue (Lin 2004; Bramhall 1997; Denys 2004) and it is suggested that it facilitates tumour cell invasion in vivo (Wang 2005).

These proteins can be the target of a tumour specific immune response in multiple types of cancer.

The Hepatitis B Virus Core Antigen peptide HBV-001 is not derived from an endogenous human tumour-associated antigen, but is derived from the Hepatitis B virus core antigen. Firstly, it allows to quantitavely compare the magnitude of T-cell responses induced by TUMAPs and hence allows important conclusions on the capacity to elicit anti-tumour responses. Secondly, it functions as an important positive control in the case of lack of any T-cell responses in the patient. And thirdly, it also allows to conclude on the status of immunocompetence of the patient.

Hepatitiv B virus (HBV) infection is among the leading causes of liver disease, affecting approximately 350 million people world-wide (Rehermann 2005). Due to the ease of horizontal and vertical transmission and the potential for chronic disease that may lead to liver cirrhosis and hepatocellular carcinoma, HBV represents a major impact on the public health system for many countries worldwide. The HBV genome (Previsani 2002) is comprised of partially double-stranded circular DNA. In HBV virions, it is packed together with the core protein HBc and other proteins to form the nucleocapsid, which is surrounded by an outer envelope containing lipids and the surface protein family HBs (also called envelope protein). The antigenic determinants which are associated with HBc and HBs are noted as HBcAg and HBsAg, respectively. These antigens are associated with serological, i.e. antibody responses found in the patient blood and are among the clinically most useful antigen-antibody systems for the diagnosis of HBV infection. HBc will represent a novel foreign antigen for all individuals without prior history of HBV infection. As immunogenic peptides are well known for this antigen (Bertoletti 1993; Livingston 1997), one ten-amino acid peptide from HBcAg was selected as a positive control antigen within IMA. The induction of HBc peptide-specific CTLs will then be used as a marker for patient immunocompetence and successful vaccination.

Immunotherapy in cancer patients aims at activating cells of the immune system specifically, especially the so-called cytotoxic T-cells (CTL, also known as "killer cells", also known as CD8-positive T-cells), against tumour cells but not against healthy tissue. Tumour cells differ from healthy cells by the expression of tumour-associated proteins. HLA molecules on the cell surface present the cellular content to the outside, thus enabling a cytotoxic T-cell to differentiate between a healthy and a tumour cell. This is realized by breaking down all proteins inside the cell into short peptides, which are then attached to HLA molecules and presented on the cell surface (Rammensee 1993). Peptides that are presented on tumour cells, but not or to a far lesser extent on healthy cells of the body, are called tumour-associated peptides (TUMAPs).

First clinical trials using tumour-associated peptides have started in the mid-1990s by Boon and colleagues mainly for the indication melanoma. Clinical responses in the best trials have ranged from 10% to 30%. Severe side effects or severe autoimmunity have not been reported in any clinical trial using peptide-based vaccine monotherapy. Mild forms of vitiligo have been reported for some patients who had been treated with melanoma-associated peptides.

However, priming of one kind of CTL is usually insufficient to eliminate all tumour cells. Tumours are very mutagenic and thus able to respond rapidly to CTL attacks by changing their protein pattern to evade recognition by CTLs. To counter-attack the tumour evasion mechanisms a variety of specific peptides is used for vaccination. In this way a broad simultaneous attack can be mounted against the tumour by several CTL clones simultaneously. This may decrease the chances of the tumour to evade the immune response. This hypothesis has been recently confirmed in a clinical study treating late-stage melanoma patients. With only few exceptions, patients that had at least 3 distinct T-cell responses, showed objective clinical responses or stable disease (Banchereau 2001) as well as increased survival (personal communication with J. Banchereau), while the vast majority of patients with less than 3 T-cell responses were diagnosed with progressive disease.

Until now, numerous strategies to target antigens into the class II or I processing pathways have been described. It is possible to incubate antigen presenting cells (APCs) with the antigen of interest in order to be taken up and processed (Chaux, P., Vantomme, V., Stroobant, V., Thielemans, K., Corthals, J., Luiten, R., Eggermont, A. M., Boon, T. & van der, B. P. (1999) J. Exp. Med 189, 767-778. Dengjel J, Schoor O, Fischer R, Reich M, Kraus M, Muller M, Kreymborg K, Altenberend F, Brandenburg J, Kalbacher H, Brock R, Driessen C, Rammensee HG, Stevanovic S. Autophagy promotes MHC class II presentation of peptides from intracellular source proteins. Proc Natl Acad Sci U S A. 2005 May 31;102(22):7922-7.). Other strategies use fusion proteins which contain lysosomal target sequences. Expressed in APCs, such fusion proteins direct the antigens into the class II processing compartment (Marks, M. S., Roche, P. A., van Donselaar, E., Woodruff, L., Peters, P. J. & Bonifacino, J. S. (1995) J. Cell Biol. 131, 351-369, Rodriguez, F., Harkins, S., Redwine, J. M., de Pereda, J. M. & Whitton, J. L. (2001) J. Virol. 75, 10421-10430).

In order for the proteins to be recognised by the cytotoxic T-lymphocytes as tumour-specific antigen, and in order to be used in a therapy, particular prerequisites must be fulfilled. The antigen should be expressed mainly by tumour cells and not by normal healthy tissues or in rather small amounts. It is furthermore desirable, that the respective antigen is not only present in one type of tumour, but also in high concentrations (e.g. copy numbers per cell). Essential is the presence of epitopes in the amino acid sequence of the antigen, since such peptide ("immunogenic peptide") that is derived from a tumour associated antigen should lead to an in vitro or in vivo T-cell-response.

Approximately 30% of patients suffer from metastatic disease at presentation and another 25% of patients present with locally advanced tumour. 40% of individuals undergoing surgical resection will eventually develop metastasis. Among individuals with metastatic disease, approximately 75% exhibit lung metastasis, 36% have lymph node and/or soft tissue involvement, 20% have bone involvement, and 18% have liver involvement. The 5-year survival rates vary depending on the Robson staging class. All together, RCC remains fatal in nearly 80% of patients (Senn HJ, Drings P, Glaus A, Jungi WF, Pralle HB, Sauer R, and Schlag PM. Checkliste Onkologie, 5th edition. Georg Thieme Verlag, Stuttgart/New York (2001), Vokes EE, and Golomb HM. Oncologic Therapies, 2nd edition. Springer-Verlag, Berlin/Heidelberg (2003)).

The classification of renal cell carcinoma is being done according to TNM (Guinan P. TNM Staging of Renal Cell carcinoma. Presented at 'Diagnosis and prognosis of Renal Cell Carcinoma: 1997 Workshop', Rochester, Minnesota, March21-22, Communication of the UICC - Union Internationale Contre le Cancer, and AJCC - American Joint Committee on Cancer, published by ASC - American Society Cancer (1997), Communication of the UICC) see tables A and B, below.

**Table A: TNM Classification of Renal Cell Carcinoma**

| | | | |
|---|---|---|---|
| T1 | <= 7.0 cm, limited to the kidney | N1 | single regional lymph node |
| T2 | > 7.0 cm, limited to the kidney | N2 | more than one regional lymph node |
| T3 | into major veins, adrenal or perinephric invasion | M0 | no distant metastasis |
| T4 | invades beyond Gerota fascia | M1 | distant metastasis |

**Staging AJCC TNM classification**

| | | | |
|---|---|---|---|
| Stage I | T1 | N0 | M0 |
| Stage II | T2 | N0 | M0 |
| Stage III | T1 | N1 | M0 |
| | T2 | N1 | M0 |
| | T3 | N0,N1 | M0 |
| Stage IV | T4 | N0,N1 | M0 |
| | AnyT | N2 | M0 |
| | AnyT | Any N | M1 |

**Table B: Robson staging of Renal Cell Carcinoma and 5-year survival**

| **Robson staging class** | **5-year survival rates*** |
|---|---|
| Stage I/II | 75% - 86% |
| Stage III | 41% - 64% |
| Stage IV (T4) | 15% - 18% |
| Stage IV (M1) | 0% - 3% |

| | |
|---|---|
| * American Foundation for Urologic Disease | |

The standard treatment for RCC is radical nephrectomy (for all stages). Radiation therapy may be used to reduce the cancer's spread, but renal cell carcinomas are often resistant to radiation. Hormonal therapy may reduce the growth of the tumour in some cases (less than 10%). To date chemotherapy has not demonstrated any significant activity in this disease. Vinblastine, 5-FU (5-fluorouracil) and floxuridane (FUDR) are the chemotherapy drugs that have been studied most, but only 5-FU and its metabolite FUDR have demonstrated a 10-12% activity rate (Vokes EE, and Golomb HM. Oncologic Therapies, 2nd edition. Springer-Verlag, Berlin/Heidelberg (2003)). The combination of gemcitabine and 5-FU resulted in a 17% response rate.

Immunological treatments such as Interferon alpha (IFNα) or Interleukin-2 (IL-2) have been evaluated in recent years by regulatory authorities in the USA and Europe for the treatment of advanced RCC. High dose IL-2 treatment is up to date still the only immunological regimen being approved by the FDA. IFNα monotherapy was initially reported to have a 25-30% response rate but many additional trials have suggested a true response rate of only about 10% (Vokes EE, and Golomb HM. Oncologic Therapies, 2nd edition. Springer-Verlag, Berlin/Heidelberg (2003)). IL-2 appears to have a similar overall response rate compared to IFNα with approximately 5% of the patients achieving durable complete remissions (Rosenberg 1987). A recent meta-analysis of more than 6,000 patients with advanced RCC came to the conclusion that, on average, only a 12,9% clinical response rate can be reached with cytokine therapy e.g., IFN-alpha, high dose IL-2 bolus injections, or IL-2 inhalation). The same analysis showed 4.3% response for placebo, and 2.5% response in non-immunotherapy control arms (Cochrane Database Syst Rev. 2000;(3):CD001425. Immunotherapy for advanced renal cell cancer. Coppin C, Porzsolt F, Awa A, Kumpf J, Coldman A, Wilt T).

Although new therapies showed clinical efficacy in many tumour entities and have been approved in recent years, the survival rates for renal cell carcinoma have not significantly changed within the last decade. The current available systemic treatment options, chemotherapy as well as immunological treatments, have shown relatively poor efficacy results and more importantly are limited by significant systemic toxicity. Consequently, there is a substantial unmet medical need for new treatment options in renal cell carcinoma.

T-helper cells play an important role in orchestrating the effector function of CTLs in anti-tumour immunity. T-helper cell epitopes that trigger a T-helper cell response of the TH1 type support effector functions of CD8-positive Killer T-cells, which include cytotoxic functions directed against tumour cells displaying tumour-associated peptide/MHC complexes on their cell surfaces. In this way tumour-associated T-helper cell peptide epitopes, alone or in combination with other tumour-associated peptides, can serve as active pharmaceutical ingredients of vaccine compositions which stimulate anti-tumour immune responses.

The major task in the development of a tumour vaccine is therefore the identification and characterisation of novel tumour associated antigens and immunogenic T-helper epitopes derived thereof, that can be recognised by CD8-positive T-cells, or CD4-positive T-cells, in particular CD4-positive T-cells of the T_{H1} type. It is therefore an object of the present invention to provide novel amino acid sequences for such peptides that have the ability to bind to a molecule of the human major histocompatibility complex (MHC) class-I (HLA class I). It is a further object of the present invention, to provide an effective anti-cancer vaccine that is, at least in part, based on said novel peptides.

According to the present invention, the first object is solved by providing a tumour associated peptide according to SEQ ID No. 2 of the attached sequence listing, or a peptide according to SEQ ID No. 2, wherein the side chains of one or two amino acid residues are altered, wherein said peptide has the ability to bind to a molecule of the human major histocompatibility complex (MHC) class-I (HLA class I) HLA-A*D2.

In the present invention, the inventors demonstrate that it is possible to isolate and characterize peptides binding to HLA class I or II molecules directly from mammalian tumours, preferentially human tumours, preferably renal cell carcinomas.

The present invention describes peptides that stem from antigens associated with tumourigenesis, and have the ability to bind sufficiently to HLA class II molecules for triggering an immune response of human leukocytes, especially lymphocytes, especially T lymphocytes, especially CD4-positive T lymphocytes, especially CD4-positive T lymphocytes mediating T_{H1}-type immune responses.

The present invention provides peptides that stem from antigens HLA-A*O2 associated with tumourigenesis, and have the ability to bind sufficiently to HLA class 1 HLA-A*O2 molecules for triggering an immune response of human leukocytes, especially lymphocytes, especially T lymphocytes, especially CD8-positive cytotoxic T-lymphocytes.

The peptides stem from tumour-associated antigens, especially tumour-associated antigens with functions in, e.g., proteolysis, angiogenesis, cell growth, cell cycle regulation, cell division, regulation of transcription, regulation of translation, tissue invasion, including, e.g., tumour-associated peptides from matrix-metalloproteinase 7 (MMP7; SEQ ID No. 1) and Apolipoprotein L1 (APOL1; SEQ ID No. 4).

In the present invention the inventors provide conclusive evidence that tumour-associated peptides sufficiently binding promiscuously to HLA-class II molecules, especially those HLA class II alleles genetically encoded by HLA DR loci of the human genome, are able to elicit immune responses mediated by human CD4-positive T-cells. CD4-positive T-cells were isolated from human peripheral blood, demonstrating that the claimed peptides are suitable for triggering T-cell responses of the human immune system against selected peptides of the tumour cell peptidome. As exemplified below with a peptide from MMP7 (SEQ ID No. 1), this promiscuously HLA-DR-binding, tumour-associated peptide was found to be recognized by CD4-positive T-cells.

Similarly, it was found that tumour-associated peptides sufficiently binding to HLA-class I molecules are able to elicit immune responses mediated by human CD8-positive cytotoxic T-lymphocytes, also demonstrating that the claimed peptides are suitable for triggering responses of the human immune system against selected peptides of the tumour cell peptidome.

As peptides can be synthesized chemically and can be used as active pharmaceutical ingredients of pharmaceutical preparations, the peptides provided by the present invention can be used for immunotherapy, preferentially cancer immunotherapy.

In order to identify HLA class I or II ligands from TAA for the development of peptide-based immunotherapy, the inventors attempted to isolate peptides directly from solid tumours, in particular from renal cell carcinoma (RCC) (see examples, below).

The reasons for focusing on RCC to demonstrate technical proof of concept were the following: Around 150,000 people worldwide are newly diagnosed with RCC each year, the disease is associated with a high mortality rate, which results in approximately 78,000 deaths per annum (Pavlovich, C.P. and L.S. Schmidt. 2004. Searching for the hereditary causes of renal-cell carcinoma. Nat. Rev. Cancer 4:381-393). If metastases are diagnosed, the one-year survival rate decreases to approximately 60% (Jemal, A., R.C. Tiwari, T. Murray, A. Ghafoor, A. Samuels, E. Ward, E.J. Feuer, and M.J. Thun. 2004. Cancer statistics, 2004. CA Cancer J. Clin. 54:8-29), underlining the high unmet medical need in this indication. Due to the fact that RCC seems to be an immunogenic tumour (Oliver RTD, Mehta A, Barnett MJ. A phase 2 study of surveillance in patients with metastatic renal cell carcinoma and assessment of response of such patients to therapy on progression. Mol Biother. 1988;1:14-20. Gleave M, Elhilali M, Frodet Y, et al. Interferon gamma-1b compared with placebo in metastatic renal cell carcinoma. N Engl J Med. 1998;338:1265), as indicated by the existence of tumorreacting and tumor-infiltrating CTL (Finke, J.H., P. Rayman, J. Alexander, M. Edinger, R.R. Tubbs, R. Connelly, E. Pontes, and R. Bukowski. 1990. Characterization of the cytolytic activity of CD4+ and CD8+ tumor-infiltrating lymphocytes in human renal cell carcinoma. Cancer Res. 50:2363-2370), clinical trials have been initiated to develop peptide-based anti-tumour vaccinations (Wierecky J, Mueller M, Brossart P. Dendritic cell-based cancer immunotherapy targeting MUC-1. Cancer Immunol Immunother. 2005 Apr 28). However, due to the lack of helper T-cell epitopes from TAA, molecularly defined vaccines usually comprise peptides functioning as class I ligands only.

The second object of the present invention is solved by providing a pharmaceutical preparation, preferably in the form of a vaccine, that is effective against cancer cells, in particular cells of solid tumours, comprising an effective amount of a peptide according to the invention, or comprising a nucleic acid encoding such a peptide. The vaccine can furthermore contain additional peptides and/or excipients to be more effective, as will be further explained below.

The peptide or peptide-encoding nucleic acid can also constitute a tumour or cancer vaccine. It may be administered directly into the patient, into the affected organ or systemically, or applied ex vivo to cells derived from the patient or a human cell line which are subsequently administered to the patient, or used in vitro to select a subpopulation from immune cells derived from the patient, which are then re-administered to the patient.

In a first aspect of the invention a peptide is provided, having an amino acid sequence according to SEQ ID No. 2 (VMAGDIYSV).

As described herein below, the peptides that form the basis of the present invention have all been identified as being presented by MHC class I bearing cells (RCC). Thus, these particular peptides as well as other peptides containing the sequence (i.e. derived peptides) all elicit a specific T-cell response, although the extent to which such response will be induced might vary from individual peptide to peptide. Differences, for example, could be caused due to mutations in said peptides (see below). The person of skill in the present art is well aware of methods that can be applied in order to determine the extent to which a response is induced by an individual peptide, in particular with reference to the examples herein and the respective literature.

Preferably, a peptide according to the present invention consists of an amino acid sequence according to SEQ ID No. 2.

"Consisting essentially of" shall mean that a peptide, in addition to the sequence according to any of SEQ ID No. 1 to SEQ ID No. 11 or a variant thereof, contains additional N- and/or C-terminally located stretches of amino acids that are not necessarily forming part of the peptide that functions as core sequence of the peptide comprising the binding motif and as an immunogenic T-helper epitope.

Nevertheless, these stretches can be important in order to provide for an efficient introduction of the peptide according to the present invention into the cells. In one embodiment of the present invention, the peptide of the present invention comprises the 80 N-terminal amino acids of the HLA-DR antigen-associated invariant chain (p33, in the following "Ii") as derived from the NCBI, GenBank Accession-number X00497 (Strubin, M., Mach, B. and Long, E.O. The complete sequence of the mRNA for the HLA-DR-associated invariant chain reveals a polypeptide with an unusual transmembrane polarity EMBO J. 3 (4), 869-872 (1984)).

By a "variant" of the given amino acid sequence we mean that the side chains of, for example, one or two of the amino acid residues are altered (for example by replacing them with the side chain of another naturally occurring amino acid residue or some other side chain) such that the peptide is still able to bind to an HLA molecule in substantially the same way as a peptide consisting of the given amino acid sequence. For example, a peptide may be modified so that it at least maintains, if not improves, the ability to interact with and bind to a suitable MHC molecule, such as HLA-DRB1 in the case of HLA class II molecules, or HLA-A2 in the case of class I molecules, and so that it at least maintains, if not improves, either the ability to generate activated CTL which can recognise and kill cells which aberrantly express a polypeptide which contains an amino acid sequence as defined in the aspects of the invention, or the ability to stimulate helper T-cells which can provide help to CD8 positive T-cells or directly attack target cells by secreting cytokines. As can be derived from the database as described in the following, certain positions of HLA-DR binding peptides are typically anchor residues forming a core sequence fitting to the binding motif of the HLA binding groove. Modifications of these and other residues involved in binding HLA-DR may enhance binding without altering CTL recognition.

Those amino acid residues that are not essential to interact with the T-cell receptor can be modified by replacement with another amino acid whose incorporation does not substantially affect T-cell reactivity and does not eliminate binding to the relevant MHC. Thus, apart from the proviso given, the peptide of the invention may be any peptide (by which term we include oligopeptide or polypeptide) which includes the amino acid sequences or a portion or variant thereof as given.

It is furthermore known for MHC-class II presented peptides that these peptides are composed of a "core sequence" having a certain HLA-specific amino acid motif and, optionally, N-and/or C-terminal extensions which do not interfere with the function of the core sequence (i.e. are deemed as irrelevant for the interaction of the peptide and the T-cell). The N- and/or C-terminal extensions can be between 1 to 10 amino acids in length, respectively. Thus, a preferred peptide exhibits an overall length of between 9 and 100, preferably between 9 and 30, and most preferred between 9 and 16 amino acids. These peptide can be used either directly in order to load MHC class II molecules or the sequence can be cloned into the vectors according to the description herein below. As these peptides form the final product of the processing of larger peptides within the cell, longer peptides can be used as well. The peptides may be of any size, but typically they may be less than 100,000 Da in molecular weight, preferably less than 50,000 Da, more preferably less than 10,000 Da and typically about 5,000 Da. In terms of the number of amino acid residues, the peptides may have fewer than 1000 residues, preferably fewer than 500 residues, more preferably fewer than 100 residues.

In the main aspect of the present invention, similar to the situation as explained above for MHC class II molecules, the peptides of the invention may be used to trigger an MHC class I specific response . A preferred MHC class I specific peptide exhibits an overall length of between 9 and 16, preferably between 9 and 12 amino acids. It shall be understood that those peptides might be used (for example in a vaccine) as longer peptides, similar to MHC class II peptides. Methods to identify MHC class I specific "Core sequences" having a certain HLA-specific amino acid motif for HLA class I-molecules are known to the person of skill and can be predicted, for example, by the computer programs PAProC (http://www.uni-tuebingen.de/uni/kxi/) and SYFPEITHI (http://www.syfpeithi.de) (see below).

The peptides of the invention are particularly useful in immunotherapeutic methods for enabling T-cells to recognize cells which aberrantly express polypeptides that form the basis for the present peptides of the invention. Since these specific peptides consisting of the given amino acid sequences bind to HLA class I HLA-A*02 molecules it is preferred that the peptides of the invention are ones which bind HLA class I HLA-A*02 molecules and when so bound the HLA-peptide complex is present on the surface of a suitable antigen-presenting cell, is capable of eliciting the stimulation of T-cells which recognise cells which aberrantly express a polypeptide comprising the given amino acid sequence.

If a peptide which is greater than around 12 amino acid residues is used directly to bind to a MHC molecule, it is preferred that the residues that flank the core HLA binding region are ones that do not substantially affect the ability of the peptide to bind specifically to the binding groove of the MHC molecule or to present the peptide to the T-cells. However, as already indicated above, it will be appreciated that larger peptides may be used, especially when encoded by a polynucleotide, since these larger peptides may be fragmented by suitable antigen-presenting cells.

Examples for peptides of MHC ligands, motifs, variants, as well as certain examples for N-and/or C-terminal extensions can be, for example, derived from the database SYFPEITHI (Rammensee H, Bachmann J, Emmerich NP, Bachor OA, Stevanovic S. SYFPEITHI: database for MHC ligands and peptide motifs. Immunogenetics. 1999 Nov; 50(3-4):213-9. Review.) at http://syfpeithi.bmi-heidelberg.com/ and the references as cited therein.

As non-limiting examples, certain peptides for HLA-DR in the database are K H K V **Y** A C E V T **H** O G L S S derived from Ig kappa chain 188-203 (Kovats et al. Eur J Immunol. 1997 Apr;27(4):1014-21); K V Q **W** K V **D** N A L Q S G N S derived from Ig kappa chain 145-159 (Kovats et al. Eur J Immunol. 1997 Apr;27(4):1014-21), L P R L I A F T S E H S **H** F derived from GAD65 270-283 (Endl et al. J Clin Invest. 1997 May 15;99(10):2405-15) or F F R M V I S **N** P **A** A T H Q D I D F L I derived from GAD65 556-575 (Endl et al. J Clin Invest. 1997 May 15;99(10):2405-15). In addition, peptides can also be derived from mutated sequences of antigens, such as in the case of A T G F K **Q S S K** A L Q R P V A S derived from bcr-abl 210 kD fusion protein (ten Bosch et al. Blood. 1996 Nov 1;88(9):3522-7), G **Y** K V **L** V L **N** P S V A A T derived from HCV-1 NS3 28-41 Diepolder et al. J Virol. 1997 Aug;71(8):6011-9), or F R K Q N P **D** I V I Q Y M D D L Y V G derived from HIV-1 (HXB2) RT 326-345 (van der Burg et al. J Immunol. 1999 Jan 1;162(1):152-60). All "anchor" amino acids (see Friede et al., Biochim Biophys Acta. 1996 Jun 7;1316(2):85-101; Sette et al. J Immunol. 1993 Sep 15;151(6):3163-70.; Hammer et al. Cell. 1993 Jul 16;74(1):197-203., and Hammer et al. J Exp Med. 1995 May 1;181(5):1847-55. As examples for HLA-DR4) have been indicated in bold, the putative core sequences have been underlined.

All the above described peptides are encompassed by the term "variants" of the given amino acid sequence.

By "peptide" the inventors include not only molecules in which amino acid residues are joined by peptide (-CO-NH-) linkages but also molecules in which the peptide bond is reversed. Such retro-inverso peptidomimetics may be made using methods known in the art, for example such as those described in Meziere et al (1997) J. Immunol. 159,3230-3237, This approach involves making pseudopeptides containing changes involving the backbone, and not the orientation of side chains. Meziere et al (1997) show that, at least for MHC class II and T helper cell responses, these pseudopeptides are useful. Retro-inverse peptides, which contain NH-CO bonds instead of CO-NH peptide bonds, are much more resistant to proteolysis.

Typically, the peptide of the invention is one which, if expressed in an antigen presenting cell, may be processed so that a fragment is produced which is able to bind to an appropriate MHC molecule and may be presented by a suitable cell and elicit a suitable T-cell response. It will be appreciated that a fragment produced from the peptide may also be a peptide of the invention. Conveniently, the peptide of the invention contains a portion which includes the given amino acid sequence and a further portion which confers some desirable property. For example, the further portion may include a further T-cell epitope (whether or not derived from the same polypeptide as the first T-cell epitope-containing portion) or it may include a carrier protein or peptide. Thus, in one embodiment the peptide of the invention is a fusion protein of a protein fragment and one or more inventive amino acid sequences.

In a particularly preferred embodiment, the peptides of the invention include the amino acid sequences of the invention and at least one further T-cell epitope wherein the further T-cell epitope is able to facilitate the production of a T-cell response directed at the type of tumour that aberrantly expresses a tumour-associated antigen. Thus, the peptides of the invention include so-called "beads on a string" polypeptides which can also be used as vaccines. Such peptides can be spaced apart by chemical linkers, which might contain amino acids (such as G-stretches), but which can - additionally or alternatively - comprise chemical linking groups (i.e. not having a function except for providing a particular spacing).

By "aberrantly expressed" we include the meaning that the polypeptide is over-expressed compared to normal levels of expression or that the gene is silent in the tissue from which the tumour is derived but in the tumour it is expressed. By "over-expressed" we mean that the polypeptide is present at a level at least 1.2 x that present in normal tissue; preferably at least 2 x and more preferably at least 5 x or 10 x the level present in normal tissue.

Peptides (at least those containing peptide linkages between amino acid residues) may be synthesised by the Fmoc-polyamide mode of solid-phase peptide synthesis as disclosed by Lu et al (1981) J. Org. Chem. 46, 3433-3436, and references therein. Temporary N-amino group protection is afforded by the 9-fluorenylmethyloxycarbonyl (Fmoc) group. Repetitive cleavage of this highly base-labile protecting group is achieved by using 20 % piperidine in N,N-dimethylformamide. Side-chain functionalities may be protected as their butyl ethers (in the case of serine threonine and tyrosine), butyl esters (in the case of glutamic acid and aspartic acid), butyloxycarbonyl derivative (in the case of lysine and histidine), trityl derivative (in the case of cysteine) and 4-methoxy-2,3,6-trimethylbenzenesulphonyl derivative (in the case of arginine). Where glutamine or asparagine are C-terminal residues, use is made of the 4,4'-dimethoxybenzhydryl group for protection of the side chain amido functionalities. The solid-phase support is based on a polydimethyl-acrylamide polymer constituted from the three monomers dimethylacrylamide (backbone-monomer), bisacryloylethylene diamine (cross linker) and acryloylsarcosine methyl ester (functionalising agent). The peptide-to-resin cleavable linked agent used is the acid-labile 4-hydroxymethyl-phenoxyacetic acid derivative. All amino acid derivatives are added as their preformed symmetrical anhydride derivatives with the exception of asparagine and glutamine, which are added using a reversed N,N-dicyclohexyl-carbodiimide/lhydroxybenzotriazole mediated coupling procedure. All coupling and deprotection reactions are monitored using ninhydrin, trinitrobenzene sulphonic acid or isotin test procedures. Upon completion of synthesis, peptides are cleaved from the resin support with concomitant removal of side-chain protecting groups by treatment with 95% trifluoroacetic acid containing a 50 % scavenger mix. Scavengers commonly used are ethanedithiol, phenol, anisole and water, the exact choice depending on the constituent amino acids of the peptide being synthesised.

Trifluoroacetic acid is removed by evaporation in vacuo, with subsequent trituration with diethyl ether affording the crude peptide. Any scavengers present are removed by a simple extraction procedure which on lyophilisation of the aqueous phase affords the crude peptide free of scavengers. Reagents for peptide synthesis are generally available from Calbiochem-Novabiochem (UK) Ltd, Nottingham NG7 2QJ, UK.

Purification may be effected by any one, or a combination of, techniques such as size exclusion chromatography, ion-exchange chromatography and (usually) reverse-phase high performance liquid chromatography.

Analysis of peptides may be carried out using thin layer chromatography, reverse-phase high performance liquid chromatography, amino-acid analysis after acid hydrolysis and by fast atom bombardment (FAB) mass spectrometric analysis, as well as MALDI and ESI-Q-TOF mass spectrometric analysis:

A further aspect of the invention provides a nucleic acid (e.g. polynucleotide) encoding a peptide of the invention. The nucleic acid according to the present invention may be DNA, cDNA, PNA, CNA, RNA or combinations thereof and it may or may not contain introns as long as it codes for the peptide. Of course, only peptides which contain naturally occurring amino acid residues joined by naturally occurring peptide bonds are encodable by a polynucleotide. A still further aspect of the invention provides an expression vector capable of expressing a polypeptide according to the invention.

A variety of methods have been developed to operably link polynucleotides, especially DNA, to vectors for example via complementary cohesive termini. For instance, complementary homopolymer tracts can be added to the DNA segment to be inserted to the vector DNA. The vector and DNA segment are then joined by hydrogen bonding between the complementary homopolymeric tails to form recombinant DNA molecules.

Synthetic linkers containing one or more restriction sites provide an alternative method of joining the DNA segment to vectors. The DNA segment, generated by endonuclease restriction digestion as described earlier, is treated with bacteriophage T4 DNA polymerase or E. coli DNA polymerase I, enzymes that remove protruding, 3'-single-stranded termini with their 3'-5'-exonucleolytic activities, and fill in recessed 3'-ends with their polymerising activities.

The combination of these activities therefore generates blunt-ended DNA segments. The blunt-ended segments are then incubated with a large molar excess of linker molecules in the presence of an enzyme that is able to catalyse the ligation of blunt-ended DNA molecules, such as bacteriophage T4 DNA ligase. Thus, the products of the reaction are DNA segments carrying polymeric linker sequences at their ends. These DNA segments are then cleaved with the appropriate restriction enzyme and ligated to an expression vector that has been cleaved with an enzyme that produces termini compatible with those of the DNA segment.

Synthetic linkers containing a variety of restriction endonuclease sites are commercially available from a number of sources including International Biotechnologies Inc, New Haven, CN, USA.

A desirable way to modify the DNA encoding the polypeptide of the invention is to use the polymerase chain reaction as disclosed by Saiki et al (1988) Science 239,487-491. This method may be used for introducing the DNA into a suitable vector, for example by engineering in suitable restriction sites, or it may be used to modify the DNA in other useful ways as is known in the art. In this method the DNA to be enzymatically amplified is flanked by two specific primers which themselves become incorporated into the amplified DNA. The said specific primers may contain restriction endonuclease recognition sites which can be used for cloning into expression vectors using methods known in the art.

The DNA (or in the case of retroviral vectors, RNA) is then expressed in a suitable host to produce a polypeptide comprising the compound of the invention. Thus, the DNA encoding the polypeptide constituting the compound of the invention may be used in accordance with known techniques, appropriately modified in view of the teachings contained herein, to construct an expression vector, which is then used to transform an appropriate host cell for the expression and production of the polypeptide of the invention. Such techniques include those disclosed in US Patent Nos. 4,440,859 issued 3 April 1984 to Rutter et al, 4,530,901 issued 23 July 1985 to Weissman, 4,582,800 issued 15 April 1986 to Crowl, 4,677,063 issued 30 June 1987 to Mark et al, 4,678,751 issued 7 July 1987 to Goeddel, 4,704,362 issued 3 November 1987 to Itakura et al, 4,710,463 issued 1 December 1987 to Murray, 4,757,006 issued 12 July 1988 to Toole, Jr. et al, 4,766,075 issued 23 August 1988 to Goeddel et al and 4,810,648 issued 7 March 1989 to Stalker.

The DNA (or in the case of retroviral vectors, RNA) encoding the polypeptide constituting the compound of the invention may be joined to a wide variety of other DNA sequences for introduction into an appropriate host. The companion DNA will depend upon the nature of the host, the manner of the introduction of the DNA into the host, and whether episomal maintenance or integration is desired.

Generally, the DNA is inserted into an expression vector, such as a plasmid, in proper orientation and correct reading frame for expression. If necessary, the DNA may be linked to the appropriate transcriptional and translational regulatory control nucleotide sequences recognised by the desired host, although such controls are generally available in the expression vector. The vector is then introduced into the host through standard techniques. Generally, not all of the hosts will be transformed by the vector. Therefore, it will be necessary to select for transformed host cells. One selection technique involves incorporating into the expression vector a DNA sequence, with any necessary control elements, that codes for a selectable trait in the transformed cell, such as antibiotic resistance.

Alternatively, the gene for such selectable trait can be on another vector, which is used to co-transform the desired host cell.

Host cells that have been transformed by the recombinant DNA of the invention are then cultured for a sufficient time and under appropriate conditions known to those skilled in the art in view of the teachings disclosed herein to permit the expression of the polypeptide, which can then be recovered.

Many expression systems are known, including bacteria (for example *E*. *coli* and *Bacillus subtilis),* yeasts (for example *Saccharomyces cerevisiae),* filamentous fungi (for example *Aspergillus),* plant cells, animal cells and insect cells. Preferably, the system can be RCC or Awells cells.

A promoter is an expression control element formed by a DNA sequence that permits binding of RNA polymerase and transcription to occur. Promoter sequences compatible with exemplary bacterial hosts are typically provided in plasmid vectors containing convenient restriction sites for insertion of a DNA segment of the present invention. Typical prokaryotic vector plasmids are pUC18, pUC19, pBR322 and pBR329 available from Biorad Laboratories, (Richmond, CA, USA) and pTrc99A and pKK223-3 available from Pharmacia, Piscataway, NJ, USA.

A typical mammalian cell vector plasmid is pSVL available from Pharmacia, Piscataway, NJ, USA. This vector uses the SV40 late promoter to drive expression of cloned genes, the highest level of expression being found in T antigen-producing cells, such as COS-1 cells. An example of an inducible mammalian expression vector is pMSG, also available from Pharmacia. This vector uses the glucocorticoid-inducible promoter of the mouse mammary tumour virus long terminal repeat to drive expression of the cloned gene. Useful yeast plasmid vectors are pRS403-406 and pRS413-416 and are generally available from Stratagene Cloning Systems, La Jolla, CA 92037, USA. Plasmids pRS403, pRS404, pRS405 and pRS406 are Yeast Integrating plasmids (YIps) and incorporate the yeast selectable markers HIS3, TRP1, LEU2 and URA3. Plasmids pRS413-416 are Yeast Centromere plasmids (Ycps). Other vectors and expression systems are well known in the art for use with a variety of host cells.

The present invention also relates to a host cell transformed with a polynucleotide vector construct of the present invention. The host cell can be either prokaryotic or eukaryotic. Bacterial cells may be preferred prokaryotic host cells in some circumstances and typically are a strain of E. coli such as, for example, the E. coli strains DH5 available from Bethesda Research Laboratories Inc., Bethesda, MD, USA, and RR1 available from the American Type Culture Collection (ATCC) of Rockville, MD, USA (No ATCC 31343). Preferred eukaryotic host cells include yeast, insect and mammalian cells, preferably vertebrate cells such as those from a mouse, rat, monkey or human fibroblastic and kidney cell lines. Yeast host cells include YPH499, YPH500 and YPH501 which are generally available from Stratagene Cloning Systems, La Jolla, CA 92037, USA. Preferred mammalian host cells include Chinese hamster ovary (CHO) cells available from the ATCC as CCL61, NIH Swiss mouse embryo cells NIH/3T3 available from the ATCC as CRL 1658, monkey kidney-derived COS-1 cells available from the ATCC as CRL 1650 and 293 cells which are human embryonic kidney cells. Preferred insect cells are Sf9 cells which can be transfected with baculovirus expression vectors.

Transformation of appropriate cell hosts with a DNA construct of the present invention is accomplished by well known methods that typically depend on the type of vector used. With regard to transformation of prokaryotic host cells, see, for example, Cohen et al (1972) Proc. Natl. Acad. Sci. USA 69,2110 and Sambrook et al (1989) Molecular Cloning, A Laboratory Manual, Cold Spring Harbor Laboratory, Cold Spring Harbor, NY. Transformation of yeast cells is described in Sherman et al (1986) Methods In Yeast Genetics, A Laboratory Manual, Cold Spring Harbor, NY. The method of Beggs (1978) Nature 275,104-109 is also useful. With regard to vertebrate cells, reagents useful in transfecting such cells, for example calcium phosphate and DEAE-dextran or liposome formulations, are available from Stratagene Cloning Systems, or Life Technologies Inc., Gaithersburg, MD 20877, USA. Electroporation is also useful for transforming and/or transfecting cells and is well known in the art for transforming yeast cell, bacterial cells, insect cells and vertebrate cells.

Successfully transformed cells, i.e. cells that contain a DNA construct of the present invention, can be identified by well known techniques. For example, cells resulting from the introduction of an expression construct of the present invention can be grown to produce the polypeptide of the invention. Cells can be harvested and lysed and their DNA content examined for the presence of the DNA using a method such as that described by Southern (1975) J. Mol. Biol. 98,503 or Berent et al (1985) Biotech. 3,208. Alternatively, the presence of the protein in the supernatant can be detected using antibodies as described below.

In addition to directly assaying for the presence of recombinant DNA, successful transformation can be confirmed by well known immunological methods when the recombinant DNA is capable of directing the expression of the protein. For example, cells successfully transformed with an expression vector produce proteins displaying appropriate antigenicity. Samples of cells suspected of being transformed are harvested and assayed for the protein using suitable antibodies. Thus, in addition to the transformed host cells themselves, the present invention also contemplates a culture of those cells, preferably a monoclonal (clonally homogeneous) culture, or a culture derived from a monoclonal culture, in a nutrient medium.

It will be appreciated that certain host cells of the invention are useful in the preparation of the peptides of the invention, for example bacterial, yeast and insect cells. However, other host cells may be useful in certain therapeutic methods. For example, antigen-presenting cells, such as dendritic cells, may usefully be used to express the peptides of the invention such that they may be loaded into appropriate MHC molecules.

Preferred host cells are recombinant RCC or Awells cells. Preferred is a method of producing a tumour associated peptide according to the present invention, the method comprising culturing the host cell according to the present invention, and isolating the peptide from the host cell or its culture medium, according to standard methods.

A further aspect of the invention provides a method of producing a peptide for oral, rectal, nasal or lingual uptake, intravenous (i.v.) injection, sub-cutaneous (s.c.) injection, intradermal (i.d.) injection, intraperitoneal (i.p.) injection, intramuscular (i.m.) injection. Preferred ways of peptide injection are s.c., i.d., i.p., i.m., and i.v. Preferred ways of DNA injection are i.d., i.m., s.c., i.p. and i.v. Doses of between 0.1 and 500 mg of peptide or DNA may be given, as is also outlined below.

A further aspect of the invention relates to the use of a tumour associated peptide according to the invention, a nucleic acid according to the invention or an expression vector according to the invention in medicine.

The object of the present invention, in a further aspect thereof, is solved by a pharmaceutical composition that contains at least one tumour associated peptide according to SEQ ID No. 2 according to the invention, a nucleic acid according to the invention or an expression vector according to the invention, and a pharmaceutically acceptable carrier. This composition is used for parenteral administration, such as subcutaneous, intradermal, intraperitoneal, intravenous, intramuscular or oral administration. For this, the peptides are dissolved or suspended in a pharmaceutically acceptable, preferably aqueous carrier. In addition, the composition can contain excipients, such as buffers, binding agents, blasting agents, diluents, flavours, lubricants, etc.. The peptides can also be administered together with immune stimulating substances, such as cytokines. An extensive listing of excipients that can be used in such a composition, can be, for example, taken from A. Kibbe, Handbook of Pharmaceutical Excipients, 3. Ed., 2000, American Pharmaceutical Association and pharmaceutical press. The composition can be used for a prevention, prophylaxis and/or therapy of adenomateous or cancerous diseases.

The pharmaceutical preparation, containing at least one of the peptides of the present invention according to SEQ ID No. 2, a nucleic acid according to the invention or an expression vector according to the invention, is administered to a patient that suffers from an adenomateous or cancerous disease that is associated with the respective peptide or antigen. By this, a T-cell-mediated immune response can be triggered. The pharmaceutical composition according to the present invention preferably further comprises at least one additional tumour associated peptide comprising a sequence according to any of SEQ ID No. 3 to SEQ ID No. 10, a respective nucleic acid or a respective expression vector.

In general, the peptides that are present in the pharmaceutical composition according to the invention have the same properties as described above for peptides of the present invention according to SEQ ID No. 2. they can have an overall length of between 9 and 100, preferably between 9 and 30, and most preferred between 9 and 16 amino acids. Furthermore, at least one peptide according to any of SEQ ID No. 1 to SEQ ID No. 11 can include non-peptide bonds. Furthermore, the respective nucleic acids can encode for between 9 and 100, preferably between 9 and 30, and most preferred between 9 and 16 amino acids.

Preferred is a pharmaceutical composition according to the invention that comprises (in particular tumour associated) peptides consisting of amino acid sequences according to SEQ ID No. 1 and SEQ ID No. 2 and SEQ ID No. 3 to SEQ ID No. 11.

Preferred is a pharmaceutical composition according to the invention, wherein the amount of (in particular tumour associated) peptide(s), of nucleic acid(s) according to the invention or expression vector(s) according to the invention as present in said composition is/are tissue, cancer, and/or patient-specific.
The peptide may also be tagged, or be a fusion protein, or be a hybrid molecule.
The peptide may be substantially pure, or combined with an immune-stimulating adjuvant, or used in combination with immune-stimulatory cytokines, or be administered with a suitable delivery system, for example liposomes. Other suitable adjuvants include Aquila's QS21 stimulon (Aquila Biotech, Worcester, MA, USA) which is derived from saponin, mycobacterial extracts and synthetic bacterial cell wall mimics, and proprietory adjuvants such as Ribi's Detox. Quil A, another saponin derived adjuvant, may also be used (Superfos, Denmark). Other adjuvants such as Freund's may also be useful. It may also be useful to give the peptide conjugated to keyhole limpet hemocyanin (KLH) or mannan (see WO 95/18145 and Longenecker et al (1993) Ann. NY Acad. Sci. 690,276-291). Since an adjuvans is defined as a substance enhancing the immune response to an antigen (MedlinePlus® Medical Dictionary, NIH) other substances with this function may be used, including but not limited to toll-like receptor agonists (TLR agonists), preferably substances that interact agonistically with TLR 3, 7, 8, and 9, more preferably TLR 9, such as protamine-stabilising RNA, CpG-oligonucleotides, CpR-oligonucleotides, bacterial DNA, imidazoquinolines etc.
Other substances known in the art to be suitable to enhance an immune response include but are not limeted to inhibitors of inducible nitric oxide synthase (iNOS), arginase (ARG1), indoleamine-2,3-dioxygenase (IDO), vascular endothelial growth factor receptor 1 (VEGFR-1), vascular endothelial growth factor (VEGF), cyclooxygenase-2 (COX-2), TGF-beta receptor I (TGF-beta-RI). Such inhibitors may be, for example, monoclonal antibodies against said molecules or small molecules. Small molecules and monoclonal antibodies known in the art to have an inhibitory function towords the factors mentioned above, and thus an immune response enhancing effect are, for example, I-MT, NCX-4016, rofecoxib, celebrex, BEC, ABH, nor-NOHA, SB-505124, SD-208, LY580276, AMD3100, axitinib, bevacizumab, JSI-124, CPA-7, XL-999, ZD2171, pazopanib, CP-547632, and VEGF Trap.
Also, substances reducing the number of regulatory T-cells (CD 4+, CD25+, FoxP3+) are suitable as an adjuvans. These include, for example, but are not limited to cyclophosphamide (Cytoxan), ONTAK (denileukin diftitox), Sunitinib, anti-CTLA-4 (MDX-010, CP-675206), anti-CD25, anti-CCL22, and anti-GITR.

In another preferred embodiment the vaccine is a nucleic acid vaccine. It is known that inoculation with a nucleic acid vaccine, such as a DNA vaccine, encoding a polypeptide leads to a T-cell response. It may be administered directly into the patient, into the affected organ or systemically, or applied ex vivo to cells derived from the patient or a human cell line which are subsequently administered to the patient, or used in vitro to select a subpopulation from immune cells derived from the patient, which are then re-administered to the patient. If the nucleic acid is administered to cells in vitro, it may be useful for the cells to be transfected so as to co-express immune-stimulating cytokines, such as interleukin-2 or GM-CSF.
The nucleic acid vaccine may also be administered with an adjuvant such as BCG or alum. However, it is preferred if the nucleic acid vaccine is administered without adjuvant.

The polynucleotide may be substantially pure, or contained in a suitable vector or delivery system. Suitable vectors and delivery systems include viral, such as systems based on adenovirus, vaccinia virus, retroviruses, herpes virus, adeno-associated virus or hybrids containing elements of more than one virus. Non-viral delivery systems include cationic lipids and cationic polymers as are well known in the art of DNA delivery. Physical delivery, such as via a "gene-gun" may also be used. The peptide or peptide encoded by the nucleic acid may be a fusion protein, for example with an epitope from tetanus toxoid which stimulates CD4-positive T-cells.

Suitably, any nucleic acid administered to the patient is sterile and pyrogen free. Naked DNA may be given intramuscularly or intradermally or subcutaneously. The peptides may be given intramuscularly, intradermally or subcutaneously.

Conveniently, the nucleic acid vaccine may comprise any suitable nucleic acid delivery means. The nucleic acid, preferably DNA, may be naked (i.e. with substantially no other components to be administered) or it may be delivered in a liposome or as part of a viral vector delivery system.

It is believed that uptake of the nucleic acid and expression of the encoded polypeptide by professional antigen presenting cells such as dendritic cells may be the mechanism of priming of the immune response; however, dendritic cells may not be transfected but are still important since they may pick up expressed peptide from transfected cells in the tissue ("cross-priming", e.g., Thomas AM, Santarsiero LM, Lutz ER, Armstrong TD, Chen YC, Huang LQ, Laheru DA, Goggins M, Hruban RH, Jaffee EM. Mesothelin-specific CD8(+) T-cell responses provide evidence of in vivo cross-priming by antigen-presenting cells in vaccinated pancreatic cancer patients. J Exp Med. 2004 Aug 2;200(3):297-306).

It is preferred if the nucleic acid vaccine, such as DNA vaccine, is administered into the muscle, whilst peptide vaccines are preferably administered s.c. or i.d. It is also preferred if the vaccine is administered into the skin. The nucleic acid vaccine may be administered without adjuvant. The nucleic acid vaccine may also be administered with an adjuvant such as BCG or alum. Other suitable adjuvants include Aquila's QS21 stimulon (Aquila Biotech, Worcester, MA, USA) which is derived from saponin, mycobacterial extracts and synthetic bacterial cell wall mimics, and proprietory adjuvants such as Ribi's Detox. Quil A, another saponin derived adjuvant, may also be used (Superfos, Denmark). It is preferred if the nucleic acid vaccine is administered without adjuvant. Other adjuvants such as Freund's may also be useful. It may also be useful to give the peptide conjugated to keyhole limpet haemocyanin, preferably also with an adjuvant.

Polynucleotide-mediated immunisation therapy of cancer is described in Conry et al (1996) Seminars in Oncology 23,135-147; Condon et al (1996) Nature Medicine 2,1122-1127; Gong et al (1997) Nature Medicine 3,558-561; Zhai et al (1996) J. Immunol. 156,700-710; Graham et al (1996) Int J. Cancer 65,664-670; and Burchell et al (1996) pp 309-313 In: Breast Cancer, Advances in biology and therapeutics, Calvo et al (eds), John Libbey Eurotext.

It may also be useful to target the vaccine to specific cell populations, for example antigen presenting cells, either by the site of injection, use of targeting vectors and delivery systems, or selective purification of such a cell population from the patient and ex vivo administration of the peptide or nucleic acid (for example dendritic cells may be sorted as described in Zhou et al (1995) Blood 86,3295-3301; Roth et al (1996) Scand. J. Immunology 43,646-651). For example, targeting vectors may comprise a tissue-or tumour-specific promoter which directs expression of the antigen at a suitable place.

The invention in a further aspect thereof relates to a pharmaceutical composition, that contains one or more of said peptides according to the invention. This composition is used for parenteral administration, such as subcutaneous, intradermal, intramuscular or oral administration. For this, the peptides are dissolved or suspended in a pharmaceutically acceptable, preferably aqueous carrier. In addition, the composition can contain excipients, such as buffers, binding agents, blasting agents, diluents, flavours, lubricants, etc.. The peptides can also be administered together with immune stimulating substances, such as cytokines. An extensive listing of excipients that can be used in such a composition, can be, for example, taken from A. Kibbe, Handbook of Pharmaceutical Excipients, 3. Ed., 2000, American Pharmaceutical Association and pharmaceutical press. The composition can be used for a prevention, prophylaxis and/or therapy of adenomateous or cancerous diseases.

The pharmaceutical preparation, containing at least one of the peptides of the present invention according to SEQ ID No. 2 is administered to a patient that suffers from a adenomateous or cancerous disease that is associated with the respective peptide or antigen. By this, a CTL-specific immune response can be triggered.

In another aspect of the present invention, a combination of two or several peptides according to the present invention can be used as vaccine, either in direct combination or within the same treatment regimen. Furthermore, combinations with other peptides, for example MHC class I or II specific peptides can be used. The person of skill will be able to select preferred combinations of immunogenic peptides by testing, for example, the generation of T-cells in vitro as well as their efficiency and overall presence, the proliferation, affinity and expansion of certain T-cells for certain peptides, and the functionality of the T-cells, e.g. by analysing the IFN-γ production (see also examples below). Usually, the most efficient peptides are then combined as a vaccine for the purposes as described above.

A suitable vaccine will preferably contain between 1 and 20 peptides, more preferably 2, 3, 4, 5, 6, 7, 8, 9, 10 or 11 different peptides, further preferred 6, 7, 8, 9, 10 or 11 different peptides, and most preferably 11 different peptides. The length of the peptide for use in a cancer vaccine may be any suitable peptide. In particular, it may be a suitable 9-mer peptide or a suitable 7-mer or 8-mer or 10-mer or 11-mer peptide or 12-mer. Longer peptides may also be suitable, 9-mer or 10-mer peptides as described in the attached Table 1 are preferred for MHC class I-peptides.

The peptide(s) constitute(s) a tumour or cancer vaccine. It may be administered directly into the patient, into the affected organ or systemically, or applied ex vivo to cells derived from the patient or a human cell line which are subsequently administered to the patient, or used in vitro to select a subpopulation from immune cells derived from the patient, which are then re-administered to the patient. The peptide may also be conjugated to a suitable carrier such as keyhole limpet haemocyanin (KLH) or mannan (see WO 95/18145 and Longenecker et al (1993) Ann. NY Acad. Sci. 690,276-291). The peptide vaccine may be administered without adjuvant. The peptide vaccine may also be administered with an adjuvant such as BCG or alum. Other suitable adjuvants include Aquila's QS21 stimulon (Aquila Biotech, Worcester, MA, USA) which is derived from saponin, mycobacterial extracts and synthetic bacterial cell wall mimics, and proprietory adjuvants such as Ribi's Detox. Quil A, another saponin derived adjuvant, may also be used (Superfos, Denmark). Other adjuvants such as Freund's may also be useful. It may also be useful to give the peptide conjugated to keyhole limpet haemocyanin, preferably also with an adjuvant. Other adjuvants, such as those mentioned above, may be used. The peptide may also be tagged, or be a fusion protein, or be a hybrid molecule. The peptides whose sequence is given in the present invention are expected to stimulate CD8⁺ CTL. However, stimulation is more efficient in the presence of help provided by CD4⁺ T-cells. Thus, the fusion partner or sections of a hybrid molecule suitably provide epitopes which stimulate CD4⁺ T-cells. CD4⁺ stimulating epitopes are well known in the art and include those identified in tetanus toxoid.

In a particularly preferred embodiment of the peptide vaccine according to the invention, said vaccine is a multiple peptide tumour vaccine for treatment of renal cell carcinoma. Preferably, said vaccine comprises a set of tumour-associated peptides according to SEQ ID No. 1 to 10 which are located and have been identified on primary renal cancer cells. This set includes HLA class I and class II peptides. The peptide set can also contain at least one peptide, such as from HBV core antigen, used as a positive control peptide serving as immune marker to test the efficiency of the intradermal administration. In one particular embodiment, the vaccine consists of 11 individual peptides (according to SEQ ID No. 1 to 11) with between about 1500 µg to about 75 µg, preferably between about 1000 µg to about 750 µg and more preferred between about 500 µg to about 600 µg, and most preferred about 578 µg of each peptide, all of which may be purified by HPLC and ion exchange chromatography and appear as a white to off-white powder. The lyophilisate is preferably dissolved in sodium hydrogen carbonate, and is used for intradermal injection within 30 min after reconstitution at room temperature. According to the present invention, preferred amounts of peptides can vary between about 0.1 and 100 mg, preferably between about 0.1 to 1 mg, and most preferred between about 300 µg to 800 µg per 500 µl of solution. Herein, the term "about" shall mean +/- 10 percent of the given value, if not stated differently. The person of skill will be able to adjust the actual amount of peptide to be used based on several factors, such as, for example, the immune status of the individual patient and/or the amount of TUMAP that is presented in a particular type of cancer. The peptides of the present invention might be provided in other suitable forms (sterile solutions, etc.) instead of a lyophilisate.

Some of the peptides whose sequence is given in the present invention are expected to stimulate CD8-positive T-cells (CTL). However, stimulation is more efficient in the presence of help provided by CD4-positive T-cells. Thus, the fusion partner or sections of a hybrid molecule suitably provide epitopes which stimulate CD4-positive T-cells. CD4-positive stimulating epitopes are well known in the art and include those identified in tetanus toxoid or the peptide from MMP7 provided by this invention.

Finally, the vaccine according to the invention can be dependent from the specific type of cancer that the patient to be treated is suffering from as well as the status of the disease, earlier treatment regimens, the immune status of the patient, and, of course, the HLA-haplotype of the patient. Furthermore, the vaccine according to the invention can contain individualised components, according to personal needs of the particular patient. Examples are different amounts of peptides according to the expression of the related TAAs in said particular patient, unwanted side-effects due to personal allergies or other treatments, and adjustments for secondary treatments following a first round or scheme of treatment.

A still further aspect of the present invention relates to the use of a peptide according to the invention, or of a polynucleotide or expression vector encoding such a peptide, in the manufacture of a medicament for killing target cells in a patient which target cells aberrantly express a polypeptide comprising an amino acid sequence of the invention. Preferred is the use as a pharmaceutical composition that is an anti-cancer vaccine.

A still further aspect of the present invention provides the use of a peptide according to the invention, or of a polynucleotide or expression vector encoding such a peptide, for the manufacture of a medicament for inducing an immune response, in particular a cellular immune response, more particularly a T-cell mediated immune response against cells of solid tumours which cells express a human class I MHC molecule on their surface and present a polypeptide comprising an amino acid sequence of the invention.

It has been surprisingly found in the context of the present invention that tumour cells of solid tumours, in contrast to healthy cells of the same tissue, express human HLA class II molecule on their surface. This fact has been described only once in Brasanac et al (Brasanac D, Markovic-Lipkovski J, Hadzi-Djokic J, Muller GA, Muller CA. Immunohistochemical analysis of HLA class II antigens and tumor infiltrating mononuclear cells in renal cell carcinoma: correlation with clinical and histopathological data. Neoplasma. 1999;46(3):173-8.), where cryostat sections of 37 renal cell carcinomas (RCC) -- 25 clear cell type, 10 granular and 2 chromophobe -- were studied with indirect inununoperoxidase method applying monoclonal antibodies (MoAb) to HLA-DR, -DP and -DQ antigens for analysis of HLA class II antigens, and anti-CD 14, -CD3, -CD4 and -CD8 MoAb for tumour infiltrating mononuclear cells (TIM). Number of positive cells was estimated semiquantitatively and results of immunohistochemical investigation were correlated with clinical (patient age and sex, tumour size and TNM stage) and histopathological (cytology, histology, grade) characteristics of RCC. All RCC expressed HLA-DR, 92% -DQ and 73% -DP antigens with level of expression in hierarchy- DR>-DQ>-DP, but no statistically important correlation could be established with any of the histopathological or clinical parameters analyzed. Monocytes were more abundant than T lymphocytes and CD4+ than CD8+ T-cells, whereas tumours with T lymphocyte predominance and approximately equal number of CD4+ and CD8+ T-cells had greatest average diameter. Inadequate activation of T lymphocytes by tumour cells (despite capability of antigen presentation) could be the reason for association of parameters which indicates more aggressive tumour behaviour with aberrant HLA class II antigen expression on RCC.

A still further aspect of the present invention provides the use of a peptide according to the invention, or of a polynucleotide or expression vector encoding such a peptide, in the manufacture of a medicament for killing target cells in a patient whose target cells express a polypeptide comprising an amino acid sequence as given in any of SEQ ID No. 2 .

A further aspect of the invention thus provides methods for producing activated T lymphocytes in vivo or in vitro, whereby a first method comprises contacting in vitro T-cells with antigen-loaded human class I MHC molecules expressed on the surface of a suitable antigen-presenting cell for a period of time sufficient to activate, in an antigen specific manner, said T-cell wherein the antigen is a peptide according to the invention. A second method, which is more preferred, is described by Walter et al. (Walter S, Herrgen L, Schoor O, Jung G, Wernet D, Buhring HJ, Rammensee HG, Stevanovic S. Cutting edge: predetermined avidity of human CD8 T-cells expanded on calibrated MHC/anti-CD28-coated microspheres. J Immunol. 2003 Nov 15;171(10):4974-8).

The MHC class II molecules may be expressed on the surface of any suitable cell and it is preferred if the cell is one which does not naturally express MHC class II molecules (in which case the cell is transfected to express such a molecule) or, if it does, it is defective in the antigen-processing or antigen-presenting pathways. In this way, it is possible for the cell expressing the MHC class II molecule to be primed substantially completely with a chosen peptide antigen before activating the CTL.

The antigen-presenting cell (or stimulator cell) typically has an MHC class I or II molecule on its surface and preferably is substantially incapable of itself loading said MHC class I or II molecule with the selected antigen. As is described in more detail below, the MHC class I or II molecule may readily be loaded with the selected antigen in vitro.

Preferably the mammalian cell lacks or has a reduced level or has reduced function of the TAP peptide transporter. Suitable cells which lack the TAP peptide transporter include T2, RMA-S and Drosophila cells. TAP is the Transporter Associated with antigen Processing.

The human peptide loading deficient cell line T2 is available from the American Type Culture Collection, 12301 Parklawn Drive, Rockville, Maryland 20852, USA under Catalogue No CRL 1992; the Drosophila cell line Schneider line 2 is available from the ATCC under Catalogue No CRL 19863; the mouse RMA-S cell line is described in Karre and Ljunggren (1985) J. Exp. Med. 162,1745.

Conveniently said host cell before transfection expresses substantially no MHC class I molecules. It is also preferred if the stimulator cell expresses a molecule important for T-cell costimulation such as any of B7.1, B7.2, ICAM-1 and LFA 3.

In a further embodiment, combinations of HLA molecules may also be used.

The use of recombinant polyepitope vaccines for the delivery of multiple CD8-positive CTL epitopes is described in Thomson et al (1996) J. Immunol. 157, 822-826 and WO 96/03144, In relation to the present invention, it is desirable and advantageous to include in a single vaccine, a peptide (or a nucleic acid encoding a peptide) wherein the peptide includes, in any order, an amino acid sequence of the present invention and a CD4-positive T-cell-stimulating epitope (such as from MMP-7). Such a vaccine would be particularly useful for treating cancers.

A number of other methods may be used for generating CTL in vitro. For example, the methods described in Peoples et al (1995) Proc. Natl. Acad. Sci. USA 92,432-436 and Kawakami et al (1992) J. Immunol. 148, 638643 use autologous tumour-infiltrating lymphocytes in the generation of CTL. Plebanski et al (1995) Eur. J. Immunol. 25, 1783-1787 makes use of autologous peripheral blood lymphocytes (PLBs) in the preparation of CTL. Jochmus et al (1997) J. Gen. Virol. 78,1689-1695 describes the production of autologous CTL by employing pulsing dendritic cells with peptide or polypeptide, or via infection with recombinant virus. Hill et al (1995) J. Exp. Med. 181, 2221-2228 and Jerome et al (1993) J. Immunol. 151,1654-1662 make use of B cells in the production of autologous CTL. In addition, macrophages pulsed with peptide or polypeptide, or infected with recombinant virus, may be used in the preparation of autologous CTL.

Allogeneic cells may also be used in the preparation of CTL and this method is described in detail in WO 97/26328. For example, in addition to Drosophila cells and T2 cells, other cells may be used to present antigens such as CHO cells, baculovirus-infected insects cells, bacteria, yeast, vaccinia-infected target cells. In addition plant viruses may be used (see, for example, Porta et al (1994) Virology 202, 449-955 which describes the development of cowpea mosaic virus as a high-yielding system for the presentation of foreign peptides.

Preferably, in the method according to the present invention, the antigen-presenting cell comprises an expression vector as above.

The activated T-cells which are directed against the peptides of the invention are useful in therapy. Thus, a further aspect of the invention provides activated T-cells obtainable by the foregoing methods of the invention.

A still further aspect of the invention provides activated T-cells which selectively recognise a cell which aberrantly expresses a polypeptide comprising an amino acid sequence of the invention. Preferably, the T-cells recognises the said cell by interacting with the HLA/peptide-complex (for example, binding). The T-cells are useful in a method of killing target cells in a patient which target cells aberrantly express a polypeptide comprising an amino acid sequence of the invention wherein the patient is administered an effective number of the activated T-cells. The T-cells which are administered to the patient may be derived from the patient and activated as described above (i.e. they are autologous T-cells). Alternatively, the T-cells are not from the patient but are from another individual. Of course, it is preferred if the individual is a healthy individual. By "healthy individual" the inventors mean that the individual is generally in good health, preferably has a competent immune system and, more preferably, is not suffering from any disease which can be readily tested for, and detected.

The activated T-cells express a T-cell receptor (TCR) which is involved in recognising cells which express the aberrant polypeptide. It is useful if the cDNA encoding the TCR is cloned from the activated T-cells and transferred into a further T-cells for expression.

In vivo, the target cells for the CD4-positive T-cells according to one embodiment of the present invention can be cells of the tumour (which sometimes express MHC class II) and/or stromal cells surrounding the tumour (tumour cells) (which sometimes also express MHC class II).

The TCRs of T-cell clones of the invention specific for the peptides of the invention are cloned. The TCR usage in the T-cells clones is determined using (i) TCR variable region-specific monoclonal antibodies and (ii) RT PCR with primers specific for Va and Vp gene families. A cDNA library is prepared from poly-A mRNA extracted from the T-cells clones. Primers specific for the C-terminal portion of the TCR a and P chains and for the N-terminal portion of the identified Va and P segments are used. The complete cDNA for the TCR a and P chain is amplified with a high fidelity DNA polymerase and the amplified products cloned into a suitable cloning vector. The cloned a and P chain genes may be assembled into a single chain TCR by the method as described by Chung et al (1994) Proc. Natl. Acad. Sci. USA 91, 12654-12658. In this single chain construct the VaJ segment is followed by the V DJ segment, followed by the Cp segment followed by the transmembrane and cytoplasmic segment of the CD3 chain. This single chain TCR is then inserted into a retroviral expression vector (a panel of vectors may be used based on their ability to infect mature human CD8-positive T lymphocytes and to mediate gene expression: the retroviral vector system Kat is one preferred possibility (see Finer et al (1994) Blood 83, 43). High titre amphotrophic retrovirus are used to infect purified CD8-positive or CD4-positive T lymphocytes isolated from the peripheral blood of tumour patients (following a protocol published by Roberts et al (1994) Blood 84, 2878-2889,). Anti-CD3 antibodies are used to trigger proliferation of purified CD8-positive T-cells, which facilitates retroviral integration and stable expression of single chain TCRs. The efficiency of retroviral transduction is determined by staining of infected CD8-positive T-cells with antibodies specific for the single chain TCR. In vitro analysis of transduced CD8-positive T-cells establishes that they display the same tumour-specific killing as seen with the allo-restricted T-cells clone from which the TCR chains were originally cloned. Populations of transduced CD8-positive T-cells with the expected specificity may be used for adoptive immunotherapy of the tumour patients. Patients may be treated with in between 10⁸ to 10¹¹ autologous, transduced T-cells. Analogously to CD8-positive, transduced CD4-positive T helper cells carrying related constructs can be generated.

Other suitable systems for introducing genes into T-cells are described in Moritz et al (1994) Proc. Natl. Acad. Sci. USA 91, 4318-4322, Eshhar et al (1993) Proc. Natl. Acad. Sci. USA 90, 720-724 and Hwu et al (1993) J. Exp. Med 178, 361-366 also describe the transfection of T-cells. Thus, the invention describes a TCR which recognises a cell which aberrantly expresses a polypeptide comprising an amino acid sequence of the invention, the TCR being obtainable from the activated T-cells.

As well as the TCR, functionally equivalent molecules to the TCR are described in the invention. These include any molecule which is functionally equivalent to a TCR which can perform the same function as a TCR. In particular, such molecules include genetically engineered three-domain single-chain TCRs as made by the method described by Chung et al (1994) Proc. Natl. Acad. Sci. USA 91, 12654-12658, and referred to above. The invention also describes a polynucleotide encoding the TCR or functionally equivalent molecule, and an expression vector encoding the TCR or functionally equivalent molecule thereof. Expression vectors which are suitable for expressing the TCR include those described above in respect of expression of the peptides of the invention.

It is, however, preferred that the expression vectors are ones that are able to express the TCR in a T-cells following transfection.

A further aspect of the invention describes a method of killing target cells in a patient which target cells aberrantly express a polypeptide comprising an amino acid sequence of the invention, the method comprising administering to the patient an effective amount of a peptide according to the invention, or an effective amount of a polynucleotide or an expression vector encoding a said peptide, or an effective number of T lymphocytes as defined above, wherein the amount of said peptide or amount of said polynucleotide or expression vector or T-cells is effective to provoke an anti-target cell immune response in said patient. The target cell is typically a tumour or cancer cell, in particular cells of solid tumors that express a human MHC class I molecule on their surface and present a polypeptide comprising an amino acid sequence as given above.

A still further aspect of the invention describes a method of killing target cells in a patient whose target cells aberrantly express a polypeptide comprising an amino acid sequence of the invention, the method comprising the steps of (1) obtaining T-cells from the patient; (2) introducing into said cells a polynucleotide encoding a TCR, or a functionally equivalent molecule, as defined above; and (3) introducing the cells produced in step (2) into the patient.

A still further aspect of the invention describes a method of killing target cells in a patient whose target cells aberrantly express a polypeptide comprising an amino acid sequence as defined above, the method comprising the steps of (1) obtaining antigen presenting cells, such as dendritic cells, from said patient; (2) contacting said antigen presenting cells with a peptide as defined in the first or second or third aspects of the invention, or with a polynucleotide encoding such a peptide, ex vivo; and (3) reintroducing the so treated antigen presenting cells into the patient.

Preferably, the antigen presenting cells are dendritic cells. Suitably, the dendritic cells are autologous dendritic cells which are pulsed with an antigenic peptide. The antigenic peptide may be any suitable antigenic peptide which gives rise to an appropriate T-cell response. T-cell therapy using autologous dendritic cells pulsed with peptides from a tumour associated antigen is disclosed in Murphy et al (1996) The Prostate 29, 371-380 and Tjua et al (1997) The Prostate 32, 272-278.

In a further embodiment the antigen presenting cells, such as dendritic cells, are contacted with a polynucleotide which encodes a peptide of the invention. The polynucleotide may be any suitable polynucleotide and it is preferred that it is capable of transducing the dendritic cell thus resulting in the presentation of a peptide and induction of immunity.

Conveniently, the polynucleotide may be comprised in a viral polynucleotide or virus. For example, adenovirus-transduced dendritic cells have been shown to induce antigen-specific antitumour immunity in relation to MHC1 (see Gong et al (1997) Gene Ther. 4,1023-1028). Similarly, adenovirus-based systems may be used (see, for example, Wan et al (1997) Hum. Gene Ther. 8, 1355-1363); retroviral systems may be used (Specht et al (1997) J. Exp. Med. 186, 1213-1221 and Szabolcs et al (1997) Blood particle-mediated transfer to dendritic cells may also be used (Tuting et al (1997) Eur. J. Immunol. 27, 2702-2707); and RNA may also be used (Ashley et al (1997) J. Exp. Med. 186, 1177 1182).

It will be appreciated that, with respect to the methods of killing target cells in a patient, it is particularly preferred that the target cells are cancer cells, more preferably renal or colon cancer cells.

In a preferred embodiment the HLA haplotype of the patient is determined prior to treatment. HLA haplotyping may be carried out using any suitable method; such methods are well known in the art.

The invention includes in particular the use of the peptides of the invention (or polynucleotides encoding them) for active in vivo vaccination; for manipulation of autologous dendritic cells in vitro followed by introduction of the so-manipulated dendritic cells in vivo to activate T-cell responses; to activate autologous T-cells in vitro followed by adoptive therapy (i.e. the so-manipulated T-cells are introduced into the patient); and to activate T-cells from healthy donors (MHC matched or mismatched) in vitro followed by adoptive therapy.

In a preferred embodiment, the vaccines of the present invention are administered to a host either alone or in combination with another cancer therapy to inhibit or suppress the formation of tumours.

The peptide vaccine may be administered without adjuvant. The peptide vaccine may also be administered with an adjuvant such as BCG or alum. Other suitable adjuvants are also described above..

The peptides according to the invention can also be used as diagnostic reagents. Using the peptides it can be analysed, whether in a T-cell-population T-cells are present that are specifically directed against a peptide or are induced by a therapy. Furthermore, the increase of precursor T-cells can be tested with those peptides that display reactivity against the defined peptide. Furthermore, the peptide can be used as marker in order to monitor the progression of the disease of a tumour that expresses said antigen of which the peptide is derived from.

In the attached Table 1 the peptides as identified are listed. In addition, in the Table the proteins are designated, from which the peptide is derived, and the respective position of the peptide in the respective protein. Furthermore the respective Acc-Numbers are given that relate to the Genbank of the "National Centre for Biotechnology Information" of the National Institute of Health (see http: www.ncbi.nlm.nih.gov).

In another preferred embodiment the peptides are used for staining of leukocytes, in particular of T-lymphocytes. This use is of particular advantage if it should be proven, whether in a CTL-population specific CTLs are present that are directed against a peptide. Furthermore the peptide can be used as marker for determining the progression of a therapy in an adenomateous or cancerous disease or disorder.

In another preferred embodiment the peptides are used for the production of an antibody. Polyclonal antibodies can be obtained in a standard fashion by Immunisation of Animals via injection of the peptide and subsequent purification of the immune globulin. Monoclonal antibodies can be produced according to standard protocols such as described, for example, in Methods Enzymol. (1986), 121, Hybridoma technology and monoclonal antibodies.

The identification of helper T-cell epitopes of TAA remains an important task in anti-tumour immunotherapy. Until now, different strategies for the identification of class I or II peptides from TAA have been carried out, ranging from the incubation of APCs with the antigen of interest in order to be taken up and processed (Chaux, P., V. Vantomme, V. Stroobant, K. Thielemans, J. Corthals, R. Luiten, A.M. Eggermont, T. Boon, and B.P. van der Bruggen. 1999. Identification of MAGE-3 epitopes presented by HLA-DR molecules to CD4(+) T lymphocytes. J. Exp. Med. 189:767-778), to various transfection strategies with fusion proteins (Dengjel, J., P.Decker, O. Schoor, F. Altenberend, T. Weinschenk, H.G. Rammensee, and S. Stevanovic. 2004. Identification of a naturally processed cyclin D1 T-helper epitope by a novel combination of HLA class II targeting and differential mass spectrometry. Eur. J. Immunol. 34:3644-3651). All these methods are very time-consuming and it often remains unclear, if the identified HLA ligands are actually presented *in vivo* by human tissue.

The inventors identified a ligand accounting for one core sequence from MMP7. The inventors found this protein to be over-expressed in renal cell carcinomas, in addition, it has been described as tumour-associated (Miyamoto, S., K. Yano, S. Sugimoto, G. Ishii, T. Hasebe, Y. Endoh, K. Kodama, M. Goya, T. Chiba, and A. Ochiai. 2004. Matrix metalloproteinase-7 facilitates insulin-like growth factor bioavailability through its proteinase activity on insulin-like growth factor binding protein 3. Cancer Res. 64:665-671; Sumi, T., T. Nakatani, H. Yoshida, Y. Hyun, T. Yasui, Y. Matsumoto, E. Nakagawa, K. Sugimura, H. Kawashima, and O. Ishiko. 2003. Expression of matrix metalloproteinases 7 and 2 in human renal cell carcinoma. Oncol. Rep. 10:567-570). The peptide bound promiscuously to HLA class II molecules and was able to activate CD4-positive T-cells from different healthy donors. Thus, the inventors' approach will be helpful in the identification of new class II peptide candidates from TAA for use in clinical vaccination protocols.

The invention will now be described in more detail by reference to the following Figures, the Sequence listing, and the Examples. The following examples are provided for illustrative purposes only and are not intended to limit the invention.

SEQ ID No 1 to SEQ ID No 2 show peptide sequences of T-cell epitope containing peptides that are presented by MHC class I or II.

SEQ ID No 3 to SEQ ID No 11 show peptide sequences of peptides that are used in the vaccine of the present invention, which is subsequently referred to as "IMA".

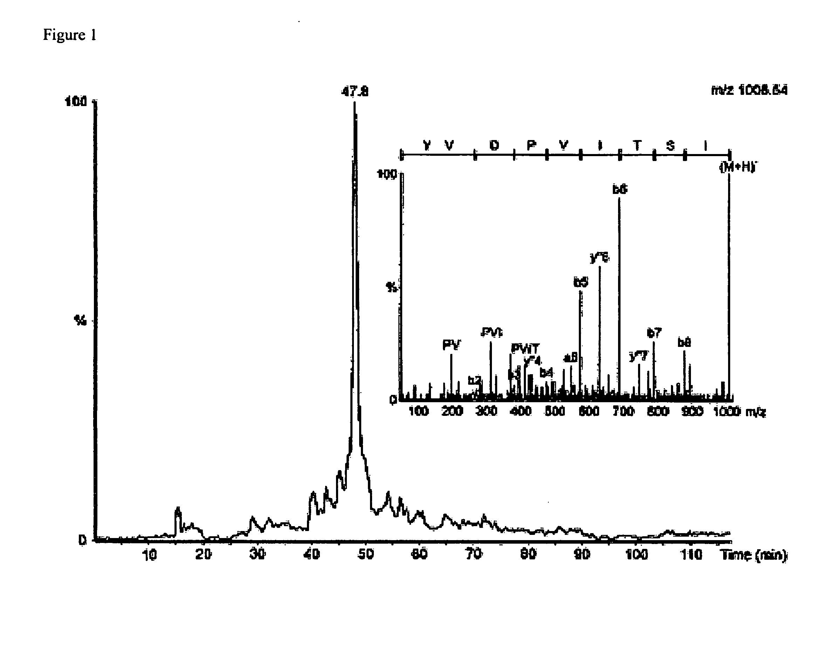

Figure 1 shows the presentation of the c-Met protooncogene derived peptide IMA-MET-001 on primary tumour sample RCC013. Nanocapillary high-performance liquid chromatography ESI MS was done on peptides eluted from RCC013. The mass chromatogram for 1006.54 ±0.5 Da shows a peak at retention time 47.8 min. Collisionally induced decay mass spectrum from m/z 1006.54, recorded in a second LC-MS run at the given retention time and shown in the inset, confirmed the presence of IMA-MET-001 (Weinschenk 2002).

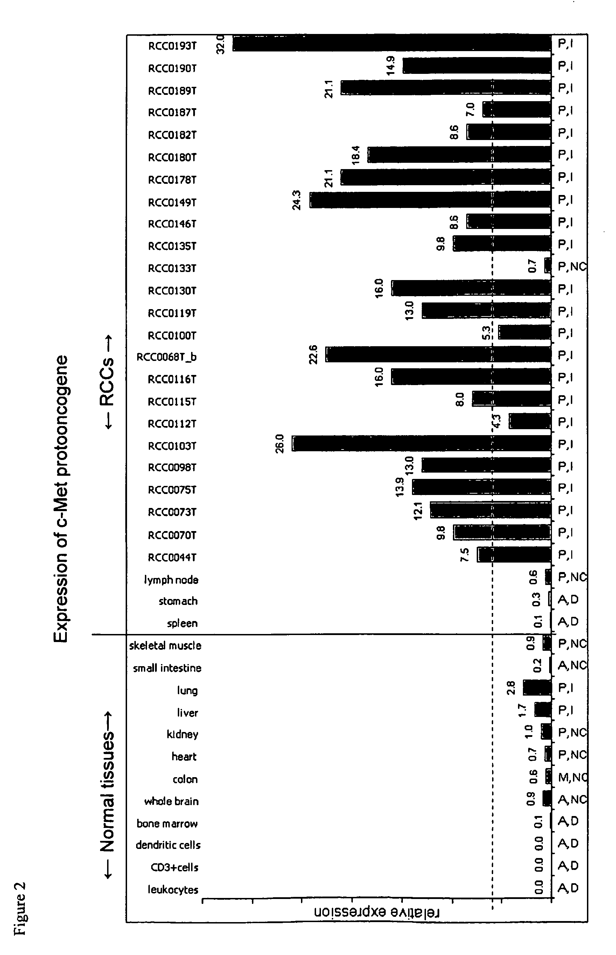

Figure 2 shows the tissue expression of c-Met protooncogene (MET). Expression was analyzed by oligonucleotide microarrays. Copy numbers are relative to kidney, which is set at 1.0. "P" means that the gene is present, "A" absent and "M" marginal according to the statistical absolute call algorithms. "I" means that expression of the gene is significantly increased relative to kidney, "D" stands for decreased expression, and "NC" means that there is no change in expression. The expression value relative to kidney is calculated from the signal log ratio and displayed on top of the bars. The dashed horizontal line shows the highest expression in normal tissues (in this case lung).

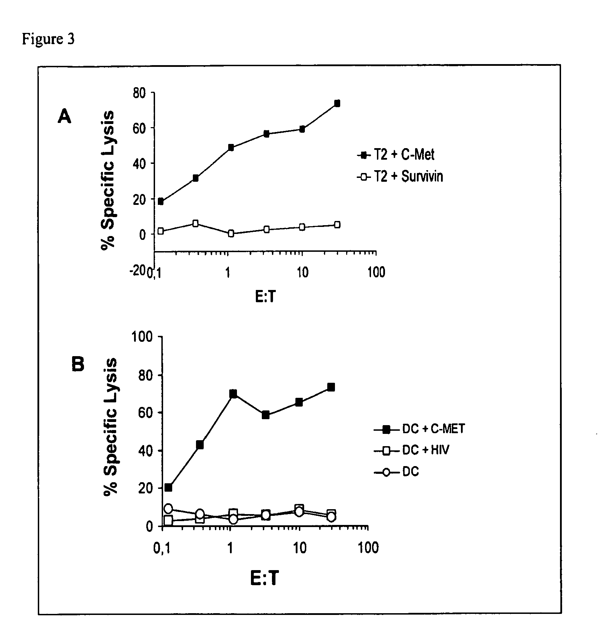

Figure 3 shows the killing of peptide-loaded target cells by CTLs primed with IMA-MET-001

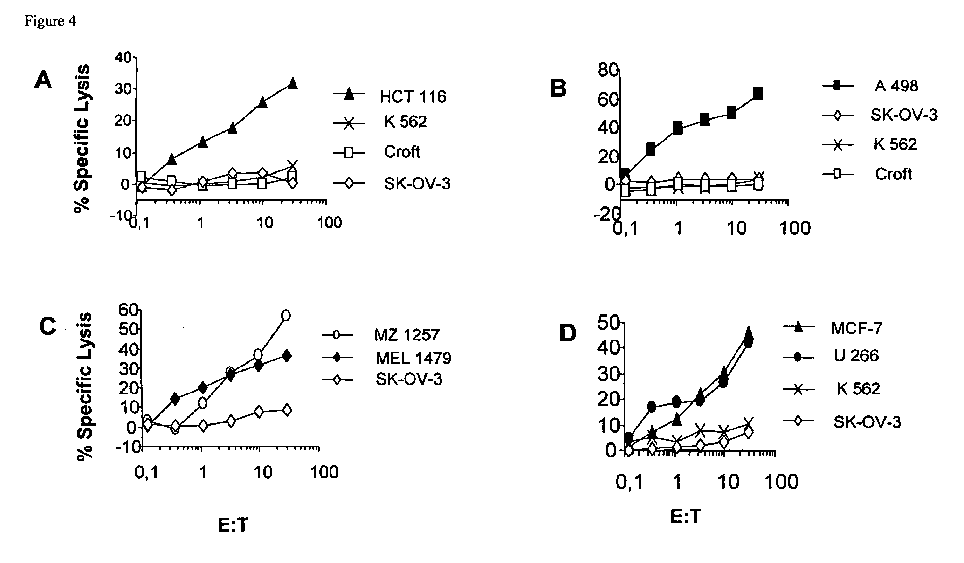

Figure 4 shows the killing of malignant cells by CTL primed with IMA-MET-001.

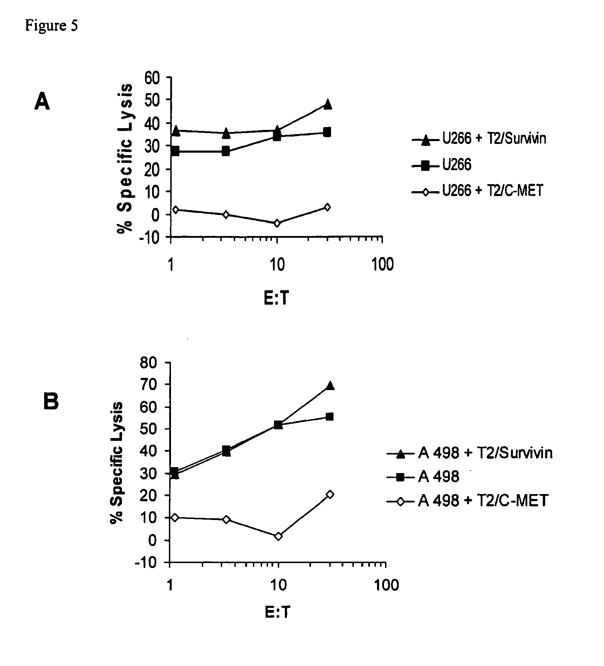

Figure 5 shows the cold target inhibition assay.

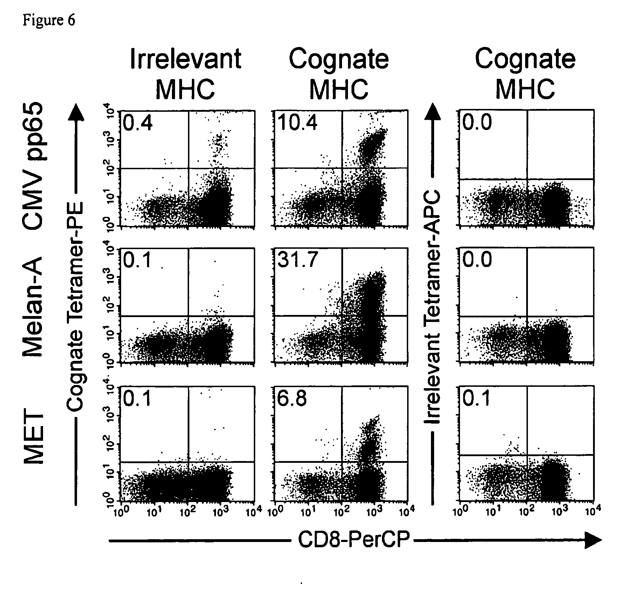

Figure 6 shows the tetrameric analysis of microsphere driven expansions.

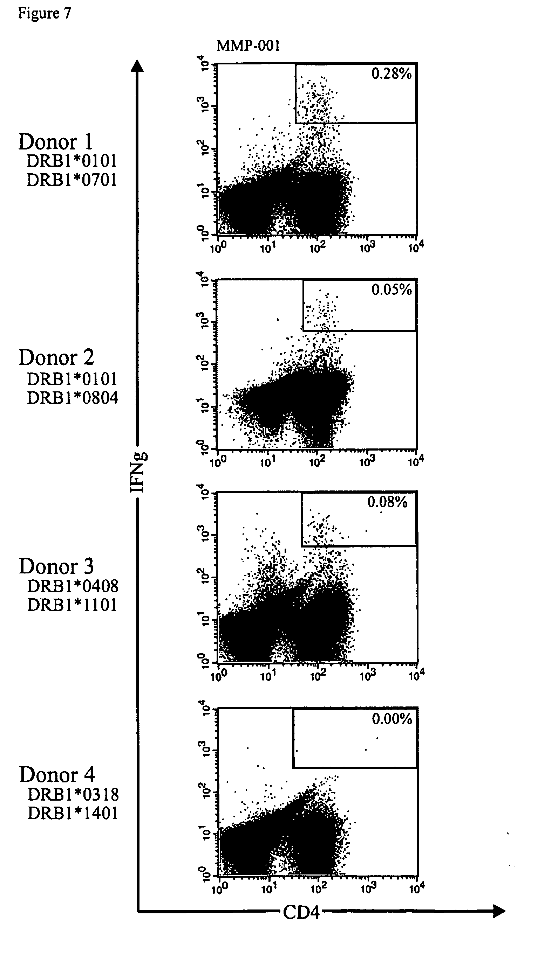

Figure 7 shows the in vitro immunogenicity of IMA-MMP-001 - Representative intracellular IFN gamma versus CD4 stainings of four healthy donors. Donor 1, 2 and 3 showed CD4-positive T-cells reactive against IMA-MMP-001 after the third and the fourth stimulation. Donor 4 was always negative.

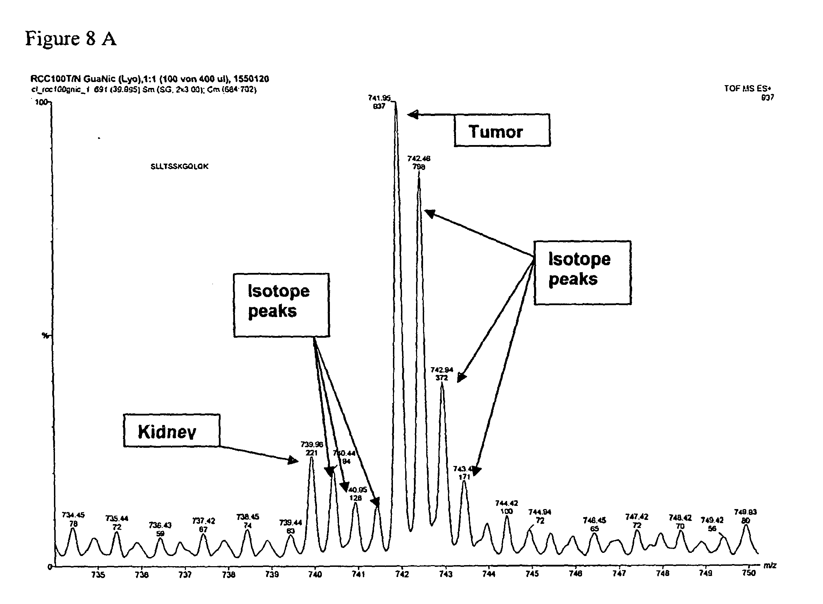

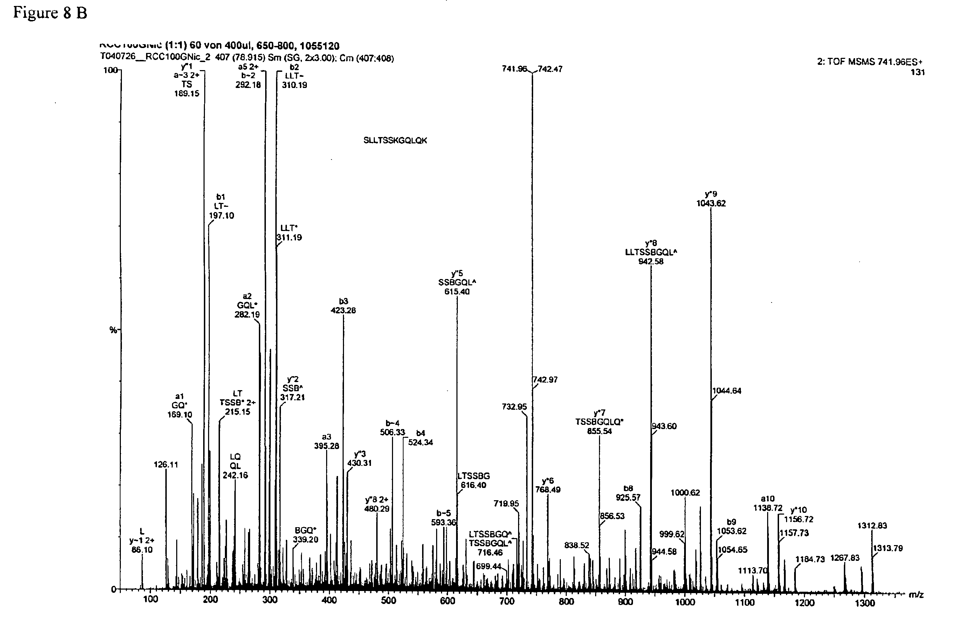

Figure 8 shows differential peptide presentation on tumour and healthy tissue - (A) Mass spectrum of two peptide species m/z 739.96 and 741.95 derived from normal kidney and renal cell carcinoma tissue of patient RCC100, respectively. The mass spectrum demonstrates an about 4-fold overpresentation of the Adipophilin peptide on the renal cell carcinoma tissue compared to the corresponding autologous normal tissue (B) The collisionally induced decay mass spectrometry analysis of m/z 741.95 (tumour) revealed the peptide sequence IMA-ADF-003, a peptide sequence derived from Adipophilin.

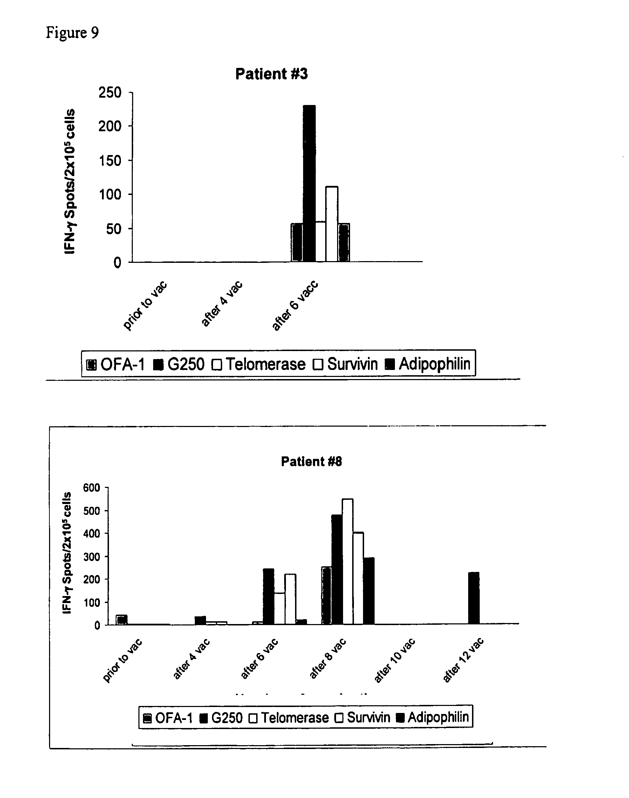

Figure 9 shows in vivo immunity against IMA-ADF-001 - T-cell immunity in 2 RCC patients against several non-vaccinated peptides in patients vaccinated with autologous dendritic cells pulsed with two TUMAPs derived from MUC. T-cells specific for IMA-ADF-001 ("Adipophilin") were not present prior to vaccination and were detected in patient #3 (upper panel) after 6 vaccinations and in patient #8 (lower panel) after 8 vaccinations.

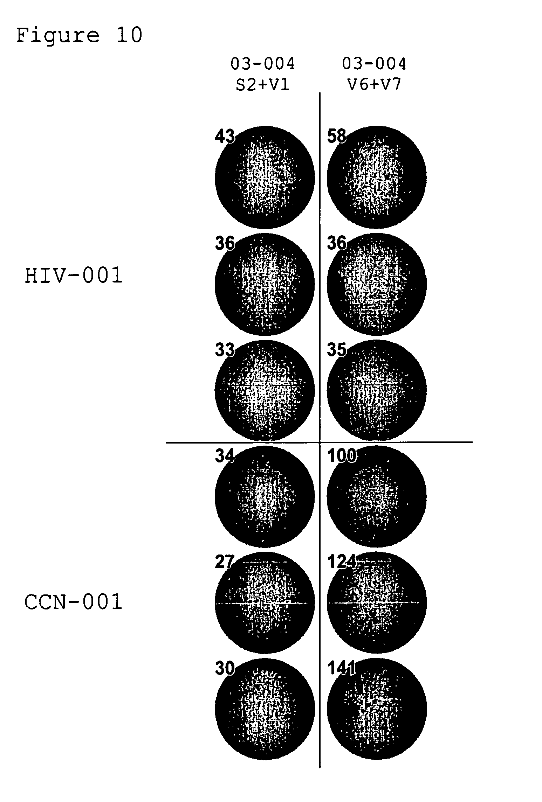

Figure 10 shows representative examples of an IMA induced T-cell response identified by the amplified ELISPOT assay for the same patient and antigen. Upper and lower column represent negative control antigen HIV-001 and single TUMAP IMA-CCN-001 used for readout, respectively. The left column shows ELISPOTs of pooled samples taken before vaccination on screening day 2 (S2) and immediately prior to the first injection (V1). The right column shows ELISPOTs of pooled samples taken during the vaccination protocol on day 22 (V6) and day 36 (V7). The number of positive cells is given for each experiment.

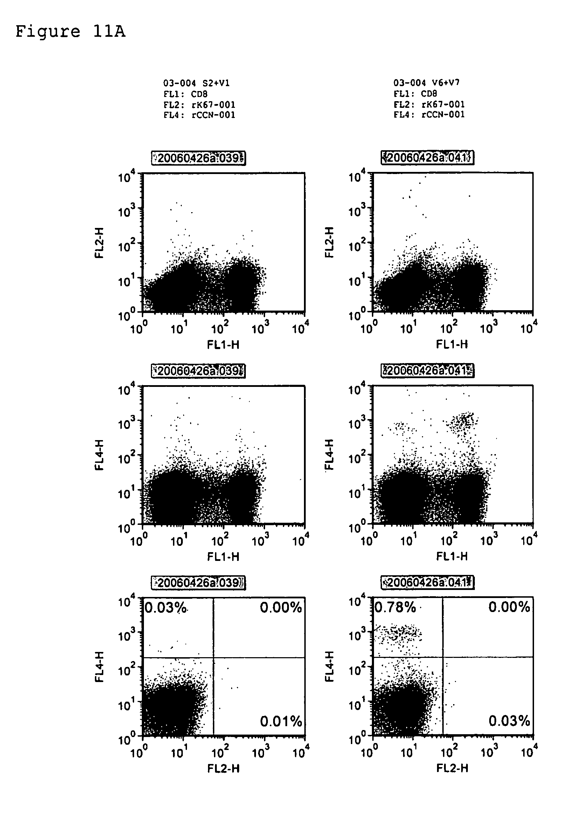

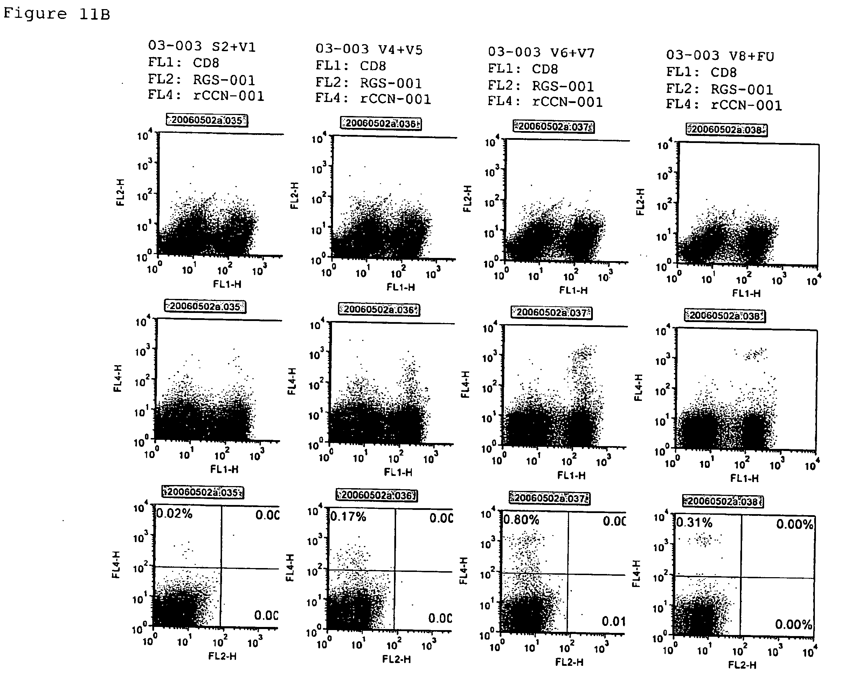

Figure 11: Representative examples of IMA induced T-cell responses identified by the amplified Tetramer staining assay. Upper and middle panels represent two-dimensional dot plots gated on CD3+ lymphocytes, lower panels are gated on CD3+ CD8+ lymphocytes. Patients, timepoints and stainings as indicated for each column. S1+V1: samples taken prior to vaccination; V4+V5: samples taken at day 8 and day 15; V6+V7: samples taken on day 22 and day 36; V8+FU: samples taken on day 64 (last vaccination) and after 85 to 92 days (end of study)
   A: Data for patient 03-004 confirming the immunological response to IMA-CCN-001 shown in Figure 10. A cell population positive for CD3+ and IMA-CCN-001 tetramer can be identified after the forth and fifth injection of IMA (V6+V7; middle panel) accounting for 0.78% of the lymphocytes (V6+V7;lower panel). No positive population was found for the K67-001 tetramer (upper panel).
   B: Data for patient 03-003 exhibiting no immunological response against IMA-RGS-001 peptide (upper panel) but developing IMA-CCN-001 tetramer positive response during the time course of the vaccination protocol (middle panel, column 3 and 4) accounting for up to 0,8 % of the lymphocytes (lower panel; column 3).

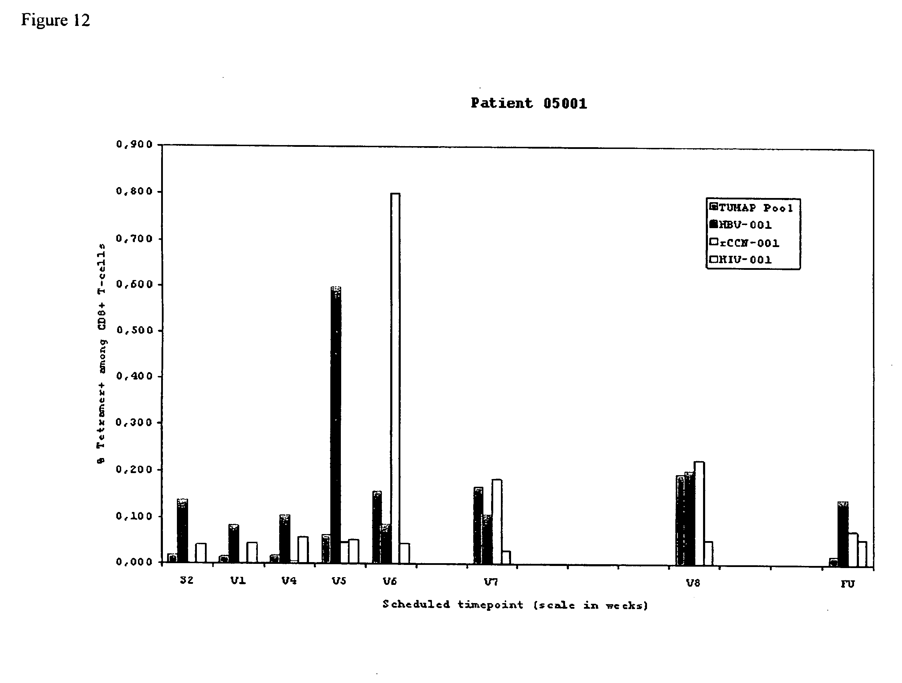

Figure 12: Observed T-cell magnitude kinetics in single time point amplified tetramer assays. Results are shown for single time point readouts for patient 05-001 for whom the vaccine-induced T-cell response had been detected by the routine amplified tetramer assay with pooled samples. Results are given for all tumour associated antigens present in IMA (TUMAP pool) and in particular for the IMA-CCN-001 peptide. Additionally, the HIV-001 and IMA-HBV-001 controls within the same single time point assay are shown.

### Examples

### Glossary

| Term or Abbreviation | Description |
|---|---|
| AE | Adverse Event |
| AJCC | American Joint Committee on Cancer |
| BfArM | Bundesinstitut für Arzneimittel und Medizinprodukte |
| CTL | Cytotoxic T-cells |
| DC | Dendritic Cells |
| GM-CSF | rhuGM-CSF (recombinant human Granulocyte-Macrophage |
| rhuGM-CSF | Colony-Stimulating Factor) |
| HBV | Hepatitis B Virus |
| HLA | Human Lymphocyte Antigen |
| IARC | International Agency for Research on Cancer |
| IMP | Investigational Medicinal Product |
| INF | Interferon |
| MAA | Marketing Authorization Application |
| MHC | Major Histocompatibility Complex |
| RCC | Renal Cell Carcinoma |
| SAE | Serious Adverse Event |
| SmPC | Summary of Product Characteristics |
| TUMAP | Tumour-Associated Peptide |

### I. Characterization of peptides

### Data regarding expression of gene products from which IMA peptides are derived

Peptides that were identified from primary RCC tissue were selected for inclusion into the vaccine IMA (see below) according to an internal ranking system mainly based on gene expression analysis, literature, and database search for known properties of an antigen from which a derived peptide has been identified. All naturally presented peptides are highly over-expressed in renal cell carcinoma tissue compared to normal kidney tissue as well as a range of other vital organs and tissues. Such a selection is necessary (1) to select for peptides that are able to induce T-cells with high specificity for recognition of the tumour but not other tissue to minimize the chance of autoimmunity induced by the vaccination of IMA and (2) to ensure that the majority of tumours in a patient population is recognized by the induced T-cell.

The average prevalence of the antigens from which the derived peptides are contained in IMA is 68% (over-expression in RCC vs. vital organs and tissues in n=24 RCC samples) ranging from 54% to 96% for the single antigens. This is significantly higher than in standard tumour antigens such as Her-2/neu (prevalence: 25-30%).

Global gene expression profiling was performed using a commercially available high-density microarray system (Affymetrix). RNA was isolated from the tissues, processed and hybridized to high density oligonucleotide microarrays. After staining and washing, the arrays were scanned and the fluorescence intensity of each spot on the array represented sort of expression level of the gene matching the DNA sequence of the oligonucleotide. Several oligonucleotides on the arrays cover the sequence of each gene. After statistical software analysis, pair wise relative expression values between two samples can be obtained for each gene. Normalization of all data from different samples using one constant sample as baseline allows relative quantification of expression levels between all samples.

RNA sources - Total RNA from human tissues were obtained commercially (Ambion, Huntingdon, UK; Clontech, Heidelberg, Germany; Stratagene, Amsterdam, The Netherlands, BioChain, Heidelberg, Germany). Total RNA from several individuals was mixed in a way that RNA from each individual was equally weighted. Quality and quantity was confirmed on the Agilent 2100 Bioanalyzer (Agilent, Waldbronn, Germany) using the RNA 6000 Nano LabChip Kit (Agilent).

High-Density Oligonucleotide Microarray Analysis - Double-stranded DNA was synthesized from 5-8 µg of total RNA using SuperScript RTII (Life Technologies, Inc., Karlsruhe, Germany) and the primer (Eurogentec, Seraing, Belgium) as given by the Affymetrix manual. In vitro transcription using the BioArray™ High Yield™ RNA Transcript Labelling Kit (ENZO Diagnostics, Inc., Farmingdale, NY), fragmentation, hybridization on Affymetrix U133A or U133 Plus 2.0 GeneChips (Affymetrix, Santa Clara, CA), and staining with streptavidin-phycoerythrin and biotinylated anti-streptavidin antibody (Molecular Probes, Leiden, The Netherlands) followed the manufacturer's protocols (Affymetrix). The Affymetrix GeneArray Scanner was used and data were analyzed with the Microarray Analysis Suite 5.0 software or the GeneChip® Operating Software (GCOS). For normalization, 100 housekeeping genes provided by Affymetrix were used. Pairwise comparisons were calculated using the expression values in kidney as baseline. Accordingly, all expression values calculated from signal log ratios are relative to kidney, which was set at 1. Significance of differential expression was judged by the "change" values given by the statistical algorithms implemented in the software. For absolute detection of expression, data were analyzed again using the statistical algorithms. Presence or absence of gene expression was determined by the absolute call algorithms.

An exemplary tissue expression panel for gene expression of c-Met protooncogene (MET) is shown in Figure 2. MET was over-expressed in 96% of renal cell carcinomas analyzed (n=24, right hand side), but not or to a much lower extend in several selected vital healthy tissues and organs as well immunologically important tissues and cells (left hand side in Figure 2):

Table 1 summarizes the peptides contained in the vaccine of the invention IMA, comprising also the peptides according to the invention.

**Table 1: Peptides contained in the vaccine of the invention**

| **Internal Sequence ID** | **Antigen** | **Sequence** | **SEQ ID No.** |
|---|---|---|---|
| IMA-MMP-001 | Matrix metalloproteinase 7 | SQDDIKGIQKLYGKRS | 1 |
| IMA-ADF-002 | Adipophilin | VMAGDIYSV | 2 |
| IMA-ADF-001 | Adipophilin | SVASTITGV | 3 |
| IMA-APO-001 | Apolipoprotein L1 | ALADGVQKV | 4 |
| IMA-CCN-001 | Cyclin D1 | LLGATCMFV | 5 |
| IMA-GUC-001 | GUCY1A3 | SVFAGVVGV | 6 |
| IMA-K67-001 | KIAA0367 | ALFDGDPHL | 7 |
| IMA-MET-001 | c-met proto-oncogene | YVDPVITSI | 8 |
| IMA-MUC- 001 | MUC1 | STAPPVHNV | 9 |
| IMA-RGS-001 | RGS-5 | LAALPHSCL | 10 |
| IMA-HBV-001 | HBV | FLPSDFFPSV | 11 |

Table 2 summarizes the expression results for all antigens coding for peptides contained in the vaccine of the invention IMA, as well as for the peptides according to the invention.

**Table 2: Frequencies of over-expression of antigens in RCC (n=24)**

| **Internal Sequence ID** | **Antigen** | **Significant over-expression RCC versus kidney¹** | **Over-expression RCCs versus all normal Tissues²** |
|---|---|---|---|
| IMA-ADF-001 & 002 | Adipophilin | 83% | 75% |
| IMA-APO-001 | Apolipoprotein L1 | 67% | 58% |
| IMA-CCN-001 | Cyclin D1 | 58% | 63% |
| IMA-GUC-001 | GUCY1A3 | 88% | 71% |
| IMA-K67-001 | KIAA0367 | 54% | 54%³ |
| IMA-MET-001 | c-met proto-oncogene | 96% | 96% |
| IMA-MUC-001 | MUC1 | No over-expression on mRNA-level | No over-expression on mRNA-level |
| IMA-RGS-001 | RGS-5 | 96% | 58% |
| IMA-MMP-001 | Matrix metalloproteinase 7 | 58% | 67% |

| | | | |
|---|---|---|---|
| 1 According to the "change" values given by the statistical algorithms implemented in the software (number of "I"s) 2 Number of RCCs with higher expression as compared to the normal tissue with the highest expression amongst all normal tissues 3 Brain is immuno-privileged and was not considered for this reason | | | |

The minimum over-expression in RCC versus all normal tissues is 54%, the maximum is 96%. This is significantly higher than in standard tumour antigens such as Her-2/neu (prevalence: 25-30%).

An exception is MUC where no over-expression can be detected for the MUC mRNA. However, following published reports have to taken into consideration:
1. Aberrant deglycosylation in malignancies is common and unmasks epitopes in tumour cells which might not be presented on normal cells. It is highly likely that such a mechanism also occurs in RCC. This would explain the specific killing of tumor cell lines expressing MUC (Brossart 1999). Please also see chapter 4.1.5 on the properties of MUC.
2. IMA-MUC-001 has been administered in conjunction with autologous dendritic cells in an investigator-initiated trial at the University of Tübingen. In this trial presented recently at the ASCO 2003 (Mueller 2003) and follow-up data at the ASCO 2005 (Wierecky 2005) Meeting no autoimmune effects were reported.
3. Other reports from clinical studies demonstrate that cytotoxic T-cells specific for IMA-MUC-001 occur naturally (without immunization) in breast carcinoma (Rentzsch 2003) and colorectal carcinoma patients (Dittmann 2004). In these patients no autoimmune effects were reported. This emphasizes the natural role of IMA-MUC-001-specific T-cells.

Based on this supportive data the administration of IMA-MUC-001 can be considered as safe although no over-expression can be detected for the MUC antigen on mRNA level alone.

### Promiscuous binding of IMA-MMP-001 to several HLA-DR alleles

IMA-MMP-001 is a peptide binding to HLA-DR, a HLA class II molecule. Class II TUMAPs activate T helper cells which play a crucial role in assisting the function of cytotoxic T-cells activated by class II TUMAPs. Promiscuous binding of a HLA-DR peptide is important to ensure that the majority (>50%) of the HLA-A*02-positive patients treated with IMA are also able to elicit a T-cell response to IMA-MMP-001. *In silico* analysis of the binding of IMA-MMP-001 indicates that IMA-MMP-001 binds promiscuously to several HLA-DR alleles (DRB1*0101, *0301, *0401, *1101 and *1501) covering a total of at least 69.6% of the HLA-A2 positive Caucasian population. Promiscuous binding of IMA-MMP-001 is confirmed experimentally by in vitro immunogenicity data.

### Principle of test

Using the SYFPEITHI algorithm developed at the University of Tübingen (Rammensee 1997; Rammensee 1999), binding of IMA-MMP-001 to several common HLA-DR alleles (see table below) was ranked. The algorithm has been successfully used to identify class I and class II epitopes from a wide range of antigens, e.g. from the human TAA TRP2 (class I) (Sun 2000) and SSX2 (class II) (Neumann 2004). The analyzed HLA-DR alleles cover at least 69.6% of the HLA-A2 positive Caucasian population (Mori 1997). The threshold for binding was defined at a score of 18 based on the analysis of binding scores of known published promiscuous HLA-DR ligands. Promiscuous binding is defined as binding of a HLA-DR peptide to several HLA-DR alleles expressed in at least 50% of the Caucasian population.

The loci of HLA-A and HLA-DR are in linkage disequilibrium yielding combinations of HLA-A2 and specific HLA-DRs that are favoured in comparison to others (Table 3).

**Table 3: Haplotype frequencies of North American Caucasians - Shown are the serological haplotypes. N.a. stands for not assigned (Mori 1997).**

| | | |
|---|---|---|
| **Haplotype** | | **Frequency** |
| **HLA-A** | **HLA-DR** | **[%]** |
| 2 | 1 | 8.8 |
| 2 | 2 | 14.9 |
| 2 | 3 | 6.1 |
| 2 | 4 | 21.3 |
| 2 | 5 | 1.2 |
| 2 | 6 | 15.2 |
| 2 | 7 | 13.0 |
| 2 | 8 | 4.2 |
| 2 | 9 | 1.2 |
| 2 | 10 | 1.4 |
| 2 | 11 | 8.7 |
| 2 | 12 | 2.6 |
| 2 | n.a. | 1.4 |

Ligands of certain MHC molecules carry chemical related amino acids in certain positions of their primary sequence which permits the definition of a peptide motif for every MHC allele (Falk 1991). SYFPEITHI uses motif matrices deduced from refined motifs exclusively based on natural ligand analysis by Edman degradation and tandem mass spectrometry (Schirle 2001). These matrices allow the exact prediction of peptides from a given protein sequence presented on MHC class I or class II molecules (Rotzschke 1991).

**Table 4: Binding scores of IMA-MMP-001 to common HLA-DR alleles**

| **Antigen** | **DRB1* allele** | | | | | |
|---|---|---|---|---|---|---|
| | **0101** | **0301** | **0401** | **0701** | **1101** | **1501** |
| | (8.8%) | (6.1%) | (21.3%) | (13.0 %) | (8.7%) | (n.a.%) |
| **IMA**-**MMP-001** | 35 | 18 | 20 | 14 | 26 | 20 |

Shown are the IMA-MMP-001 SYFPEITHI binding scores for the most common HLA-DRB1 alleles in the Caucasian population. The frequencies of the corresponding serological haplotypes of HLA-A2 positive Caucasians are given in brackets. The peptide was considered as binding to a HLA molecule when the score was equal or higher than 18.

Based on the prediction by the SYFPEITHI algorithm IMA-MMP-001 is likely to bind to several HLA-DR alleles (DRB1*0101, *0301, *0401, *1101 and *1501) covering at least 69.6 % of the HLA-A2 positive Caucasian population. As no frequency data of HLA-DR15 is available this allele was omitted in the calculation. Thus, it is very likely that the coverage of the population is even higher than 69.9%. Experimental confirmation for promiscuous binding of IMA-MMP-001 is obtained by in vitro immunogenicity data (see below).

### Comparison of expression of antigen and presentation of derived peptide on tumour and autologous normal tissue.

Overexpressed antigens are supposed to be overpresented on HLA molecules on the cell surface. Exemplary, the HLA-A*03 peptide derived from Adipophilin, an overexpressed antigen from which the HLA-A*02 peptides IMA-ADF-001 and IMA-ADF-002, both contained in IMA, are derived, was shown to be highly overpresented on renal cell carcinoma tissue compared to the autologous normal tissue from patient RCC100 employing the QUALITEA strategy. This demonstrates in this exemplary case that overexpression of an antigen (in this case Adipophilin) correlates with overpresentation of derived peptides of the same antigen.

The method is described in detail by (Lemmel 2004). QUALITEA represents a strategy for differential quantitation of HLA-eluted peptides from tumour and normal tissue. HLA ligands derived from the two different sources are N-terminally derivatised either by a ¹Hₓ- or ²Dₓ-reagent and combined. Following reduction of the peptide complexity by high performance liquid chromatography, peptides are quantitated by ESI-MS analysis according to their peak areas. A pair of derivatised peptides (¹Hₓ-derivatisation and ²Dₓ-derivatisation) is physicochemically identical and easily detectable because it essentially coelutes in chromatographic systems. Furthermore, there is a constant mass difference measured in the mass spectrometric scans. This difference depends on the number of stable isotopes in the derivative. Sequence identification of a ligand is revealed by ESI-MSMS analysis and computer-assisted database search of the spectrum recorded. Thus, this analysis provides information about qualitative and quantitative aspects of peptide presentation on tumour and normal tissue.

An example for differential HLA peptide presentation on tumour and normal tissue of an overexpressed antigen is shown in Figure 8. The peptide IMA-ADF-003 which was 4-fold overpresented on tumour tissue vs. healthy kidney tissue from patient RCC100 was identified by collisionally induced decay mass spectrometry analysis among many equally presented peptides. This peptide overpresented on tumour tissue was derived from Adipophilin. Gene expression analysis of the same patient RCC100 revealed also a 2.64 fold overexpression of Adipophilin in this tumour tissue compared to healthy kidney (data not shown). This data confirm in this particular case that overexpression in tumour tissue on gene level leads to peptide overpresentation on the tumour cell surface.

### In vivo immunogenicity against IMA-ADF-001

Autologous dendritic cells (DCs) generated from RCC patients were pulsed with two TUMAPs derived from MUC, among these IMA-MUC-001. IMA-ADF-001 was not vaccinated. Vaccinations were performed sc every two weeks four times and repeated monthly until tumour progression. After the fifth DC injection patients additionally received 3 injections/week of low dose IL-2 (1Mio IE/m2) sc. The activation of T-cell precursor was monitored using IFN-gamma ELISPOT. Besides induction of T-cells against to the two vaccinated peptides also T-cell activity against several other known TUMAPs, among them IMA-ADF-001 was tested.

The results were recently shown in a presentation by Dr. Peter Brossart (University of Tübingen) at the Annual Meeting of the American Society of Clinical Oncology (ASCO) 2005, the full presentation is published on the ASCO website. In two patients (pt #8 and pt #13) vaccinated with two MUC peptides pulsed on autologous DCs T-cell immunity to other peptides than the vaccinated ones were detected after vaccination (Figure 9). Because immunity was not present before vaccination it is highly likely that such T-cells were induced by epitope spreading. Epitope spreading may occur when tumour cells are disrupted (e.g. by necrosis, lysis by vaccine-induced T-cells etc.) and release antigens which are then taken up by antigen-presenting cells (APCs, e.g. DCs). These APCs may then process the antigen intracellulary and present a T-cell epitope (i.e. TUMAP) to prime T-cell responses. This data emphasizes the strong potential role of IMA-ADF-001 as a naturally occurring T-cell antigen.

### II. Production and use of the vaccine "IMA" according to the invention

IMA is a vaccine containing a set of tumour-associated peptides which are located and have been identified on primary renal cancer cells. This set includes HLA class I and class II peptides. The peptide set also contains one peptide from HBV core antigen used as a positive control peptide serving as immune marker to test the efficiency of the intradermal administration. Peptide vaccination in general needs to be adjuvanted, and such, GM-CSF will be exploited as adjuvant in this vaccination schedule (Human GM-CSF is commercially available as Sargramostim, Leukine®, Berlex).

8 of the 10 tumour-associated peptides contained in IMA were identified with the XPRESIDENT technology described below. For these peptides natural presentation in the context of HLA molecules expressed by the tumour is therefore demonstrated by direct evidence. The peptides IMA-MUC-001 and IMA-CCN-001 were identified using other technologies. For both latter peptides, natural presentation of these peptides by tumour cell lines is demonstrated based on indirect evidence in the in vitro immunogenicity assay (see below).

### Principle of test

HLA molecules from shock-frozen processed primary renal cell carcinoma tissue are purified and HLA-associated peptides are isolated. These peptides either are separated off-line by HPLC and fractions are analyzed or sequence analysis by mass spectrometry is done by online HPLC-MSMS experiments. The resulting sequences are verified by synthesis of the identified peptides and comparison of the fragment spectra of identified and synthesized peptides. As the identified peptides are directly derived from HLA molecules of the primary tumours these results present direct evidence on the natural processing and presentation of the identified peptides on primary renal cell carcinoma tissue.

### Method

The method is described in detail by (Weinschenk 2002). Briefly, shock-frozen patient samples obtained from the Department of Urology at the University of Tübingen (approved by local ethics committee) were lysed, HLA molecules were purified by affinity chromatography using the HLA class I-specific antibody W6/32 or the HLA-A*02-specific antibody BB7.2 or (in the case of IMA-MMP-001) the HLA-DR-specific antibody L243. HLA-associated peptides were eluted by acid treatment and isolated from the MHC alpha chain-protein by ultrafiltration. The isolated peptides either were separated off-line by reversed-phase high performance liquid chromatography and fractions were analyzed by nano-ESI MS on a hybrid quadrupole orthogonal acceleration time-of-flight tandem mass spectrometer (Q-TOF I or Q-TOF Ultima, Waters) or on-line LC-MSMS analysis was done using the same instruments. A blank run was always included to ensure that the system was free of peptide. Calibrations were done at least once per day and analyses on standard compounds were done in appropriate intervals to guarantee optimal performance of the systems. Interpretation of the fragment spectra was done manually. Verification of the analysis was obtained by database searches and solid-phase synthesis of the putative peptide sequence and comparison of the fragment spectra of identified and synthesized peptide. All peptides contained in IMA (data not shown) except IMA-MUC-001 and IMA-CCN-001 were identified in the identical fashion confirming the natural presentation of these peptides on primary renal cell carcinoma tissue.

### Ingredients of IMA

Peptides for this clinical development are synthesized by standard and well-established Fmoc-chemistry. Purification is done with preparative HPLC and ion exchange. Importantly, identity and purity of the peptides can be determined easily and with high accuracy using mass spectrometry and HPLC. The formulation of IMA consists of 11 individual drug substances which are described in further details below.

**Table 5: Antigens in IMA**

| | **Peptide ID** | **Type** | **Antigen** | **Common acronyms and synonyms** |
|---|---|---|---|---|
| 1 | IMA-ADF-001 | Class I TUMAP | Adipophilin | adipose differentiation-related protein, ADRP |
| 2 | IMA-ADF-002 | Class I TUMAP | Adipophilin | see above |
| 3 | IMA-APO-001 | Class I TUMAP | Apolipoprotein L1 | APOL1 |
| 4 | IMA-CCN-001 | Class I TUMAP | Cyclin D1 | CCND1, PRAD1, parathyroid adenomatosis 1, BCL-1 |
| 5 | IMA-GUC-001 | Class I TUMAP | GUCY1A3 | guanylate cyclase 1-soluble-alpha 3 |
| 6 | IMA-K67-001 | Class I TUMAP | KIAA0367 | -- |
| 7 | IMA-MET-001 | Class I TUMAP | c-met proto-oncogene | MET, HGF (hepatocyte growth factor) receptor, HGFR |
| 8 | IMA-MUC-001 | Class I TUMAP | MUC1 | mucin, CD227, episialin, epithelial membrane antigen |
| 9 | IMA-RGS-001 | Class I TUMAP | RGS-5 | regulator of G-protein signalling 5 |
| 10 | IMA-MMP-001 | Class II TUMAP | Matrix Metalloproteinase 7 | MMP7, matrilysine, uterine |
| 11 | IMA-HBV-001 | Viral control peptide | HBV core Antigen | HBc, HBcAg, cAg |

All peptides are synthesized by Fmoc solid phase chemistry and are purified by HPLC and ion exchange chromatography to a purity >95 %. The correct structure is determined by amino acid analysis and mass spectrometry.

**Table 6: Physico-chemical characteristics of peptides in IMA**

| | **Peptide ID** | **Peptide length (No. of amino acids)** | **Molecular mass (g/mol net)** | **Salt form** | **Physical Form** | **Solubility (Clear and colourless solution 1 mg/ml)** | **Hygroscopicity** |
|---|---|---|---|---|---|---|---|
| 1 | IMA-ADF-001 | 9 | 833.9 | Acetate salt | White to off-white powder | 10% acetic acid | Stored as freeze dried powder. Lyophilized peptides generally have hygroscopic properties |
| 2 | IMA-ADF-002 | 9 | 954.1 | | | 10% acetic acid | |
| 3 | IMA-APO-001 | 9 | 900.0 | | | water | |
| 4 | IMA-CCN-001 | 9 | 954.2 | | | 50% acetic acid | |
| 5 | IMA-GUC-001 | 9 | 834.0 | | | 90% acetic acid | |
| 6 | IMA-K67-001 | 9 | 984.1 | | | 20% acetic acid | |
| 7 | IMA-MET-001 | 9 | 1006.2 | | | 10% acetic acid | |
| 8 | IMA-MUC-001 | 9 | 921.0 | | | 10% acetic acid | |
| 9 | IMA-RGS-001 | 9 | 924.1 | | | water | |
| 10 | IMA-MMP-001 | 16 | 1836.1 | | | water | |
| 11 | IMA-HBV-001 | 10 | 1155.3 | | | 10% acetic acid | |

The drug product IMA is presented as a lyophilisate for intradermal application containing 11 peptides - 578 µg of each peptide - in form of their salts (acetates). For application of the clinical trial formula to the patients the powder for injection containing 578 µg of each peptide will be dissolved in 700 µl sodium hydrogen carbonate (4.2 %). After reconstitution of the solution 500 µl (equals a single dose of 413 µg of each peptide per injection and a total single dose of 4.5 mg IMA per injection) will be injected intradermally.

**Table 7: Other ingredients in IMA**

| | | |
|---|---|---|
| Water for Injection* | Solvent | According to Ph. Eur. |
| Acetic Acid* | Solvent | According to Ph. Eur. |
| Nitrogen* | Inert gas | According to Ph. Eur. |

| | | |
|---|---|---|
| *removed during the manufacturing process | | |

The quality of IMA is guaranteed both by the use of active substances and excipients which meet the requirements of Ph. Eur.

### In vitro immunogenicity of peptides contained in IMA

IMA contains 9 HLA class I tumour-associated peptides, 1 HLA class II tumour-associated peptide and 1 HLA-class I viral control peptide. In vitro immunogenicity could be demonstrated for the vast majority of peptides contained in IMA.

In vitro immunogenicity was demonstrated for 8 of the 10 HLA class I sequences contained in IMA mainly using two T-cell assays: A. cytotoxic killing of target cells in chromium-release assays and/or B. detection of T-cells by HLA tetramers. These assays demonstrate evidence for the presence of specific precursor cells in the blood of HLA-A*02 positive donors as well as the ability of such specific T-cells to kill target cells. As in the latter case also several tumour cell lines endogenously expressing the antigen are recognized. This gives additional (indirect) indications for the natural presentation of the used peptides on tumour cells and shows that cytotoxic T-cells generated using these peptides have a large avidity for the recognition of tumour cells. In vitro immunogenicity was demonstrated for the HLA class II peptide IMA-MMP-001 contained in IMA using intracellular cytokine staining in flow cytometry (see below).

**Table 8: Summary of in vitro immunogencity data for peptides contained in IMA**

| **#** | **Peptide ID** | **In vitro immunogenicity** | **References** |
|---|---|---|---|
| 1 | IMA-ADF-001 | Killer assay | Schmidt et al., 2004 |
| 2 | IMA-ADF-002 | Tetramer detection | unpublished |
| 3 | IMA-APO-001 | n/a | unpublished |
| 4 | IMA-CCN-001 | Killer assay (allogeneic T cells) | Sadovnikova et al., 1998 |
| 5 | IMA-GUC-001 | n/a | unpublished |
| 6 | IMA-K67-001 | Tetramer detection | unpublished |
| 7 | IMA-MET-001 | Killer assay, cytokine staining, tetramer detection | Schag et al., 2004 |
| 8 | IMA-MUC-001 | Killer assay, cytokine release | Brossart et al., 1999 |
| 9 | IMA-RGS-001 | Tetramer detection | unpublished |
| 10 | IMA-MMP-001 | Cytokine staining | unpublished |
| 11 | IMA-HBV-001 | Killer assay | Wentworth et al., 1995 |

Killer assay: cytotoxic killing of target measured by chromium release assay; Cytokine release: release of cytokines by T-cells measured by ELISA; Cytokine staining: synthesis of cytokines by T-cells measured by intracellular flow cytometry; Tetramer detection: detection of peptide-specific T-cells by HLA tetramers.

### In vitro immunogenicity of HLA class I peptides contained in IMA

IMA contains 10 HLA class I-binding peptides. To test the peptides regarding their *in vitro* immunogenicity, CD8-positive cytotoxic T-cells were generated from autologous peripheral blood mononuclear cells (PBMC) from healthy donors using single peptides contained in IMA and the activity of these cytotoxic T-cells was tested with chromium release assays and detection of T-cells with HLA tetramers in flow cytometry. Detailed data is shown for one exemplary peptide (IMA-MET-001) for both methods, the data for the other peptides is summarized in Table 8 above.

In the first step, cytotoxic T-cells are generated (primed) in vitro by repeated stimulation of peripheral blood mononuclear cells (PBMC) from healthy HLA-A*02 positive donors with the specific peptide to be tested. The priming can be done either using autologous dendritic cells generated from blood monocytes of the donor or using HLA tetramer-loaded beads.

A. Cytotoxic killing of target: In the second step, cytoxicity of such primed cytotoxic T-cells (CTLs) are tested by labelling target cells with radioactive chromium and incubating target cells with generated CTLs. The amount of radioactive chromium released into the supernatant can be correlated directly to proportion of killed target cells.

B. Detection of T-cells with HLA tetramers: Alternatively, in the second step primed CTLs with specificity for a given peptide are detected using HLA tetramers. Tetramers consist of four peptide-loaded HLA-A*02 molecules coupled to each other. These constructs allow specific labelling of the cognate T-cell receptor that recognizes the HLA-peptide-complex in the tetramer and by labelling of the tetramer with a fluorochrome followed by analysis in flow cytometry (FACS).

### Priming of cytotoxic T-cells with dendritic cells.

For CTL induction, 5x10⁵ DC were pulsed with 50 µg/ml of the synthetic peptide IMA-MET-001 for 2 h, washed, and incubated with 2.5x10⁶ autologous PBMNC in RP10 medium. After 7 days of culture, cells were restimulated with autologous peptide pulsed PBMNC and 1 ng/ml human recombinant IL-2 (R&D Systems) was added on days 1, 3 and 5. The cytolytic activity of induced CTL was analyzed on day 5 after the last restimulation in a standard ⁵¹Cr-release assay (Brossart 1999). (see below)

### In vitro priming of cytotoxic T-cells with tetramer-loaded beads.

In vitro priming was performed at indicated before (Walter 2003) or with minor modifications. Briefly, biotinylated recombinant HLA-A*0201 molecules lacking the transmembrane domain and being biotinylated at the carboxy terminus of the heavy chain were produced as previously described (Altman 1996). The purified costimulatory mouse IgG2a anti human CD28 Ab 9.3 (Jung 1987) was chemically biotinylated using Sulfo-N-hydroxysuccinimidobiotin under conditions recommended by the manufacturer (Perbio Science, Bonn, Germany). Microspheres used were 5.60 µm diameter streptavidin coated polystyrene particles with a binding capacity of approx. 0.06 µg biotin-FITC/mg microspheres (Bangs Laboratories, Fishers, Illinois/USA). For microsphere handling, a sterile PBS/BSA/EDTA buffer was used. For coupling to biotinylated molecules, microspheres were washed and resuspended at 2 × 10⁶ particles/ml in buffer containing biotinylated MHC and / or antibodies at various concentrations. Binding was allowed at room temperature for 30 min while agitating. Coated beads were washed three times, resuspended in above buffer and stored for up to 4 weeks at 4 °C before use.

PBMCs were isolated from fresh buffy coats using standard gradient separation medium (Linaris, Wertheim-Bettingen, Germany or PAA Laboratories, Linz, Austria). When indicated, untouched CD8 T-cells were magnetically enriched by negative depletion using a CD8 T-cell isolation kit (Miltenyi Biotec, Bergisch Gladbach, Germany) according to the manufacturer's conditions, which resulted in a purity of CD8-positive TCR-positive cells of more than 80%. In vitro stimulations were initiated in 24 well plates with 5 × 10⁶ responder cells plus 1 x 10⁶ APCs or microspheres per well in 1.5 ml T-cell medium. If not stated otherwise, 5 ng/ml human IL-12 p70 (R&D) was added with APCs or microspheres. After 3-4 days co-incubation at 37 °C, fresh medium and 20 U/ml human IL-2 (R&D) was added and cells were further incubated at 37 °C for 3-4 days. This stimulation cycle was repeated twice.

### Killing of target cells by CTLs by using chromium-release assay.

The standard ⁵¹Cr-release assay was performed as described (Brossart 2001). Target cells were pulsed with 50 µg/ml peptide for 2 h and labelled with ⁵¹Cr-sodium chromate in RP10 for 1 h at 37°C. 10⁴ cells were transferred to a well of a round-bottomed 96-well plate. Varying numbers of CTL were added to give a final volume of 200 µl and incubated for 4 h at 37° C. At the end of the assay supernatants (50 µl/well) were harvested and counted in a beta-plate counter. The percent specific lysis was calculated as: 100 x (experimental release - spontaneous release / maximal release-spontaneous release). Spontaneous and maximal release were determined in the presence of either medium or 2% Triton X-100, respectively. Antigen specificity of tumour cell lysis was further determined in a cold target inhibition assay by analyzing the capacity of peptide pulsed unlabeled T2 cells to block lysis of tumour cells at a ratio of 20:1 (inhibitor to target ratio).

### Detection of cytotoxic T-cells with HLA tetramers.

Tetramer staining was performed at indicated before (Walter 2003) or with minor modifications. Briefly, biotinylated recombinant HLA-A*0201 molecules lacking the transmembrane domain and being biotinylated at the carboxy terminus of the heavy chain were produced as previously described (Altman 1996). Fluorescent tetramers were generated by coincubating biotinylated HLA-A*0201 with streptavidin-PE or streptavidin-APC (Molecular Probes, Leiden, The Netherlands) at a 4:1 molar ratio. For tetrameric analyses, cells were washed in PBS/BSA/EDTA containing 10 mg/ml sodium azid (Merck, Darmstadt, Germany) and stained at 4 °C for 20 minutes in the same buffer containing Abs CD4-FITC and CD8-PerCP clone SK1 (both from Becton Dickinson). After microsphere stimulation experiments, 100 µg/ml unlabeled streptavidin (Sigma) was included. Cells were washed in PBS containing 2 % heat-incactivated FCS (PAN Biotech, Aidenbach, Germany), 2 mM sodium EDTA and 10 mg/ml sodium azid and tetramer stained at 4 °C for 30 minutes in PBS/FCS/EDTA/Azid but including 50 % FCS. Tetramer reagents were always used at MHC concentrations of 5 µg/ml. Stained cells were washed extensively in PBS/FCS/EDTA/Azid and fixed with 1 % formaldehyde (Merck). Cells were analyzed on a four-color FACSCalibur (Becton Dickinson). The results are shown exemplary in detail for IMA-MET-001 for method A as well as method B. The results for the other HLA class I peptides are summarized in Table 8.

### A. Cytotoxic killing of target

The results were published by (Schag 2004). The relevant information is summarized in the following.

In the first step, the cytotoxicity of the induced CTL was analyzed in a standard ⁵¹Cr-release assay using peptide loaded T2 cells and autologous DC as targets. As shown in Figure 3, the CTL line obtained after two weekly restimulations demonstrated antigen-specific killing. The T-cells only recognized T2 cells or DC coated with the cognate peptide while they did not lyse target cells pulsed with irrelevant HLA-A2 binding peptides derived from survivin protein or HIV-1 reverse transcriptase confirming the specificity of the cytolytic activity.

In the second step, the ability of the in vitro induced CTL to lyse tumour cells that express the c-Met protein endogenously was analyzed using HLA-A*02 positive cell lines HCT 116 (colon cancer), A498, MZ 1257 (renal cell carcinoma, RCC), MCF-7 (breast cancer), Mel 1479 (malignant melanoma) and U266 (multiple myeloma) that express c-Met as targets in a standard ⁵¹Cr-release assay. The EBV-transformed B-cell line Croft (HLA-2+/c-Met-) and the ovarian cancer cell line SK-OV-3 (HLA-A3+/c-Met+) were included to determine the specificity and HLA-restriction of the CTL. As demonstrated in Figure 4, the c-Met peptide specific CTL were able to efficiently lyse malignant cells expressing both HLA-A2 and c-Met. There was no recognition of the ovarian cancer cells SK-OV-3 or Croft cells demonstrating that the presentation of c-Met peptide in context of HLA-A2 molecules on the tumour cells is required for the efficient lysis of target cells and confirm the antigen specificity and MHC restriction of the CTL. The in vitro induced T-cells did not recognize the K 562 cells indicating that the cytotoxic activity was not NK-cell mediated.

To further verify the antigen specificity and MHC restriction of the in vitro induced CTL lines we performed cold target inhibition assays. The lysis of the target cells (U 266 and A 498) could be blocked in cold target inhibition assays. The addition of cold (not labelled with ⁵¹Cr) T2-cells pulsed with the cognate peptide reduced the lysis of tumour cells whereas T2-cells pulsed with an irrelevant peptide showed no effect (Figure 5)

### B. Detection of T-cells with HLA tetramers

Enriched CD8 T-cells of one A*02+ healthy donor were stimulated 3 times with beads coated in the presence of 10 nM CD28 Ab plus either 10 nM of an irrelevant A*02 complex (left panel) or A*02 refolded with indicated antigens (middle and right panel). Indicated Antigens were peptides NLVPMVATV from CMV pp65 (Wills 1996), modified peptide ELAGIGILTV from Melan-A/MART-1 (Kawakami 1994) and peptide IMA-MET-001. All T-cell lines were surface stained with CD8-PerCP Ab, cognate tetramer-PE (Figure 6; left and middle panel) and irrelevant A*02/ILKEPVHGV tetramer-APC (right panel). Percentage of tetramer+ cells among CD8-positive lymphocytes is indicated in each plot.

### In vitro immunogenicity of the HLA- class II peptide IMA-MMP-001 contained in IMA

IMA contains one HLA class II peptide from matrix metalloproteinase 7, IMA-MMP-001. To test the peptide regarding its *in vitro* immunogenicity and regarding its promiscuous binding characteristics, CD4-positive T-cells were generated from autologous peripheral blood mononuclear cells (PBMC) from healthy donors with different HLA genotypes using the IMA-MMP-001 peptide and the activity of these Helper T-cells tested with intracellular cytokine staining in flow cytometry.

First, CD4-positive T-cells were generated (primed) *in vitro* by repeated stimulation of peripheral blood mononuclear cells (PBMC) from healthy donors with the specific peptide to be tested in the presence of IL-12. The priming was performed using autologous dendritic cells generated from blood monocytes of the donors. In the second step, the activity of primed CD4-positive T-cells specific for the given peptide were tested by measurement of IFNγ production by intracellular IFNγ staining using a fluorescently labelled antibody. The analysis was done by flow cytometry (FACS).

### Generation of dendritic cells (DCs)

Human DCs were prepared out of PBMCs from freshly drawn blood from healthy donors. PBMCs were isolated using a Ficoll density gradient (Lymphocyte Separation Medium, PAA Laboratories GmbH, Pasching, Austria). The obtained cells were washed, resuspended in X-Vivo 15 medium supplemented with 50 U/ml penicillin, 50 µg/ml streptomycin and 2mM L-Glutamine (BioWhittaker, Verviers, Belgium) and plated at a density of 7 × 10⁶ cells/ml. After 2 hours at 37°C, adherent monocytes were cultured for 6 days in X-Vivo medium with 100 ng/ml GM-CSF and 40 ng/ml IL-4 (AL-ImmunoTools, Friesoythe, Germany). On day 7 immature DCs were activated with 10 ng/ml TNF-α (R&D Systems, Wiesbaden, Germany) and 20 µg/ml poly(IC) (Sigma Aldrich, Steinheim, Germany) for 3 days. The differentiation state of DCs was examined by flow cytometry, mature DCs being predominantly CD14-, CD40-positive, CD80-positive, CD83-positive, CD86-positive and HLA-DR+ (data not shown).

### Generation of antigen-specific CD4-positive T-cells

To generate CD4-positive T-cells, 10⁶ PBMCs were stimulated with 2 × 10⁵ autologous DCs. After priming, restimulations were done with cryopreserved autologous PBMCs every 6 to 8 days. For stimulation, cells were pulsed with 5 µg/ml peptide for 90' at 37°C and irradiated (60 Gy; Gammacell 1000 Elite, Nordion International Inc, Ontario, Canada). Cells were incubated in 96-well plates (7 wells per donor and per peptide) with T-cell medium: RPMI 1640 containing HEPES and L-glutamin (Gibco, Paisley, UK) supplemented with 10% heat-inactivated human serum (PAA, Cölbe, Germany), 50 U/ml penicillin, 50 µg/ml streptomycin and 20 µg/ml gentamycin (BioWhittaker) in the presence of 10 ng/ml IL-12 (Promocell, Heidelberg, Germany). After 3 to 4 days of co-incubation at 37°C, fresh medium with 80 U/ml IL-2 (Proleukin, Chiron Corporation, Emeryville, CA, USA) and 5 ng/ml IL-7 (Promocell) was added. Analyses were performed after the third and the fourth stimulation by intracellular IFNγ staining.

### Intracellular IFNγ staining

### Cryopreserved PBMCs were thawed, washed two times in X-Vivo 15 medium, resuspended at 10⁷ cells/ml in T-cell medium and cultured overnight to reduce unspecific IFNγ production (Provenzano 2002). On the next day, PBMCs were pulsed with 5 µg/ml peptide for 2 h, washed three times with X-Vivo 15 medium and incubated with effector cells in a ratio of 1:1 for 6 h. Golgi-Stop (Becton Dickinson, Heidelberg, Germany) was added for the final 4 h of incubation. Cells were analyzed using a Cytofix/Cytoperm Plus kit (Becton Dickinson) and CD4-FITC- (Immunotools), IFNγ-PE- and CD8-PerCP clone SK1-antibodies (Becton Dickinson). After staining, cells were analyzed on a three-color FACSCalibur (Becton Dickinson).

To generate antigen-specific CD4-positive T-cells and to test the peptide on promiscuous binding, PBMCs of 4 healthy donors with different HLA-DR alleles (Figure 7) were stimulated using peptide-pulsed autologous DCs. As a read-out system for the generation of antigen-specific CD4-positive T-cells IFNγ production was assessed by flow cytometry. T-cells were analyzed after the third and the fourth stimulation by intracellular IFNγ staining plus CD4-FITC and CD8-PerCP staining to determine the percentage of IFNγ-producing cells in specific T-cell subpopulations. In all experiments, stimulations with irrelevant peptide and without peptide were performed as negative controls. IFNγ response was considered as positive if the detection of IFNγ producing CD4-positive T-cells was more than two fold higher compared to negative control. (Horton 2004). In three of four donors we were able to generate CD4-positive T-cells specifically reacting to the peptide of interest (Figure 7). T-cell responses could not be observed in Donor 4 neither after the third nor after the fourth stimulation. The highest frequencies of IFNγ producing CD4-positive T-cells specific for IMA-MMP-001 were seen in Donor 1 and 2, respectively.

Thus, IMA-MMP-001 is a promiscuous binder being able to elicit CD4-positive T-cell responses in three out of four healthy donors carrying different HLA alleles. According to the binding predictions and the obtained results, it is highly likely that the peptide is presented by HLA-DRB1*0101, HLA-DRB1*0401/*0408 and HLA-DRB1*1101. All four alleles have a Glycine residue at position 86 and an Aspartic acid residue at position 57 of their β chains (data not shown). Therefore, they have very similar binding motives sharing binding characteristics for their binding pockets P1 and P4 (Rammensee 1997; Hammer 1993; Marshall 1994). Donor 4 carries with HLA-DRB1*0318 and DRB1*1401 alleles with very different binding motifs. This would explain why it was not possible to elicit a T-cell response with cells from this donor using the peptides mentioned above.

### Effects in Humans

Short peptides similar in length and amino acid distribution to those described here have been immunized in thousands of patients in various phase 1 to 3 clinical trials since 1996. In none of these studies any severe adverse events were reported. Additionally, the peptide IMA-MUC-001 contained in IMA has already been used for vaccination being loaded on dendritic cells in an investigator initiated trial at the University of Tübingen, Germany and were very well tolerated (Wierecky 2005).

The peptides with SEQ-ID-Nr. 1, 2, 3, 4, 5, 6, 7, 8, 9, 10 were tested in in 30 patients, 26 of which completed a full course of vaccination over a period of ten weeks. A high proportion of patients, 74%, showed a specific immune response against peptide sequences included in the vaccine. Already in this small phase I cohort, the in vivo biological function of 8 of the 9 peptides (SEQ-ID-Nr. 2, 3, 4, 5, 6, 7, 8, 9) binding to MHC class I (allele HLA-A*02) could be confirmed according to amplified ELISPOT and/or tetramer staining assay results conducted as described below. The peptide with SEQ-ID-Nr. 1 (IMA-MMP-001) for which a different read-out would have been required could not be tested due to limited availability of PBMC from patient blood.

### Specific Immunologic Parameters (Immunomonitoring)

To date, immunomonitoring has been a common practice in thousands of patients in various therapeutic vaccination studies (Romero 2004). Many different immunomonitoring methods have been reported to date, including functional and specific assays. The immunogenic components of the therapeutic vaccine IMA are HLA-binding peptides which are supposed to induce specific T lymphocytes in vivo. This activation will lead to their proliferation and acquisition of effector functions, which includes their ability to secrete cytokines upon antigen contact.

As a surrogate marker for T-cell activation the frequency of specific cytokine secreting mononuclear cells in blood can be tested using ELISPOT assays. Such assays are especially appropriate for this purpose as they are single cell based and result in a parameter (i. e. the number of spot forming cells among mononuclear cells) that is expected to be directly correlated to the true frequency of cytokine secreting antigen specific T lymphocytes in blood samples. As such, assays also enable the processing of relatively large numbers of samples and peptides in parallel, they have widely been used already for immunomonitoring studies in a large number of clinical studies so far (Schmittel 2000).

### Immune Response

The immunological activity/efficacy can be described by T-cell response analysis. Experience and results from different clinical studies regarding immune response for different indications can be found in the literature. T-cell responses of up to 100 % are described by Disis et al., 1999, in patients with ovarian and breast cancer. Epitope spreading in 84% of the patients (n = 64) in a 3-arms study with Her2/neu antigen plus GM-CSF was reported by Disis et al. in 2002 in patients with ovarian, breast and non-small-cell lung cancer. Other authors have published results regarding T-cell responses between 33% and 83% in patients with melanoma (Keilholz/Schadendorf, 2003; Slingluff et al., 2004). Gaudernack/Gjertsen, 2003 report about an immune response of several CD4-positive and CD8-positive T-cell clones specific for the antigen used in this study.

Also, results were presented by Wierecky et al., ASCO 2005: autologous mature monocyte derived dendritic cells (DC) were pulsed with two HLA-A2 binding peptides deduced form MUC1 peptide. For the recruiting and activation of CD4-positive T-cells DC were further incubated with the PAN-DR binding peptides PADRE. Vaccinations were performed s. c. every two weeks, four times, and repeated monthly until tumour progression in this therapeutic approach. After the 5th DC injection patients additionally received 3 injections/week of low dose IL-2 (1 Mio IE/m2). The enhancement of T-cell precursor was monitored using IFN-γ ELISPOT and ⁵¹Cr-release assays. Furthermore the ability of PBMC to produce cytokines in response to challenge with vaccine-related epitopes was tested by quantitative real-time PCR.

In this study by Wierecky et al., MUC1 peptide specific T-cell responses in vivo were detected in the PBMC of all patients with OR. These in vivo induced T-cells were able to recognize target cells pulsed with the cognate peptide or matched allogeneic tumour cells (A498) constitutively expressing MUC1 in an antigen and HLA restricted manner after in vivo restimulation. In patients responding to the treatment, T-cell responses to antigens not used for vaccination like adipophilin, telomerase or OFA could be detected indicating that epitope spreading might occur. Proliferative response to the PADRE peptide were detectable in 11/16 patients, in some patients already after the 2nd vaccination. Conclusion: analysis of epitope spreading in vaccinated patients might be a useful parameter to correlate clinical and immunological responses.

### IMMUNE RESPONSES IN VIVO

### Clinical Trial Formulation

The clinical trial formulation of IMA consists of:
- Lyophilisate including 11 peptides in 3 ml vials
- Diluent (Sodium hydrogen carbonate 4.2 %)

### Dosage forms of IMA

Powder for injection in 3 ml glass-vials. Packaging information: 4 vials are packed into boxes. The diluent consists of 700 µl sodium hydrogen carbonate packed as 250 ml bottles. 1 vial of IMA is reconstituted by the addition of 700 µl of 4.2 % sodium hydrogen carbonate solution (diluent). In order to dissolve IMA the vial and the diluent is shaken vigorously for 3 minutes and treated by ultra sonic for 1 minute. Thereafter the vial is shaken again for 1 minute. 500 µl of this solution are administered within 30 minutes after reconstitution.

### Vaccination

In this study advanced renal cell carcinoma patients received eight vaccinations over a time period of ten weeks. Altogether, a total of 24 patients of the HLA-A*02 positive HLA type. had been enrolled. An intradermal vaccination (GM-CSF plus IMA) was given at day 1, 2, 3, 8, 15, 22, 36, and day 64. Blood samples were taken a different time points during the study and T-cells contained within the patient's peripheral blood mononuclear cells (PBMCs) were isolated from heparin blood by density gradient centrifugation, counted using hemocytometers and were preserved to be stored at cryogenic temperature until assayed. Two different routine ELISPOT assays and one routine tetramer assays were then performed by the applicant.

### Amplified ELISPOT assay

In the "ex vivo ELISPOT" assay, cells are thawed from different time points, the number of live cells is counted and samples are assayed by one-day incubation with different peptides or controls in triplicate wells. The ex vivo ELISPOT assay delivers quantitative data in a much quicker fashion compared to the other assays below. Additionally, this is the only assay that allows measurement of the one HLA class II peptide (IMA-MMP-001) contained in IMA. However, this assay is of limited sensitivity and positive data was only expected in the case of very strong T-cell responses comparable to memory (recall) immune responses to viruses.

In the "amplified ELISPOT" assay, cells from different time points are pooled, counted and pre-stimulated with antigens for approximately two weeks to allow specific cells to divide. Cells are then harvested from culture, recounted and then assayed as above for IFN-γ spot production upon one-day restimulation with antigen. Cells that are activated under such conditions secrete IFN-γ which is detected by an enzyme linked sandwich antibody method. Spots are visualized by a colour forming reaction and counted with an automated high resolution digital camera (herein called "ELISPOT reader"). The number of spots that correlates to the true frequency of activated antigen specific T lymphocytes in the sample is determined by a software algorithm from images taken by the ELISPOT reader. This assay has often been used to detect T-cell response to vaccinated tumour antigens in various third-party clinical trials. The possibility of false-positive data due to "in vitro priming" of T-cells is excluded by using various controls in this assay. Compared to the Tetramer Assay below, this assay gives additional functional information, i.e. IFN-γ cytokine release.

The amplified ELISPOT assay was performed for 28 patients in the study and all antigens present in IMA prior and during the vaccination protocol at different time points. Figure 10 shows representative examples of an IMA induced T-cell response identified by the amplified ELISPOT assay for the same patient and antigen. Upper and lower column represent negative control antigen HIV-001 and single TUMAP IMA-CCN-001 used for readout, respectively. The number of positive cells is given for each experiment. The left column shows ELISPOTs of pooled samples taken before vaccination while the right column shows ELISPOTs of pooled samples taken during the vaccination protocol. While the control antigen did not lead to an increase in the number of spots after the induction of an immune response, injection of IMA led to a multiplication of spot numbers for IMA-CCN-001. The number of positive, i.e. IFN-γ secreting cells, increased from 27-34 prior to vaccination to 100-141 after fourth and fifth injection. Treatment with IMA resulted in an increased frequency of activated T lymphocytes specific for the IMA-CCN-001 antigen in this patient.

### Amplified tetramer assay

In the "amplified Tetramer" assay cells from different time points were thawn, counted and pre-stimulated with antigens for approximately two weeks to allow antigen-specific T-cells to divide. Cells are then harvested from culture, recounted and stained with PE- and APC-fluorochrome labeled MHC multimers plus antibodies to define CD8+ T-cells (one well per staining & timepoint). MHC multimers are recombinant peptide-MHC complexes that are multimerized and conjugated to a fluorescent dye. Only tetrameric HLA-A*0201 multimers that are fully equivalent to those originally introduced to the field (Altmann 1996) which are herein briefly called Tetramers were used. Stained and fixed samples were analyzed on a flow cytometer using standard procedures well known in the art, resulting in a single cell based dataset for each sample. This "amplified Tetramer" assay is of very high sensitivity but less quantitative compared to ex vivo assays.

For primary analysis of tetramer data the number of totally evaluated CD8+ T-cells, single-tetramer positive and double-tetramer positive cells per sample was electronically counted from cytometry list mode files using commercial software. CD8+ T-cells were identified by forward and sideward scatter gating on live lymphocytes and subgating on CD8+ CD3+ events based on antibody fluorescence. Definition of lymphocyte gates and CD3/CD8 gates was identical for all stainings of one patient within an assay. Tetramer positive populations were identified from CD8+ T-cells by analyzing double tetramer dot plots with quadrants or gates. Definition of tetramer+ cells was identical for each staining condition for a given patient in an assay and and based on recognizable cell populations. The amplified Tetramer assay was performed for 28 patients in the study and all antigens present in IMA prior and during the vaccination protocol at different time points.

Figure 11 shows two representative examples of IMA induced T-cell responses identified by the amplified Tetramer staining assay. Upper and middle panels represent two-dimensional dot plots gated on CD3+ lymphocytes, lower panels are gated on CD3+ CD8+ lymphocytes. Patients, timepoints and stainings were as indicated for each column. In Figure 11 A the immunological response to IMA-CCN-001 in patient 03-004, that was already shown by ELISPOT assay (Figure 10) was confirmed by tetramer assay. A cell population positive for CD3+ and IMA-CCN-001 tetramer was identified after the forth and fifth injection of IMA (V6+V7; middle panel) while no positive population was found for the K67-001 tetramer (upper panel). The IMA-CCN-001 positive cells increased from 0.03% prior vaccination to 0.78% of the lymphocytes (V6+V7; lower panel) after the first three injections.

Patient 03-003 exhibited no immunological response against RGS-001 peptide (Fig. 11B; upper panel) but developed IMA-CCN-001 tetramer positive response during the time course of the vaccination protocol (S1+V1: samples taken prior to vaccination; V4+V5: samples taken at day 8 and day 15; V6+V7: samples taken on day 22 and day 36; V8+FU: samples taken on day 64 (last vaccination) and after 85 to 92 days; end of study). In the middle panel, column 3 and 4 show distinct cell populations positive for CNN-001 and CD3+. During the time course of vaccination the amount of these cells increased from 0.02% prior vaccination to 0.8 % of the lymphocytes (lower panel; column 3) after the first three injections and decreased to 0.31% after day 64 (lower panel; column 4; V8+FU).

For selected patients the amplified tetramer assay was performed for single time points, i.e. blood samples were not pooled, allowing a more precise evaluation of the T-cell kinetics an example of which is depicted in Figure 12. The observed T-cell magnitude kinetics in single time point amplified tetramer assays are shown for patient 05-001. The tumour associated antigens present in IMA (TUMAP pool) were highest on day 22, 36 and 64 (V6, V7 and V8) while response to the IMA-CCN-001 peptide peaked earlier at day 22 (V6). The HBV-001 positive control resulted in an even faster response that reached its maximum at day 15 (V5).

Two patients with a very high response to IMA-CCN-001 were selected to confirm the results of the amplified tetramer assay in a non amplified experiment. These ex vivo tetramer assays were performed without cultivating the cells for two weeks.

Results are summarised in Table 9 below. Shown are all evaluable results from ex vivo tetramer and patient / antigen matched amplified tetramer assays where a vaccine-induced response was detected in the amplified assay. For the "ex vivo" method, % Tetramer+ among total CD8+ T-cells is indicated. As for the "amplified, routine" evaluation method, subpopulations of CD8+ T-lymphocytes may be analyzed, a second re-evaluation of the routine data is shown ("amplified, quantitative") with calculations based on total CD8+ T-lymphocytes. The "amplification factor" was calculated if a discrete tetramer+ population was seen in ex vivo and amplified tetramer assay.

The results clearly demonstrate that immunological responses measured with the amplified tetramer assay are not due to cell expansion during the two week incubation but are based on previously, "ex vivo" lymphocytes present in the patients blood.

**Table 9: Comparison of T-cell response magnitudes calculated from ex vivo and amplified tetramer assays.**

| **PATIENT** | **Assay ID** | **Timepoint** | **Method** | **Antigen** | **% Tetramer+ among CD8+ T-lymphocytes** | **Amplification factor** |
|---|---|---|---|---|---|---|
| **01-001** | TET-0013/20060511a | S2 | ex vivo | MUC-001 | 0,003 | |
| **01-001** | TET-0013/20060511a | V1 | ex vivo | MUC-001 | 0,004 | |
| **01-001** | TET-0013/20060511a | V4 | ex vivo | MUC-001 | 0,007 | |
| **01-001** | TET-0013/20060511a | V5 | ex vivo | MUC-001 | 0,003 | |
| **01-001** | TET-0013/20060511a | V6 | ex vivo | MUC-001 | 0,001 | |
| **01-001** | TET-0013/20060511a | V7 | ex vivo | MUC-001 | 0,003 | |
| **01-001** | TET-0013/20060511a | V8 | ex vivo | MUC-001 | 0,004 | |
| **01-001** | TET-0013/20060511a | FU | ex vivo | MUC-001 | 0,005 | |
| **01-001** | TET-0001/20060425a | S2;V1 | amplified, routine | MUC-001 | 2,976* | |
| **01-001** | TET-0001/20060425a | V4;V5 | amplified, routine | MUC-001 | 2,391* | |
| **01-001** | TET-0001/20060425a | V6;V7 | amplified, routine | MUC-001 | 4,502* | |
| **01-001** | TET-0001/20060425a | V8;FU | amplified, routine | MUC-001 | 4,900* | |
| **01-001** | TET-0001/20060425a | S2;V1 | amplified, quantitative | MUC-001 | 3,044* | |
| **01-001** | TET-0001/20060425a | V4;V5 | amplified, quantitative | MUC-001 | 2,485* | |
| **01-001** | TET-0001/20060425a | V6;V7 | amplified, quantitative | MUC-001 | 4,545* | |
| **01-001** | TET-0001/20060425a | V8;FU | amplified, quantitative | MUC-001 | 5,037* | |
| **01-003** | 20060727a | V1 | ex vivo | HBV-001 | 0,006 | |
| **01-003** | 20060727a | V7 | ex vivo | HBV-001 | 0,021 | |
| **01-003** | TET-0007/20060510b | V1 | amplified, quantitative | HBV-001 | 0,036* | |
| **01-003** | TET-0007/20060510b | V7 | amplified, quantitative | HBV-001 | 2,587* | 123 |
| **01-003** | TET-0007/20060510b | V1 | amplified, routine | HBV-001 | (0,084*) | |
| **01-003** | TET-0007/20060510b | V7 | amplified, routine | HBV-001 | (5,186*) | (247) |
| **01-009** | 20060727a | V1 | ex vivo | rCCN-001 | 0,010* | |
| **01-009** | 20060727a | V5 | ex vivo | rCCN-001 | 0,092* | |
| **01-009** | 20060727a | V6 | ex vivo | rCCN-001 | 0,052* | |
| **01-009** | TET-0026/20060711a | V1 | amplified, quantitative | TUMAP Pool# | 0,051 | |
| **01-009** | TET-0026/20060711a | V5 | amplified, quantitative | TUMAP Pool# | 5,323* | 58 |
| **01-009** | TET-0026/20060711a | V6 | amplified, quantitative | TUMAP Pool# | 1,398* | 27 |
| **01-009** | TET-0026/20060711a | V1 | amplified, routine | TUMAP Pool# | (0,059) | |
| **01-009** | TET-0026/20060711a | V5 | amplified, routine | TUMAP Pool# | (16,961*) | (184) |
| **01-009** | TET-0026/20060711a | V6 | amplified, routine | TUMAP Pool# | (6,181*) | (119) |
| **03-009** | 20060727a | V1 | ex vivo | rCCN-001 | 0,009 | |
| **03-009** | 20060727a | V7 | ex vivo | rCCN-001 | 0,034* | |
| **03-009** | TET-0015/20060531a | V1 | amplified, quantitative | TUMAP Pool# | 0,124 | |
| **03-009** | TET-0015/20060531a | V7 | amplified, quantitative | TUMAP Pool# | 7,521* | 221 |
| **03-009** | TET-0015/20060531a | V1 | amplified, routine | TUMAP Pool# | (0,247) | |
| **03-009** | TET-0015/20060531a | V7 | amplified, routine | TUMAP Pool# | (18,953*) | (557) |

| | | | | | | |
|---|---|---|---|---|---|---|
| *: Discrete tetramer+ population detected #: Separate assay showed clear evidence that the TUMAP Pool response could be solely contributed to rCCN-001. | | | | | | |

### Overall Patient Responsiveness

T-cell responses as measured by the assays described above were evaluated for all peptides contained in IMA for 28 patients at the different time points of the study. A patient was scored "responsive", i.d. showed a vaccine induced immune response, if one of the blood samples taken at an after vaccination time point contained teramer-positive lymphocytes or secreted IFN-γ upon stimulation with one of the peptides.

As expected, patients reacted individually towards the different peptides contained in IMA. Taking into account the small number of patients a surprisingly good responsiveness was achieved since the majority of patients (23 out of 27 evaluable patients) developed an immune response to at least one of the peptides. 8 of 27 evaluable patients (30%) even showed a T-cell response against multiple TUMAPs.

### Literature

Altman JD, Moss PA, Goulder PJ, Barouch DH, McHeyzer-Williams MG, Bell JI, McMichael AJ, and Davis MM. Phenotypic analysis of antigen-specific T lymphocytes. Science 274:94-96 (1996).

Apostolopoulos V and McKenzie IF. Cellular mucins: targets for immunotherapy. Crit Rev. Immunol. 14:293-309 (1994).

Bamias A, Chorti M, Deliveliotis C, Trakas N, Skolarikos A, Protogerou B, Legaki S, Tsakalou G, Tamvakis N, and Dimopoulos MA. Prognostic significance of CA 125, CD44, and epithelial membrane antigen in renal cell carcinoma. Urology 62:368-373 (2003).

Barnd DL, Lan MS, Metzgar RS, and Finn OJ. Specific, Major Histocompatibility Complex-Unrestricted Recognition of Tumor-Associated Mucins by Human Cytotoxic T-cells. PNAS 86:7159-7163 (1989).

Bates S, Bonetta L, MacAllan D, Parry D, Holder A, Dickson C, and Peters G. CDK6 (PLSTIRE) and CDK4 (PSK-J3) are a distinct subset of the cyclin-dependent kinases that associate with cyclin D1. Oncogene 9:71-79 (1994).

Beilmann M, Vande Woude GF, Dienes HP, and Schirmacher P. Hepatocyte growth factor-stimulated invasiveness of monocytes. Blood 95:3964-3969 (2000).

Berger M, Bergers G, Arnold B, Hammerling GJ, and Ganss R. Regulator of G-protein signaling-5 induction in pericytes coincides with active vessel remodeling during neovascularization. Blood 105:1094-1101 (2005).

Bertoletti A, Chisari FV, Penna A, Guilhot S, Galati L, Missale G, Fowler P, Schlicht HJ, Vitiello A, Chesnut RC, and Definition of a minimal optimal cytotoxic T-cell epitope within the hepatitis B virus nucleocapsid protein. J Virol. 67:2376-2380 (1993).

Bladt F, Riethmacher D, Isenmann S, Aguzzi A, and Birchmeier C. Essential role for the c-met receptor in the migration of myogenic precursor cells into the limb bud. Nature 376:768-771 (1995).

Borset M, Seidel C, Hjorth-Hansen H, Waage A, and Sundan A. The role of hepatocyte growth factor and its receptor c-Met in multiple myeloma and other blood malignancies. Leuk. Lymphoma 32:249-256 (1999).

Bottaro DP, Rubin JS, Faletto DL, Chan AM, Kmiecik TE, Vande Woude GF, and Aaronson SA. Identification of the hepatocyte growth factor receptor as the c-met proto-oncogene product. Science 251:802-804 (1991).

Bramhall SR, Neoptolemos JP, Stamp GW, and Lemoine NR. Imbalance of expression of matrix metalloproteinases (MMPs) and tissue inhibitors of the matrix metalloproteinases (TIMPs) in human pancreatic carcinoma. J Pathol. 182:347-355 (1997).

Brossart P, Heinrich KS, Stuhler G, Behnke L, Reichardt VL, Stevanovic S, Muhm A, Rammensee HG, Kanz L, and Brugger W. Identification of HLA-A2-restricted T-cell epitopes derived from the MUC1 tumor antigen for broadly applicable vaccine therapies. Blood 93:4309-4317 (1999).

Brossart P, Schneider A, Dill P, Schammann T, Grunebach F, Wirths S, Kanz L, Buhring HJ, and Brugger W. The epithelial tumor antigen MUC1 is expressed in hematological malignancies and is recognized by MUC1-specific cytotoxic T-lymphocytes. Cancer Res. 61:6846-6850 (2001).

Brossart P, Wirths S, Stuhler G, Reichardt VL, Kanz L, and Brugger W. Induction of cytotoxic T-lymphocyte responses in vivo after vaccinations with peptide-pulsed dendritic cells. Blood 96:3102-3108 (2000).

Browner MF, Smith WW, and Castelhano AL. Matrilysin-inhibitor complexes: common themes among metalloproteases. Biochemistry 34:6602-6610 (1995).

Cao Y, Karsten U, Zerban H, and Bannasch P. Expression of MUC1, Thomsen-Friedenreich-related antigens, and cytokeratin 19 in human renal cell carcinomas and tubular clear cell lesions. Virchows Arch. 436:119-126 (2000).

Chen X, Higgins J, Cheung ST, Li R, Mason V, Montgomery K, Fan ST, van de RM, and So S. Novel endothelial cell markers in hepatocellular carcinoma. Mod. Pathol. 17:1198-1210 (2004).

De VL, Zheng B, Fischer T, Elenko E, and Farquhar MG. The regulator of G protein signaling family. Annu. Rev. Pharmacol. Toxicol. 40:235-271 (2000).

Delsol G, Al ST, Gatter KC, Gerdes J, Schwarting R, Caveriviere P, Rigal-Huguet F, Robert A, Stein H, and Mason DY. Coexpression of epithelial membrane antigen (EMA), Ki-1, and interleukin-2 receptor by anaplastic large cell lymphomas. Diagnostic value in so-called malignant histiocytosis. Am. J. Pathol. 130:59-70 (1988).

Denys H, De Wever O, Nusgens B, Kong Y, Sciot R, Le AT, Van Dam K, Jadidizadeh A, Tejpar S, Mareel M, Alman B, and Cassiman JJ. Invasion and MMP expression profile in desmoid tumours. Br. J Cancer 90:1443-1449 (2004).

Deshpande A, Sicinski P, and Hinds PW. Cyclins and cdks in development and cancer: a perspective. Oncogene 24:2909-2915 (2005).

Di Renzo MF, Olivero M, Giacomini A, Porte H, Chastre E, Mirossay L, Nordlinger B, Bretti S, Bottardi S, Giordano S, and Overexpression and amplification of the met/HGF receptor gene during the progression of colorectal cancer. Clin. Cancer Res. 1:147-154 (1995).

Dittmann J, Keller-Matschke K, Weinschenk T, Kratt T, Heck T, Becker HD, Stevanovic S, Rammensee HG, and Gouttefangeas C. CD8(+) T-cell response against MUC1-derived peptides in gastrointestinal cancer survivors. Cancer Immunol Immunother. (2004).

Dong G, Chen Z, Li ZY, Yeh NT, Bancroft CC, and Van WC. Hepatocyte growth factor/scatter factor-induced activation of MEK and PI3K signal pathways contributes to expression of proangiogenic cytokines interleukin-8 and vascular endothelial growth factor in head and neck squamous cell carcinoma. Cancer Res. 61:5911-5918 (2001).

Duchateau PN, Pullinger CR, Cho MH, Eng C, and Kane JP. Apolipoprotein L gene family: tissue-specific expression, splicing, promoter regions; discovery of a new gene. J. Lipid Res. 42:620-630 (2001).

Duchateau PN, Pullinger CR, Orellana RE, Kunitake ST, Naya-Vigne J, O'Connor PM, Malloy MJ, and Kane JP. Apolipoprotein L, a new human high density lipoprotein apolipoprotein expressed by the pancreas. Identification, cloning, characterization, and plasma distribution of apolipoprotein L. J. Biol. Chem. 272:25576-25582 (1997).

Duperray C, Klein B, Durie BG, Zhang X, Jourdan M, Poncelet P, Favier F, Vincent C, Brochier J, Lenoir G, and. Phenotypic analysis of human myeloma cell lines. Blood 73:566-572 (1989).

Falk K, Rotzschke O, Stevanovic S, Jung G, and Rammensee HG. Allele-specific motifs revealed by sequencing of self peptides eluted from MHC molecules. Nature 351:290-296 (1991).

Ferracini R, Di Renzo MF, Scotlandi K, Baldini N, Olivero M, Lollini P, Cremona O, Campanacci M, and Comoglio PM. The Met/HGF receptor is over-expressed in human osteosarcomas and is activated by either a paracrine or an autocrine circuit. Oncogene 10:739-749 (1995).

Finn OJ, Jerome KR, Henderson RA, Pecher G, Domenech N, Magarian-Blander J, and Barratt-Boyes SM. MUC-1 epithelial tumor mucin-based immunity and cancer vaccines. Immunol. Rev. 145:61-89 (1995).

Fischer J, Palmedo G, von KR, Bugert P, Prayer-Galetti T, Pagano F, and Kovacs G. Duplication and overexpression of the mutant allele of the MET proto-oncogene in multiple hereditary papillary renal cell tumours. Oncogene 17:733-739 (1998).

Fujita K, Denda K, Yamamoto M, Matsumoto T, Fujime M, and Irimura T. Expression of MUC1 mucins inversely correlated with post-surgical survival of renal cell carcinoma patients. Br. J. Cancer 80:301-308 (1999).

Furge KA, Zhang YW, and Vande Woude GF. Met receptor tyrosine kinase: enhanced signaling through adapter proteins. Oncogene 19:5582-5589 (2000).

Furge KA, Kiewlich D, Le P, Vo MN, Faure M, Howlett AR, Lipson KE, Woude GFV, and Webb CP. Suppression of Ras-mediated tumorigenicity and metastasis through inhibition of the Met receptor tyrosine kinase. PNAS 98:10722-10727 (2001).

Furuya M, Nishiyama M, Kimura S, Suyama T, Naya Y, Ito H, Nikaido T, and Ishikura H. Expression of regulator of G protein signalling protein 5 (RGS5) in the tumour vasculature of human renal cell carcinoma. J. Pathol. 203:551-558 (2004).

Gaire M, Magbanua Z, McDonnell S, McNeil L, Lovett DH, and Matrisian LM. Structure and expression of the human gene for the matrix metalloproteinase matrilysin. J Biol. Chem. 269:2032-2040 (1994).

Gendler S, Taylor-Papadimitriou J, Duhig T, Rothbard J, and Burchell J. A highly immunogenic region of a human polymorphic epithelial mucin expressed by carcinomas is made up of tandem repeats. J. Biol. Chem. 263:12820-12823 (1988).

Gherardi E and Stoker M. Hepatocyte growth factor--scatter factor: mitogen, motogen, and met. Cancer Cells 3:227-232 (1991).

Girling A, Bartkova J, Burchell J, Gendler S, Gillett C, and Taylor-Papadimitriou J. A core protein epitope of the polymorphic epithelial mucin detected by the monoclonal antibody SM-3 is selectively exposed in a range of primary carcinomas. Int. J. Cancer 43:1072-1076 (1989).

Gursky S, Olopade OI, and Rowley JD. Identification of a 1.2 Kb cDNA fragment from a region on 9p21 commonly deleted in multiple tumor types. Cancer Genet. Cytogenet. 129:93-101 (2001).

Halaban R. Melanoma cell autonomous growth: the Rb/E2F pathway. Cancer Metastasis Rev. 18:333-343 (1999).

Hammer J, Valsasnini P, Tolba K, Bolin D, Higelin J, Takacs B, and Sinigaglia F. Promiscuous and allele-specific anchors in HLA-DR-binding peptides. Cell 74:197-203 (1993).

Hedberg Y, Davoodi E, Roos G, Ljungberg B, and Landberg G. Cyclin-D1 expression in human renal-cell carcinoma. Int. J. Cancer 84:268-272 (1999).

Heid HW, Moll R, Schwetlick I, Rackwitz HR, and Keenan TW. Adipophilin is a specific marker of lipid accumulation in diverse cell types and diseases. Cell Tissue Res. 294:309-321 (1998).

Horton H, Russell N, Moore E, Frank I, Baydo R, Havenar-Daughton C, Lee D, Deers M, Hudgens M, Weinhold K, and McElrath MJ. Correlation between interferon- gamma secretion and cytotoxicity, in virus-specific memory T-cells. J. Infect. Dis. 190:1692-1696 (2004).

Jadeski LC, Chakraborty C, and Lala PK. Nitric oxide-mediated promotion of mammary tumour cell migration requires sequential activation of nitric oxide synthase, guanylate cyclase and mitogen-activated protein kinase. Int. J. Cancer 106:496-504 (2003).

Jager, E., Y. Nagata, S. Gnjatic, H. Wada, E. Stockert, J. Karbach, P. R. Dunbar, S. Y. Lee, A. Jungbluth, D. Jager, M. Arand, G. Ritter, V. Cerundolo, B. Dupont, Y. T. Chen, L. J. Old, and A. Knuth. Monitoring CD8 T-cell responses to NY-ESO-1: correlation of humoral and cellular immune responses. Proc. Natl. Acad. Sci. U. S. A 97:4760 (2000).

Jucker M, Gunther A, Gradl G, Fonatsch C, Krueger G, Diehl V, and Tesch H. The Met/hepatocyte growth factor receptor (HGFR) gene is overexpressed in some cases of human leukemia and lymphoma. Leuk. Res. 18:7-16 (1994).

Jung G, Ledbetter JA, and Muller-Eberhard HJ. Induction of cytotoxicity in resting human T lymphocytes bound to tumor cells by antibody heteroconjugates. Proc. Natl. Acad. Sci. U. S. A 84:4611-4615 (1987).

Kawakami Y, Eliyahu S, Sakaguchi K, Robbins PF, Rivoltini L, Yannelli JR, Appella E, and Rosenberg SA. Identification of the immunodominant peptides of the MART-1 human melanoma antigen recognized by the majority of HLA-A2-restricted tumor infiltrating lymphocytes. J Exp. Med. 180:347-352 (1994).

Koochekpour S, Jeffers M, Rulong S, Taylor G, Klineberg E, Hudson EA, Resau JH, and Vande Woude GF. Met and hepatocyte growth factor/scatter factor expression in human gliomas. Cancer Res. 57:5391-5398 (1997).

Kraus S, Abel PD, Nachtmann C, Linsenmann HJ, Weidner W, Stamp GW, Chaudhary KS, Mitchell SE, Franke FE, and Lalani e. MUC1 mucin and trefoil factor 1 protein expression in renal cell carcinoma: correlation with prognosis. Hum. Pathol. 33:60-67 (2002).

Kurokawa Y, Matoba R, Nakamori S, Takemasa I, Nagano H, Dono K, Umeshita K, Sakon M, Monden M, and Kato K. PCR-array gene expression profiling of hepatocellular carcinoma. J. Exp. Clin. Cancer Res. 23:135-141 (2004).

Lemmel C, Weik S, Eberle U, Dengjel J, Kratt T, Becker HD, Rammensee HG, and Stevanovic S. Differential quantitative analysis of MHC ligands by mass spectrometry using stable isotope labeling. Nat. Biotechnol. 22:450-454 (2004).

Leroy X, Copin MC, Devisme L, Buisine MP, Aubert JP, Gosselin B, and Porchet N. Expression of human mucin genes in normal kidney and renal cell carcinoma. Histopathology 40:450-457 (2002).

Lew DJ, Dulic V, and Reed SI. Isolation of three novel human cyclins by rescue of G1 cyclin (Cln) function in yeast. Cell 66:1197-1206 (1991).

Li G, Schaider H, Satyamoorthy K, Hanakawa Y, Hashimoto K, and Herlyn M. Downregulation of E-cadherin and Desmoglein 1 by autocrine hepatocyte growth factor during melanoma development. Oncogene 20:8125-8135 (2001).

Lin TS, Chiou SH, Wang LS, Huang HH, Chiang SF, Shih AY, Chen YL, Chen CY, Hsu CP, Hsu NY, Chou MC, Kuo SJ, and Chow KC. Expression spectra of matrix metalloproteinases in metastatic non-small cell lung cancer. Oncol Rep. 12:717-723 (2004).

Livingston BD, Crimi C, Grey H, Ishioka G, Chisari FV, Fikes J, Grey H, Chesnut RW, and Sette A. The hepatitis B virus-specific CTL responses induced in humans by lipopeptide vaccination are comparable to those elicited by acute viral infection. J Immunol. 159:1383-1392 (1997).

Loden M, Stighall M, Nielsen NH, Roos G, Emdin SO, Ostlund H, and Landberg G. The cyclin D1 high and cyclin E high subgroups of breast cancer: separate pathways in tumorogenesis based on pattern of genetic aberrations and inactivation of the pRb node. Oncogene 21:4680-4690 (2002).

Louhelainen J, Wijkstrom H, and Hemminki K. Initiation-development modelling of allelic losses on chromosome 9 in multifocal bladder cancer. Eur. J. Cancer 36:1441-1451 (2000).

Mark AS and Mangkornkanok M. B-cell lymphoma marking only with anti-epithelial membrane antigen. Cancer 63:2152-2155 (1989).

Marshall KW, Liu AF, Canales J, Perahia B, Jorgensen B, Gantzos RD, Aguilar B, Devaux B, and Rothbard JB. Role of the polymorphic residues in HLA-DR molecules in allele-specific binding of peptide ligands. J. Immunol. 152:4946-4957 (1994).

Maulik G, Kijima T, Ma PC, Ghosh SK, Lin J, Shapiro GI, Schaefer E, Tibaldi E, Johnson BE, and Salgia R. Modulation of the c-Met/hepatocyte growth factor pathway in small cell lung cancer. Clin. Cancer Res. 8:620-627 (2002).

Miyazaki K, Hattori Y, Umenishi F, Yasumitsu H, and Umeda M. Purification and characterization of extracellular matrix-degrading metalloproteinase, matrin (pump-1), secreted from human rectal carcinoma cell line. Cancer Res. 50:7758-7764 (1990).

Mizuno K, Higuchi O, Ihle JN, and Nakamura T. Hepatocyte growth factor stimulates growth of hematopoietic progenitor cells. Biochem. Biophys. Res. Commun. 194:178-186 (1993). Monajemi H, Fontijn RD, Pannekoek H, and Horrevoets AJ. The apolipoprotein L gene cluster has emerged recently in evolution and is expressed in human vascular tissue. Genomics 79:539-546 (2002).

Montesano R, Soriano JV, Malinda KM, Ponce ML, Bafico A, Kleinman HK, Bottaro DP, and Aaronson SA. Differential effects of hepatocyte growth factor isoforms on epithelial and endothelial tubulogenesis. Cell Growth Differ. 9:355-365 (1998).

Mori M, Beatty PG, Graves M, Boucher KM, and Milford EL. HLA gene and haplotype frequencies in the North American population: the National Marrow Donor Program Donor Registry. Transplantation 64:1017-1027 (1997).

Mott JD and Werb Z. Regulation of matrix biology by matrix metalloproteinases. Curr. Opin. Cell Biol. 16:558-564 (2004).

Mueller MRWJ, Brugger W, Gouttefangeas.C., Kanz L, and Brossart P. Vaccinations with peptide pulsed dendritic cells induces clinical and immunological responses in patients with metastatic renal cell carcinoma. Proc Am Soc Clin Oncol 22, 168. 1-6-2003. Ref Type: Abstract

Naldini L, Vigna E, Narsimhan RP, Gaudino G, Zarnegar R, Michalopoulos GK, and Comoglio PM. Hepatocyte growth factor (HGF) stimulates the tyrosine kinase activity of the receptor encoded by the proto-oncogene c-MET. Oncogene 6:501-504 (1991).

Neumann F, Wagner C, Stevanovic S, Kubuschok B, Schormann C, Mischo A, Ertan K, Schmidt W, and Pfreundschuh M. Identification of an HLA-DR-restricted peptide epitope with a promiscuous binding pattern derived from the cancer testis antigen HOM-MEL-40/SSX2. Int. J. Cancer 112:661-668 (2004).

Noto H, Takahashi T, Makiguchi Y, Hayashi T, Hinoda Y, and Imai K. Cytotoxic T lymphocytes derived from bone marrow mononuclear cells of multiple myeloma patients recognize an underglycosylated form of MUC1 mucin. Int. Immunol. 9:791-798 (1997).

Ohara O, Nagase T, Ishikawa K, Nakajima D, Ohira M, Seki N, and Nomura N. Construction and characterization of human brain cDNA libraries suitable for analysis of cDNA clones encoding relatively large proteins. DNA Res. 4:53-59 (1997).

Pachter JS, de Vries HE, and Fabry Z. The blood-brain barrier and its role in immune privilege in the central nervous system. J. Neuropathol. Exp. Neurol. 62:593-604 (2003).

Pass, H. A., S. L. Schwarz, J. R. Wunderlich, and S. A. Rosenberg. Immunization of patients with melanoma peptide vaccines: immunologic assessment using the ELISPOT assay. Cancer J. Sci. Am. 4:316 (1998).

Pons E, Uphoff CC, and Drexler HG. Expression of hepatocyte growth factor and its receptor c-met in human leukemia-lymphoma cell lines. Leuk. Res. 22:797-804 (1998).

Ponzetto C, Bardelli A, Maina F, Longati P, Panayotou G, Dhand R, Waterfield MD, and Comoglio PM. A novel recognition motif for phosphatidylinositol 3-kinase binding mediates its association with the hepatocyte growth factor/scatter factor receptor. Mol. Cell Biol. 13:4600-4608 (1993).

Previsani N and Lavanchy D. Hepatitis B. World Health Organization Department of Communicable Diseases Surveillance and Response. 2002 WHO/CDS/CSR/LYO/2002. 2:Hepatitis B. (2002).

Qian CN, Guo X, Cao B, Kort EJ, Lee CC, Chen J, Wang LM, Mai WY, Min HQ, Hong MH, Vande Woude GF, Resau JH, and Teh BT. Met protein expression level correlates with survival in patients with late-stage nasopharyngeal carcinoma. Cancer Res. 62:589-596 (2002).

Quantin B, Murphy G, and Breathnach R. Pump-1 cDNA codes for a protein with characteristics similar to those of classical collagenase family members. Biochemistry 28:5327-5334 (1989).

Rae FK, Stephenson SA, Nicol DL, and Clements JA. Novel association of a diverse range of genes with renal cell carcinoma as identified by differential display. Int. J. Cancer 88:726-732 (2000).

Ramirez R, Hsu D, Patel A, Fenton C, Dinauer C, Tuttle RM, and Francis GL. Over-expression of hepatocyte growth factor/scatter factor (HGF/SF) and the HGF/SF receptor (cMET) are associated with a high risk of metastasis and recurrence for children and young adults with papillary thyroid carcinoma. Clin Endocrinol. (Oxf) 53:635-644 (2000).

Rammensee H, Bachmann J, Emmerich NP, Bachor OA, and Stevanovic S. SYFPEITHI: database for MHC ligands and peptide motifs. Immunogenetics 50:213-219 (1999).

Rammensee,H.G., Bachmann,J., and Stevanovic,S. (1997). MHC Ligands and Peptide Motifs. Springer-Verlag, Heidelberg, Germany).

Rehermann B and Nascimbeni M. Immunology of hepatitis B virus and hepatitis C virus infection. Nat. Rev. Immunol. 5:215-229 (2005).

Rentzsch C, Kayser S, Stumm S, Watermann I, Walter S, Stevanovic S, Wallwiener D, and Guckel B. Evaluation of pre-existent immunity in patients with primary breast cancer: molecular and cellular assays to quantify antigen-specific T lymphocytes in peripheral blood mononuclear cells. Clin Cancer Res. 9:4376-4386 (2003).

Rotzschke O, Falk K, Stevanovic S, Jung G, Walden P, and Rammensee HG. Exact prediction of a natural T-cell epitope. Eur. J. Immunol. 21:2891-2894 (1991).

Rubin JS, Bottaro DP, and Aaronson SA. Hepatocyte growth factor/scatter factor and its receptor, the c-met proto-oncogene product. Biochim. Biophys. Acta 1155:357-371 (1993).

Saha S, Bardelli A, Buckhaults P, Velculescu VE, Rago C, St CB, Romans KE, Choti MA, Lengauer C, Kinzler KW, and Vogelstein B. A phosphatase associated with metastasis of colorectal cancer. Science 294:1343-1346 (2001).

Saino M, Maruyama T, Sekiya T, Kayama T, and Murakami Y. Inhibition of angiogenesis in human glioma cell lines by antisense RNA from the soluble guanylate cyclase genes, GUCY1A3 and GUCY1B3. Oncol. Rep. 12:47-52 (2004).

Schag K, Schmidt SM, Muller MR, Weinschenk T, Appel S, Weck MM, Grunebach F, Stevanovic S, Rammensee HG, and Brossart P. Identification of C-met oncogene as a broadly expressed tumor-associated antigen recognized by cytotoxic T-lymphocytes. Clin Cancer Res. 10:3658-3666 (2004).

Scheibenbogen, C., A. Schmittel, U. Keilholz, T. Allgauer, U. Hofmann, R. Max, E. Thiel, and D. Schadendorf.. Phase 2 trial of vaccination with tyrosinase peptides and granulocyte-macrophage colony-stimulating factor in patients with metastatic melanoma. J. Immunother. 23:275. (2000)

Schirle M, Weinschenk T, and Stevanovic S. Combining computer algorithms with experimental approaches permits the rapid and accurate identification of T-cell epitopes from defined antigens. J. Immunol. Methods 257:1-16 (2001).

Schmidt C, Bladt F, Goedecke S, Brinkmann V, Zschiesche W, Sharpe M, Gherardi E, and Birchmeier C. Scatter factor/hepatocyte growth factor is essential for liver development. Nature 373:699-702 (1995).

Siddiqui J, Abe M, Hayes D, Shani E, Yunis E, and Kufe D. Isolation and Sequencing of a cDNA Coding for the Human DF3 Breast Carcinoma-Associated Antigen. PNAS 85:2320-2323 (1988).

Slingluff, C. L., Jr., G. R. Petroni, G. V. Yamshchikov, D. L. Barnd, S. Eastham, H. Galavotti, J. W. Patterson, D. H. Deacon, S. Hibbitts, D. Teates, P. Y. Neese, W. W. Grosh, K. A. Chianese-Bullock, E. M. Woodson, C. J. Wiernasz, P. Merrill, J. Gibson, M. Ross, and V. H. Engelhard. Clinical and immunologic results of a randomized phase II trial of vaccination using four melanoma peptides either administered in granulocyte-macrophage colony-stimulating factor in adjuvant or pulsed on dendritic cells. J. Clin. Oncol. 21:4016. (2003)

Sun Y, Song M, Stevanovic S, Jankowiak C, Paschen A, Rammensee HG, and Schadendorf D. Identification of a new HLA-A(*)0201-restricted T-cell epitope from the tyrosinase-related protein 2 (TRP2) melanoma antigen. Int. J. Cancer 87:399-404 (2000).

Takahashi T, Makiguchi Y, Hinoda Y, Kakiuchi H, Nakagawa N, Imai K, and Yachi A. Expression of MUC1 on myeloma cells and induction of HLA-unrestricted CTL against MUC1 from a multiple myeloma patient. J. Immunol. 153:2102-2109 (1994).

Takayama H, Larochelle WJ, Sharp R, Otsuka T, Kriebel P, Anver M, Aaronson SA, and Merlino G. Diverse tumorigenesis associated with aberrant development in mice overexpressing hepatocyte growth factor/scatter factor. Proc. Natl. Acad. Sci. U. S. A 94:701-706 (1997).

Takayama H, LaRochelle WJ, Anver M, Bockman DE, and Merlino G. Scatter factor/hepatocyte growth factor as a regulator of skeletal muscle and neural crest development. PNAS 93:5866-5871 (1996).

Teofili L, Di Febo AL, Pierconti F, Maggiano N, Bendandi M, Rutella S, Cingolani A, Di RN, Musto P, Pileri S, Leone G, and Larocca LM. Expression of the c-met proto-oncogene and its ligand, hepatocyte growth factor, in Hodgkin disease. Blood 97:1063-1069 (2001). Tripathi A, Dasgupta S, Roy A, Sengupta A, Roy B, Roychowdhury S, and Panda CK. Sequential deletions in both arms of chromosome 9 are associated with the development of head and neck squamous cell carcinoma in Indian patients. J. Exp. Clin. Cancer Res. 22:289-297 (2003).

Troussard X, vet-Loiseau H, Macro M, Mellerin MP, Malet M, Roussel M, and Sola B. Cyclin D1 expression in patients with multiple myeloma. Hematol. J. 1:181-185 (2000). Tuck AB, Park M, Stems EE, Boag A, and Elliott BE. Coexpression of hepatocyte growth factor and receptor (Met) in human breast carcinoma. Am. J. Pathol. 148:225-232 (1996). Uehara Y, Minowa O, Mori C, Shiota K, Kuno J, Noda T, and Kitamura N. Placental defect and embryonic lethality in mice lacking hepatocyte growth factor/scatter factor. Nature 373:702-705 (1995).

van d, V, Taher TE, Keehnen RM, Smit L, Groenink M, and Pals ST. Paracrine regulation of germinal center B cell adhesion through the c-met-hepatocyte growth factor/scatter factor pathway. J. Exp. Med. 185:2121-2131 (1997).

Vasef MA, Brynes RK, Sturm M, Bromley C, and Robinson RA. Expression of cyclin D1 in parathyroid carcinomas, adenomas, and hyperplasias: a paraffin immunohistochemical study. Mod. Pathol. 12:412-416 (1999).

Walter S, Herrgen L, Schoor O, Jung G, Wernet D, Buhring HJ, Rammensee HG, and Stevanovic S. Cutting Edge: Predetermined Avidity of Human CD8 T-cells Expanded on Calibrated MHC/Anti-CD28-Coated Microspheres. J Immunol 171:4974-4978 (2003).

Wang FQ, So J, Reierstad S, and Fishman DA. Matrilysin (MMP-7) promotes invasion of ovarian cancer cells by activation of progelatinase. Int. J Cancer 114:19-31 (2005).

Wang R, Ferrell LD, Faouzi S, Maher JJ, and Bishop JM. Activation of the Met receptor by cell attachment induces and sustains hepatocellular carcinomas in transgenic mice. J. Cell Biol. 153:1023-1034 (2001).

Weber RG, Rieger J, Naumann U, Lichter P, and Weller M. Chromosomal imbalances associated with response to chemotherapy and cytotoxic cytokines in human malignant glioma cell lines. Int. J. Cancer 91:213-218 (2001).

Weinschenk T, Gouttefangeas C, Schirle M, Obermayr F, Walter S, Schoor O, Kurek R, Loeser W, Bichler KH, Wernet D, Stevanovic S, and Rammensee HG. Integrated functional genomics approach for the design of patient-individual antitumor vaccines. Cancer Res. 62:5818-5827 (2002).

Wierecky J, Muller MR, Horger MS, Brugger W, Kanz L, and Brossart P. Induction of clinical and immunological responses in patients with metastatic renal cell carcinoma after vaccinations with peptide pulsed dendritic cells. J Clin Oncol (Meeting Abstracts) 23:2507 (2005).

Wills MR, Carmichael AJ, Mynard K, Jin X, Weekes MP, Plachter B, and Sissons JG. The human cytotoxic T-lymphocyte (CTL) response to cytomegalovirus is dominated by structural protein pp65: frequency, specificity, and T-cell receptor usage of pp65-specific CTL. J Virol. 70:7569-7579 (1996).

Xiong Y, Connolly T, Futcher B, and Beach D. Human D-type cyclin. Cell 65:691-699 (1991).

Yewdell JW, Reits E, and Neefjes J. Making sense of mass destruction: quantitating MHC class I antigen presentation. Nat. Rev. Immunol. 3:952-961 (2003).

Young AN, Amin MB, Moreno CS, Lim SD, Cohen C, Petros JA, Marshall FF, and Neish AS. Expression profiling of renal epithelial neoplasms: a method for tumor classification and discovery of diagnostic molecular markers. Am. J. Pathol. 158:1639-1651 (2001).

Zarnegar R and Michalopoulos GK. The many faces of hepatocyte growth factor: from hepatopoiesis to hematopoiesis. J. Cell Biol. 129:1177-1180 (1995).

Zhou YT, Guy GR, and Low BC. BNIP-2 induces cell elongation and membrane protrusions by interacting with Cdc42 via a unique Cdc42-binding motif within its BNIP-2 and Cdc42GAP homology domain. Exp. Cell Res. 303:263-274 (2005).

Romero P, Cerottini JC, Speiser DE. Monitoring tumor antigen specific T-cell responses in cancer patients and phase I clinical trials of peptide-based vaccination. Cancer Immunol Immunother. 2004 Mar;53(3):249-55.

Schmittel A, Keilholz U, Thiel E, Scheibenbogen C. Quantification of tumor-specific T lymphocytes with the ELISPOT assay. J Immunother. 2000 May-Jun;23(3):289-95.

Wierecky J, Müller M, Horger M, Brugger W, Kanz L, Brossart P. Induction of clinical and immunological responses in patients with metastatic renal cell carcinoma after vaccinations with peptide pulsed dendritic cells. Abstract No. 2507 presented at the Annual Meeting of the American Society of Clinical Oncology ASCO (2005).

## Claims

1. A tumour associated peptide according to SEQ ID No. 2 (VMAGDIYSV) or a peptide according to SEQ ID No. 2, wherein the side chains of one or two amino acid residues are altered, having the ability to bind to a molecule of the human major histocompatibility complex (MHC) class I HLA-A*02.

2. The tumour associated peptide according to Claim 1, wherein the peptide includes non-peptide bonds.

3. A fusion protein comprising the tumour associated peptide according to any of claims 1 or 2, in particular further comprising N-terminal amino acids of the HLA-DR antigen-associated invariant chain (Ii).

4. A nucleic acid, consisting of a sequence encoding a peptide according to Claim 1.

5. The nucleic acid according to Claim 4 which is DNA, cDNA, PNA, CNA, RNA or combinations thereof.

6. An expression vector capable of expressing a nucleic acid according to Claim 4 or 5.

7. A tumour associated peptide according to any one of Claims 1 to 3, a nucleic acid according to Claim 4 or 5 or an expression vector according to Claim 6 for use in medicine.

8. A host cell comprising a nucleic acid according to Claim 4 or 5 or an expression vector according to Claim 6.

9. The host cell according to Claim 8 that is a recombinant RCC or Awells cell.

10. A pharmaceutical composition comprising at least one tumour associated peptide according to any one of Claims 1 to 3, a nucleic acid according to Claim 4 or 5 or an expression vector according to Claim 6, and a pharmaceutically acceptable carrier.

11. The pharmaceutical composition according to claim 10, further comprising a peptide according to SEQ ID No. 1 or a peptide according to SEQ ID No. 1, wherein the side chains of one or two amino acid residues are altered, having the ability to bind to a molecule of the human major histocompatibility complex (MHC) class-II.

12. The pharmaceutical composition according to claim 10 or 11, further comprising at least one additional peptide comprising a sequence according to any of SEQ ID No. 3 to SEQ ID No. 11.

13. The pharmaceutical composition according to any of claims 10 to 12, wherein at least one peptide includes non-peptide bonds.

14. The pharmaceutical composition according to any of claims 10 to 13, comprising peptides consisting of amino acid sequences according to SEQ ID No. 1 and SEQ ID No. 2 and SEQ ID No. 3 to SEQ ID No. 11.

15. The pharmaceutical composition according to any of claims 10 to 14, further comprising at least one suitable adjuvant.

16. The pharmaceutical composition according to claim 15, wherein said adjuvant is selected from the group of colony-stimulating factors, such as Granulocyte Macrophage Colony Stimulating Factor (GM- CSF).

17. Use of a pharmaceutical composition according to any of claims 10 to 16 for the preparation of a vaccine against cancer.

18. A method of producing a tumour associated peptide according to any one of Claims 1 to 3 the method comprising culturing the host cell according to Claim 8, and isolating the peptide from the host cell or its culture medium.

19. Use of a tumour associated peptide according to any one of Claims 1 to 3 or a nucleic acid according to Claim 4 or 5 or an expression vector according to Claim 6 in the manufacture of a medicament for killing target cells in a patient which target cells express a polypeptide comprising an amino acid sequence as given in Claim 1.

20. An *in vitro* method for producing activated cytotoxic T lymphocytes (CTL), the method comprising contacting *in vitro* CTL with antigen loaded human class I MHC molecules expressed on the surface of a suitable antigen-presenting cell for a period of time sufficient to activate said CTL in an antigen specific manner, wherein said antigen is a peptide according to any one of Claims 1 to 3.

21. The method according to Claim 20, wherein the antigen is loaded onto class I MHC molecules expressed on the surface of a suitable antigen-presenting cell by contacting a sufficient amount of the antigen with an antigen-presenting cell.

22. The method according to Claim 20, wherein the antigen-presenting cell comprises an expression vector according to Claim 6.

23. Activated cytotoxic T lymphocytes (CTL), produced by the method according to any one of Claims 20 to 22, which selectively recognise a cell which expresses a polypeptide comprising an amino acid sequence given in claim 1.

24. Use of a tumour associated peptide to any of claims 1 to 3, a nucleic acid according to claim 4 or 5, an expression vector according to claim 6, a pharmaceutical composition according to any one of claim 10 to 16 in the manufacture of a medicament for killing cancer cells in a patient.

25. Use according to claim 24, wherein said cancer cells are renal cancer cells.

## Patentansprüche

1. Ein tumorassoziierte Peptid gemäß SEQ ID Nr. 2 (VMAGDIYSV) oder ein Peptid gemäß SEQ ID Nr. 2, wobei die Seitenketten von einer oder zwei der Aminosäurereste verändert wurden, welches die Fähigkeit hat, an ein Molekül des menschlichen Haupthistokompatibilitätskomplexes (MHC) Klasse I. HLA-A*02 zu binden.

2. Das tumorassoziierte Peptid nach Anspruch 1, wobei das Peptid nicht-peptidische Bindungen einschließt.

3. Ein Fusionsprotein, umfassend das tumorassoziierte Peptid gemäß einem der Ansprüche 1 oder 2, insbesondere weiter umfassend die N-terminalen Aminosäuren der HLA-DR Antigen-assoziierten invarianten Kette (Ii).

4. Eine Nukleinsäure bestehend aus einer Sequenz, die ein Peptid nach Anspruch 1 kodiert.

5. Nukleinsäure nach Anspruch 4, die DNA, cDNA, PNA, CNA, RNA oder Kombinationen davon ist.

6. Ein Expressionsvektor, der in der Lage ist, eine Nukleinsäure nach Anspruch 4 oder 5 zu exprimieren.

7. Ein tumorassoziiertes Peptid nach einem der Ansprüche 1 bis 3, eine Nukleinsäure nach Anspruch 4 oder 5 oder ein Expressionsvektor nach Anspruch 6 zur Verwendung in der Medizin.

8. Eine Wirtszelle umfassend eine Nukleinsäure nach Anspruch 4 oder 5 oder einen Expressionsvektor nach Anspruch 6.

9. Wirtszelle nach Anspruch 8, die eine rekombinante RCC- oder Awells-Zelle ist.

10. Eine pharmazeutische Zusammensetzung umfassend zumindest ein tumorassoziiertes Peptid nach einem der Ansprüche 1 bis 3, eine Nukleinsäure nach Anspruch 4 oder 5 oder einen Expressionsvektor nach Anspruch 7 und einen pharmazeutisch akzeptablen Träger.

11. Die pharmazeutische Zusammensetzung nach Anspruch 10, des Weiteren umfassend ein Peptid gemäß SEQ ID Nr. 1 oder ein Peptid gemäß SEQ ID Nr. 1, in dem die Seitenketten von einer oder zwei der Aminosäurereste verändert wurden, welches die Fähigkeit hat, an ein Molekül des menschlichen Haupthistokompatibilitätskomplexes (MHC) Klasse II zu binden.

12. Die pharmazeutische Zusammensetzung gemäß Anspruch 10 oder 11, des Weiteren umfassend zumindest ein zusätzliches tumorassoziiertes Peptid umfassend eine Sequenz gemäß einer der SEQ ID Nr. 3 bis SEQ ID Nr. 11.

13. Die pharmazeutische Zusammensetzung nach einem der Ansprüche 10 bis 14, wobei mindestens ein Peptid nicht-peptidische Bindungen einschließt.

14. Die pharmazeutische Zusammensetzung nach einem der Ansprüche 10 bis 13, umfassend Peptide, die aus den Aminosäuresequenzen gemäß SEQ ID Nr. 1 und SEQ ID Nr. 2 und SEQ ID Nr. 3 bis SEQ ID Nr. 11 bestehen.

15. Die pharmazeutische Zusammensetzung nach einem der Ansprüche 10 bis 14 weiterhin umfassend mindestens ein geeignetes Adjuvants.

16. Die pharmazeutische Zusammensetzung nach Anspruch 15, wobei besagtes Adjuvants aus der Gruppe der koloniestimulierenden Faktoren ausgewählt wird, wie zum Beispiel dem Granulocyten-Macrophagen-Kolonie-stimulierenden Faktor (GM-CSF).

17. Verwendung einer pharmazeutischen Zusammensetzung nach einem der Ansprüche 10 bis 16 für die Herstellung eines Impfstoffs gegen Krebs.

18. Verfahren zur Herstellung eines tumorassoziierten Peptids nach einem der Ansprüche 1 bis 3, wobei das Verfahren Kultivieren der Wirtszelle nach Anspruch 8 und Isolieren des Peptids aus der Wirtszelle oder ihrem Kulturmedium umfasst.

19. Verwendung eines tumorassoziierten Peptids nach einem der Ansprüche 1 bis 3 oder einer Nukleinsäure nach Anspruch 4 oder 5 oder eines Expressionsvektors nach Anspruch 6 zur Herstellung eines Medikaments zur Abtötung von Zielzellen in einem Patienten, wobei die Zielzellen ein Polypeptid umfassend eine Aminosäuresequenz, wie in Anspruch 1 angegeben, exprimieren.

20. Ein *in vitro* Verfahren zur Produktion aktivierter zytotoxischer T-Lymphozyten (CTL), das Verfahren umfassend das *in vitro* in Kontakt bringen von CTL mit Antigenbeladenen humanen Klasse I MHC Molekülen, die an der Oberfläche einer geeigneten Antigen-präsentierenden Zelle für eine ausreichende lange Zeit exprimiert werden, um besagte CTL in einer Antigen-spezifischen Art und Weise zu aktivieren, wobei das Antigen ein Peptid nach einem der Ansprüche 1 bis 3 ist.

21. Das Verfahren nach Anspruch 20, wobei das Antigen auf ein MHC-Klasse-I-Molekül beladen ist, welches an der Oberfläche einer geeigneten Antigen-präsentierenden Zelle durch in Kontakt bringen einer ausreichenden Menge des Antigens mit der Antigen-präsentierenden Zelle exprimiert wird.

22. Verfahren nach Anspruch 20, wobei die Antigen-präsentierende Zelle einen Expressionsvektor nach Anspruch 6 umfasst.

23. Aktivierte zytotoxische T-Lymphozyten (CTL) hergestellt nach dem Verfahren nach einem der Ansprüche 20 bis 22, welche selektiv eine Zelle erkennen, die ein Polypeptid umfassend eine Aminosäuresequenz, angegeben in Anspruch 1, exprimiert.

24. Verwendung eines tumorassoziierten Peptids nach einem der Ansprüche 1 bis 3, einer Nukleinsäure nach Anspruch 4 oder 5, eines Expressionsvektors nach Anspruch 6, einer pharmazeutischen Zusammensetzung nach einem der Ansprüche 10 bis 16 zur Herstellung eines Medikaments zur Abtötung von Krebszellen in einem Patienten.

25. Verwendung nach Anspruch 24, wobei besagte Krebszellen Nierenkrebszellen sind.

## Revendications

1. Peptide associé à une tumeur correspondant à la séquence SEQ ID n° 2 (VMAGDIYSV) ou peptide correspondant à la séquence SEQ ID n° 2, dans lequel les chaînes latérales d'un ou de deux résidus d'acides aminés sont modifiées, et capable de se lier à une molécule du complexe majeur d'histocompatibilité (CMH) humain de classe I. HLA-A*02.

2. Peptide associé à une tumeur conforme à la revendication 1, dans lequel ledit peptide comprend des liaisons non peptidiques.

3. Protéine de fusion comprenant le peptide associé à une tumeur conforme à la revendication 1 ou 2, comprenant en particulier des acides aminés N-terminaux de la chaîne invariante (li) associée aux antigènes du système HLA-DR.

4. Acide nucléique, consistant en une séquence codant pour un peptide tel que décrit dans la revendication 1.

5. Acide nucléique conforme à la revendication 4, qui est un ou des combinaisons d'ADN, ADNc, PNA, CNA, ARN.

6. Vecteur d'expression capable d'exprimer un acide nucléique conforme à la revendication 4 ou 5.

7. Peptide associé à une tumeur conforme à l'une des revendications 1 à 3, acide nucléique conforme à la revendication 4 ou 5 ou vecteur d'expression conforme à la revendication 6, pour usage médical.

8. Cellule hôte comprenant un acide nucléique conforme à la revendication 4 ou 5 ou un vecteur d'expression conforme à la revendication 6.

9. Cellule hôte conforme à la revendication 8, qui est une cellule Awells ou une cellule de néphrocarcinome recombinante.

10. Composition pharmaceutique comprenant au moins un peptide associé à une tumeur conforme à l'une des revendications 1 à 3, un acide nucléique conforme à la revendication 4 ou 5 ou un vecteur d'expression conforme à la revendication 7, ainsi qu'un transporteur pharmaceutiquement acceptable.

11. Composition pharmaceutique conforme à la revendication 10, comprenant également un peptide correspondant à la séquence SEQ ID n° 1 ou un peptide correspondant à la séquence SEQ ID n° 1, dans lequel les chaînes latérales d'un ou de deux résidus d'acides aminés sont modifiés, et capable de se lier à une molécule du complexe majeur d'histocompatibilité (CMH) humain de classe II.

12. Composition pharmaceutique conforme à la revendication 10 ou 11, comprenant également au moins un peptide supplémentaire qui comprend une séquence correspondant à l'une des séquences SEQ ID n° 3 à SEQ ID n° 11.

13. Composition pharmaceutique conforme à l'une des revendications 10 à 14, dans laquelle au moins un peptide comprend des liaisons non peptidiques.

14. Composition pharmaceutique conforme à l'une des revendications 10 à 13, comprenant des peptides consistant en des séquences d'acides aminés correspondant aux séquences SEQ ID n° 1 et SEQ ID n° 2 et SEQ ID n° 3 à SEQ ID n° 11.

15. Composition pharmaceutique conforme à l'une des revendications 10 à 14, comprenant également au moins un adjuvant approprié.

16. Composition pharmaceutique conforme à la revendication 15, dans laquelle ledit adjuvant est sélectionné à partir d'un groupe de facteurs de croissance hématopoïétique tels que le facteur stimulant la formation de colonies de granulocytes et de macrophages (GM- CSF).

17. Utilisation d'une composition pharmaceutique conforme à l'une des revendications 10 à 16 pour la préparation d'un vaccin anticancéreux.

18. Méthode de production d'un peptide associé à une tumeur conforme à l'une des revendications 1 à 3, comprenant la culture de la cellule hôte telle que décrite dans la revendication 8 et l'isolement du peptide de la cellule hôte ou de son milieu de culture.

19. Utilisation d'un peptide associé à une tumeur conforme à l'une des revendications 1 à 3 ou d'un acide nucléique conforme à la revendication 4 ou 5 ou d'un vecteur d'expression conforme à la revendication 6 dans la fabrication d'un médicament destiné à tuer les cellules cibles chez un patient dont les cellules cibles expriment un polypeptide comprenant une séquence d'acides aminés telle que décrite dans la revendication 1.

20. Méthode *in vitro* de production de lymphocytes T cytotoxiques (CTL) activés, comprenant la mise en contact *in vitro* des CTL avec des molécules du CMH de classe I humain chargées d'antigènes exprimées à la surface d'une cellule présentatrice d'antigènes appropriée pendant un temps suffisant pour activer, de manière antigène-spécifique, lesdits CTL dans lesquels l'antigène est un peptide conforme à l'une des revendications 1 à 3.

21. Méthode conforme à la revendication 20, dans laquelle l'antigène est chargé sur les molécules du CMH de classe I exprimées à la surface d'une cellule présentatrice d'antigènes appropriée, en mettant en contact une quantité suffisante de l'antigène avec une cellule présentatrice d'antigènes.

22. Méthode conforme à la revendication 20, dans laquelle la cellule présentatrice d'antigènes comprend un vecteur d'expression conforme à la revendication 6.

23. Lymphocytes T cytotoxiques (CTL) activés, produits par la méthode conforme à l'une des revendications 20 à 22, qui reconnaissent de façon sélective une cellule exprimant un polypeptide comprenant une séquence d'acides aminés telle que décrite dans la revendication 1.

24. Utilisation d'un peptide associé à une tumeur conforme à l'une des revendications 1 à 3 ou d'un acide nucléique conforme à la revendication 4 ou 5 ou d'un vecteur d'expression conforme à la revendication 6, d'une composition pharmaceutique conforme à l'une des revendications 10 à 16 dans la fabrication d'un médicament destiné à tuer les cellules cancéreuses chez un patient.

25. Utilisation conforme à la revendication 24, dans laquelle lesdites cellules cancéreuses sont des cellules cancéreuses rénales.


### SEQUENCE LISTING

<110> Immatics biotechnologies GmbH
<120> Tumor-associated peptides binding to human leukocyte antigen (HLA) class I or II molecules and related anti-cancer vaccine
<130> I30559PCT
<160> 11
<170> PatentIn version 3.3
<210> 1
   <211> 16
   <212> PRT
   <213> Homo sapiens
<400> 1
   Ser Gln Asp Asp Ile Lys Gly Ile Gln Lys Leu Tyr Gly Lys Arg Ser
   1               5                   10                  15
<210> 2
   <211> 9
   <212> PRT
   <213> Homo sapiens
<400> 2
   Val Met Ala Gly Asp Ile Tyr Ser Val
   1               5
<210> 3
   <211> 9
   <212> PRT
   <213> Homo sapiens
<400> 3
   Ser Val Ala Ser Thr Ile Thr Gly Val
   1 5
<210> 4
   <211> 9
   <212> PRT
   <213> Homo sapiens
<400> 4
   Ala Leu Ala Asp Gly Val Gln Lys Val
   1 5
<210> 5
   <211> 9
   <212> PRT
   <213> Homo sapiens
<400> 5
   Leu Leu Gly Ala Thr Cys Met Phe Val
   1 5
<210> 6
   <211> 9
   <212> PRT
   <213> Homo sapiens
<400> 6
   Ser Val Phe Ala Gly Val Val Gly Val
   1 5
<210> 7
   <211> 9
   <212> PRT
   <213> Homo sapiens
<400> 7
   Ala Leu Phe Asp Gly Asp Pro His Leu
   1 5
<210> 8
   <211> 9
   <212> PRT
   <213> Homo sapiens
<400> 8
   Tyr Val Asp Pro Val Ile Thr Ser Ile
   1               5
<210> 9
   <211> 9
   <212> PRT
   <213> Homo sapiens
<400> 9
   Ser Thr Ala Pro Pro Val His Asn Val
   1               5
<210> 10
   <211> 9
   <212> PRT
   <213> Homo sapiens
<400> 10
   Leu Ala Ala Leu Pro His Ser Cys Leu
   1               5
<210> 11
   <211> 10
   <212> PRT
   <213> Hepatitis B virus
<400> 11
   Phe Leu Pro Ser Asp Phe Phe Pro Ser Val
   1               5                   10